(19) Europäisches Patentamt
European Patent Office
Office européen des brevets

(11) EP 2 591 123 B2

(12) **NEW EUROPEAN PATENT SPECIFICATION**
After opposition procedure

(45) Date of publication and mention of the opposition decision:
**29.05.2024 Bulletin 2024/22**

(45) Mention of the grant of the patent:
**05.10.2016 Bulletin 2016/40**

(21) Application number: **11745831.5**

(22) Date of filing: **07.07.2011**

(51) International Patent Classification (IPC):
***C12N 9/28*** *(2006.01)* ***C12Q 1/40*** *(2006.01)*
***C12Q 1/00*** *(2006.01)*

(52) Cooperative Patent Classification (CPC):
**C12N 9/2417; A23K 20/174; A23K 20/189; A23K 20/20; A23K 40/10; A23K 40/30; A23K 50/30; A23K 50/75; A23K 50/80; C12Q 1/40; C12Y 302/01001;** A23K 40/25; C07K 2319/02; Y02A 40/818

(86) International application number:
**PCT/IB2011/053018**

(87) International publication number:
**WO 2012/004759 (12.01.2012 Gazette 2012/02)**

(54) **A FEED SUPPLEMENT FOR A MONOGASTRIC ANIMAL, USE THEREOF AND METHOD OF PRODUCING IT**

FUTTERZUSATZ FÜR EIN MONOGASTRISCHES TIER, DESSEN VERWENDUNG UND VERFAHREN ZU SEINER HERSTELLUNG

SUPPLÉMENT ALIMENTAIRE POUR UN ANIMAL MONOGASTRIQUE, SON UTILISATION ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2010 US 363865 P**
**08.07.2010 GB 201011513**

(43) Date of publication of application:
**15.05.2013 Bulletin 2013/20**

(73) Proprietor: **DuPont Nutrition Biosciences ApS**
**1411 Copenhagen K (DK)**

(72) Inventor: **ISAKSEN, Mai Faurschou**
**8270 Hojbjerg (DK)**

(74) Representative: **D Young & Co LLP**
**3 Noble Street**
**London EC2V 7BQ (GB)**

(56) References cited:
**WO-A1-98/26078**
**WO-A2-03/049550**
**WO-A2-2008/080093**
**WO-A2-2008/112729**
**WO-A2-2008/153805**
**WO-A2-2012/110778**
**JP-A- 7 143 880**
**US-A- 5 372 811**
**US-B1- 6 211 134**

- **HRISTOV A N ET AL: "EFFECT OF DIETARY OR ABOMASAL SUPPLEMENTATION OF EXOGENOUS POLYSACCHARIDE-DEGRADING ENZYMES ON RUMEN FERMENTATION AND NUTRIENTDIGESTIBILITY", JOURNAL OF ANIMAL SCIENCE, AMERICAN SOCIETY OF ANIMAL SCIENCE, US, vol. 76, no. 12, 1 January 1998 (1998-01-01), pages 3146-3156, XP000992906, ISSN: 0021-8812**
- **GRACIA et al.: "?-Amylase Supplementation of Broiler Diets Based on Corn", Poultry Science, vol. 82, 2003, pages 436-442,**

EP 2 591 123 B2

## Description

### FIELD OF THE INVENTION

The present invention relates to pepsin resistant alpha amylases. More specifically, the present invention relates to methods for identifying pepsin resistant alpha amylases for use in feed supplements and feedstuffs, feed supplements and feedstuffs comprising pepsin resistant alpha amylases, uses thereof and pepsin resistant alpha amylases for use in feed supplements and feedstuffs.

### TECHNICAL BACKGROUND AND PRIOR ART

Feed, including any feed supplements, consumed by monogastric animals, is subjected to a very acidic (pH 2-3) environment along with enzyme degradation in the stomach. This harsh low pH environment is followed in the small intestine by a more neutral environment (pH 6-7) and degradation by further digestive enzymes (trypsin, chymotrypsin, elastase).

Poultry and swine are omnivorous and given the opportunity would satisfy their nutrient requirements via the consumption of a range of seeds, roots, inorganic material and insects. However in order to satisfy consumer preference for 'vegetarian animal production' and to minimize feed costs associated with the industrial production of farm animals, the feed which is presented to these animals is rarely optimal for the digestive system, especially in the neonate.

For example the non-starch polysaccharide (NSP) fraction of some cereals such as wheat and barley increases viscosity in the gut, which compromises diffusion of nutrients. This anti-nutritional effect can be reduced by addition of the xylanase and/or beta-glucanase which fragment hemicellulose polymers, xylan and beta-glucan, respectively.

Another example of improving the availability of nutrients is degradation of phytic acid, the plants phosphate storage, which is not readily hydrolysed by enzymes produced by the animal. Addition of phytase to the feed ensures release of phosphate from phytic acid, and can thereby partly or totally cover the animals need for phosphate.

So, in some instances exogenous enzymes can bridge a gap between the nature of the feed and the animals own digestive enzyme complement. However research has shown that although both poultry and swine are capable of significant endogenous amylase and protease synthesis, there may still be an opportunity to augment the animal digestion through the use of exogenous enzymes and thereby improve animal performance.

Alpha-amylases amongst other supplements are added to feed to improve the starch utilisation of the feed. Historically, the amount of a particular amylase added to feed has been determined using standard amylase assays such as the tablet assay and, more recently, the KoneLab assay.

WO 2008/006881 solely relates to bovine feed and discloses the use of a bacterial amylase. However, the conditions in the gastro intestinal tracts of bovines differs markedly from that of monogastric animals and the composition of feed for bovines is markedly different to that for monogastric animals. For example, the majority of starch fed to ruminants is metabolised by microbes in the rumen which has a typical pH of 5.0 to 5.5, whereas in monogastric animals endogenous alpha amylase breaks starch down to glucose. Furthermore, pepsin, which is responsible for the breakdown of protein, is first released in the abomasums in the fourth and last compartment of the bovine stomach.

US 2008-0206401 discloses a very specific dry pet food comprising a thermostable alpha-amylase.

US 5,372,811 discloses a feed supplement comprising animal plasma protein and a microbial fermentation product of primarily amylase which are blended and spray dried.

WO 2008/080093 discloses polypeptides having an amylase and/or glucoamylase activity, which may be used to catalyse the hydrolysis of polysaccharide, oligosaccharide or starch into sugars. However, all the enzymes evaluated using an in vitro "stimulated gastric fluid" (SGF) test were digested by the gastric protease pepsin.

Accordingly, there is a need to identify alpha-amylases for use in feedstuffs which provide improved animal performance and/or which allow the alpha amylases to be used in reduced amounts with no detrimental effect on animal performance.

### SUMMARY OF THE INVENTION

The present invention is based on the inventor's surprising discovery that alpha amylases having pepsin resistance are particularly useful in feed, such as in feed for monogastric animals because they result in improved animal performance and/or allow the alpha amylases to be used in reduced amounts.

Pepsin is a digestive protease excreted by the animals in the first part of the digestive system. Pepsin degrades protein which makes the protein available as a nutrient for the animal. The exogenous enzymes, i.e. enzymes added to the feed, are also proteins and they will be degraded if they are susceptible to degradation by the pepsin. This will in most cases destroy the enzyme activity.

Without wishing to be bound by any theory, the inventor hypothesises that a pepsin resistant alpha amylase enzyme may result in the enzyme having increased activity in the ileum which allows for improved uptake of starch metabolites. Furthermore, the increased activity of the pepsin resistant enzyme may result in a decrease in the production of endogenous alpha amylases thereby increasing feed efficacy.

We describe herein a method for identifying a pepsin resistant alpha amylase enzyme for use in a feed supplement comprising:

i) providing an alpha amylase enzyme;
ii) admixing said alpha amylase with corn based feed and pepsin, incubating at pH 3 and analysing the alpha amylase activity of said alpha amylase compared to a control sample; wherein said control sample differs in that no pepsin is present during incubation at pH 3; and
iii) selecting an alpha amylase enzyme which substantially maintains alpha amylase activity under the assay conditions.

By "substantially maintains alpha amylase activity" it is meant that an alpha amylase which is subjected to the pepsin resistance assay detailed herein retains at least about 75% enzyme activity such as from about 75% to 125% enzyme activity when compared to the activity of the enzyme in the absence of pepsin. Thus, an alpha amylase which "substantially maintains alpha amylase activity" will on incubation with at least 9000 U/ml pepsin in the presence of corn based feed for at least 2 hours at pH3, retain at least about 75% (such as 75 to 125%) activity compared with the same enzyme on incubation with corn based feed for the same time (i.e. at least 2 hours) and the same pH (i.e. pH3).

By "pepsin resistant alpha amylase" it is meant an alpha amylase which retains at least about 75% (such as about 75% to 125%) activity when subjected to the pepsin resistance assay as detailed herein.

Without wishing to be bound by theory, the present inventor has surprisingly found that the pepsin resistance of an alpha-amylase is key in obtaining an alpha amylase which substantially maintains activity during passage through the gastro intestinal tract of an animal. Thus, pepsin resistant alpha-amylases can be used in lower doses compared with conventional alpha-amylases and may increase the starch utilisation of feed.

In another aspect of the present invention there is provided a method for preparing a feed supplement for a monogastric animal comprising admixing a pepsin resistant alpha amylase with at least one physiologically acceptable carrier selected from at least one of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof, wherein the pepsin resistant $\alpha$ amylase is as defined in claim 1 or claim 4.

In a further aspect of the present invention there is provided a feed supplement for monogastric animals comprising a pepsin resistant alpha amylase and at least one physiologically acceptable carrier selected from at least one of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof, wherein the pepsin resistant $\alpha$ amylase is as defined in claim 1 or claim 4.

The present inventor has surprisingly found that alpha-amylases from *Trichoderma* (such as *Trichoderma reesei*) are particularly effective for use in feed and are surprisingly pepsin resistant.

In a further aspect, the present invention provides a poultry feed supplement comprising a pepsin resistant alpha amylase, wherein the pepsin resistant $\alpha$ amylase is as defined in claim 1 or claim 4.

According to another aspect of the present invention there is provided: 1) a method for preparing a feedstuff for a monogastric animal comprising mixing a feed supplement of the present invention with one or more feed materials; and 2) feedstuffs prepared by said method.

The present invention further provides a feedstuff for a monogastric animal comprising a pepsin resistant alpha amylase, wherein said feedstuff comprises less than 3000 units alpha amylase per kilogram feed, preferably less than 2800, or less than 2600, or less than 2400 or less than 2200 or less than 2100 or less than 2000 units alpha amylase per kilogram feed, wherein the pepsin resistant $\alpha$ amylase is as defined in claim 1 or claim 4.

It will be understood that one amylase U is the amount of enzyme that releases 1 mmol of glucosidic linkages from a water insoluble cross-linked starch polymer substrate per min at pH 6.5 and 37°C.

The present invention further provides: a method of increasing weight gain in poultry or swine comprising feeding said poultry or swine a feedstuff comprising a pepsin resistant alpha amylase; and use of a pepsin resistant alpha amviase to reduce the expression of endoaenous alpha amviase in an animal, wherein said pepsin resistant $\alpha$ amylase is as defined in claim 1 or claim 4.

In another aspect of the present invention there is provided use of a feed supplement comprising a pepsin resistant alpha amylase for improving monogastric animal performance and/or increasing energy absorption and/or feed efficacy and/or for improving weight gain in an animal.

We describe herein a method for identifying a pepsin resistant alpha amylase enzyme for use in a feed supplement comprising:

i) providing an alpha amylase enzyme;
ii) admixing said alpha amylase with corn based feed, incubating at pH 3 and analysing the alpha amylase activity of said alpha amylase compared to a control sample; wherein said control sample differs in that no pepsin is present during the incubation at pH 3; and
iii) selecting an alpha amylase enzyme which substantially maintains alpha amylase activity under the assay conditions.

## PREFERED ASPECTS OF THE PRESENT INVENTION

Suitably, the level of pepsin in step ii) is at least about 9000 U/ml, at least about 10000 U/ml, at least 10500 U/ml, at least 11000 U/ml, at least 12000 U/ml, at least 13000 U/ml, at least 14000 U/ml, at least 15000 U/ml, at least 16000 U/ml, at least 17000 U/ml, at least 18000 U/ml, at least 19000 U/ml, at least 20000 U/ml, at least 21000 U/ml, at least 22000 U/ml or at least 23000 U/ml pepsin.

The alpha amylase may be incubated with pepsin for at least 2 hours, at least 2.5 hours, at least 3 hours or at least 3.5 hours. Preferably, the amylase is incubated, with pepsin for less than 6 hours.

The alpha amylase may be incubated with pepsin for about 2 to about 6 hours, preferably for about 2 to about 4 hours or for about 2 hours.

Suitably, the alpha amylase may retain at least about 80%, at least about 85%, at least about 90%, at least about 95%, at least about 97%, at least about 98%, at least about 99% activity compared with its activity after being incubated in the absence of pepsin. Suitably, the alpha amylase may have about 80-120%, about 85-115%, about 90-110%, about 95-105%, about 96-104%, about 97-103%, about 98-102%, or about 99-101% compared with its activity in the absence of pepsin. The alpha amylase may have the same activity compared with its activity after being incubated in the absence of pepsin.

In embodiments of the present invention set out below, the alpha amylase may suitably be, the alpha amylase as set forth in SEQ ID NO: 1. Suitably, the alpha amylase as set forth in SEQ ID NO: 1 may retain at least about 80%, at least about 85%, at least about 90%, at least about 95%, at least about 97%, at least about 98%, at least about 99% activity compared with its activity after being incubated in the absence of pepsin. Suitably, the alpha amylase as set forth in SEQ ID NO: 1 may have about 80-120%, about 85-115%, about 90-110%, about 95-105%, about 96-104%, about 97-103%, about 98-102%, or about 99-101 % compared with its activity in the absence of pepsin. The alpha amylase as set forth in SEQ ID NO: 1 may have the same activity compared with its activity after being incubated in the absence of pepsin.

By "absence of pepsin" it is meant that there is no pepsin specifically added (i.e. no exogenous pepsin is added to the control sample). However, it will readily understood that trace amounts of pepsin may be present in other constituents added, such as in the corn based feed.

The feed supplement may be for a monogastric animal, such as, for example, poultry, swine, domestic pets or fish. In some aspects, the monogastric animals are preferably poultry.

The terms "feed supplement" and "feed additive" as used herein are interchangeable.

The present invention provides a method for preparing a feed supplement for a monogastric animal comprising admixing a pepsin resistant alpha amylase with at least one physiologically acceptable carrier selected from at least one of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof, wherein the pepsin resistant $\alpha$ amylase is as defined in claim 1 or claim 4.

As described herein a feed supplement may be formulated as a premix. By way of example only the premix may comprise one or more feed components, such as one or more minerals and/or one or more vitamins.

The pepsin resistant amylase may be identified by using the method for identifying a pepsin resistant alpha amylase enzyme described herein.

The pepsin resistant amylase has an amino acid sequence:

i) as set forth in SEQ ID No. 1;
ii) having at least 85% (preferably, at least 90%, 95%, 97%, 98% or 99%) identity to SEQ ID No. 1;
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code;
v) which is produced by expression of a nucleotide sequence which has at least 70% (preferably, at least 75%, 80%, 85%, 90%, 95%, 97%, 98% or 99%) identity to SEQ ID No. 2, or on gap alignment with SEQ ID No. 1, the pepsin resistant alpha amylase of the present invention comprises any one or more of the following amino acids selected from the group consisting of: K88; I103; H133; Y175; Y290; F292; R442 and H450, wherein the amino acid numbering relates to SEQ ID No. 1.

Suitably, the pepsin resistant alpha amylase may comprise one or more of the following amino acid sequences:

i) SAIKSL (SEQ ID NO:7);
ii) DWINH (SEQ ID NO:8);
iii) SGEHLI (SEQ ID NO:9);
iv) NRIYKF (SEQ ID NO:10);
v) PLHYQFHA (SEQ ID NO:11);
vi) YVGRQN (SEQ ID NO:12); and
vii) ETWHDI (SEQ ID NO:13)

or may comprise an amino acid sequence having at least 80% or 85% or 90% identity to any of i) to vii).

Furthermore, the pepsin resistant alpha amylase may comprise one or more of the following amino acid sequences:

i) SAIKSL (SEQ ID NO:7); Position 85-90
ii) DVVINH (SEQ 10 NO:8); Position 100-105
iii) SGEHLI (SEQ ID NO:9); Position 130-135
iv) NRIYKF (SEQ ID NO:10); Position 172-177
v) PLHYQFHA (SEQ ID NO:11);Position 287-295
vi) YVGRQN (SEQ ID NO:12); Position 439-444
vii) ETWHDI (SEQ ID NO:13) Position 447-452

or may comprise an amino acid sequence having at least 80% or 85% or 90% identity to any of i) to vii), wherein the amino acid numbering relates to SEQ ID NO:1.

It will be understood that any combinations of i) to vii) and/or any combination of the specifically recited amino acids are encompassed herein.

The present invention provides a feed supplement for monogastric animals comprising a pepsin resistant alpha amylase and at least one physiologically acceptable carrier selected from at least one of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof is also provided wherein the pepsin resistant $\alpha$ amylase is as defined in claim 1 or claim 4.

Suitably, the pepsin resistant alpha amylases may be identified using the method for identifying a pepsin resistant alpha amylase enzyme disclosed herein.

Suitably, the alpha amylase may retain at least about 80%, at least about 85%, at least about 90%, at least about 95%, at least about 97%, at least about 98%, at least about 99% activity compared with its activity after being incubated in the absence of pepsin. Suitably, the alpha amylase may have about 80-120%, about 85-115%, about 90-110%, about 95-105%, about 96-104%, about 97-103%, about 98-102%, or about 99-101% compared with its activity in the absence of pepsin. The alpha amylase may have the same activity compared with its activity after being incubated in the absence of pepsin.

The pepsin resistant alpha amylase may has an amino acid sequence:

i) as set forth in SEQ ID No. 1;
ii) having at least 85% (preferably, at least 90%, 95%, 97%, 98% or 99%) identity to SEQ ID No. 1; or
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code; or
vii) which is produced by expression of a nucleotide sequence which has at least 70% (preferably, at least 75%, 80%, 85%, 90%, 95%, 97%, 98% or 99%) identity to SEQ ID No. 2, or

the pepsin resistant alpha amylase on gap alignment with SEQ ID No.1 comprises any one or more of the following amino acids selected from the group consisting of: K88; I103; H133; Y175; Y290; F292; R442 and H450, wherein the amino acid numbering relates to SEQ ID No. 1 and may comprise one or more of the following amino acid sequences:

i) SAIKSL (SEQ ID NO:7);
ii) DWINH (SEQ ID NO:8);
iii) SGEHLI (SEQ ID NO:9);
iv) NRIYKF (SEQ ID NO:10);
v) PLHYQFHA (SEQ ID NO:11);
vi) YVGRQN (SEQ ID NO:12); and
vii) ETWHDI (SEQ ID NO:13)

or may comprise an amino acid sequence having at least 80%, 85%, or 90% identity to any of i) to vii).

Furthermore, the pepsin resistant alpha amylase may comprise one or more of the following amino acid sequences:

i) SAIKSL (SEQ ID NO:7); Position 85-90
ii) DWINH (SEQ ID NO:8); Position 100-105
iii) SGEHLI (SEQ ID NO:9); Position 130-135
iv) NRIYKF (SEQ ID NO:10); Position 172-177
v) PLHYQFHA (SEQ ID NO:11); Position 287-295
vi) YVGRQN (SEQ ID NO:12); Position 439-444
vii) ETWHDI (SEQ ID NO:13) Position 447-452

or may comprise an amino acid sequence having at least 80% or 85% or 90% identity to any of i) to vii), wherein the amino acid numbering relates to SEQ ID NO:1.

It will be understood that any combinations of i) to vii) and/or any combination of the specifically recited amino acids are encompassed herein.

When the monogastric animal is poultry, it is advantageous for the pepsin resistant alpha amylase to substantially maintain pepsin resistance for at least 2 to 4 hours after consumption of the feed comprising the pepsin resistant alpha amylase.

When the monogastric animal is swine, it is advantageous for the pepsin resistant alpha amylase to substantially maintain pepsin resistance for at least 2 to 6 hours after consumption of the feed comprising the pepsin resistant alpha amylase.

The present invention also provides a method for preparing a feedstuff for a monogastric animal comprising mixing a feed supplement of the present invention with one or more feed materials.

Suitably, the alpha amylase may retain at least about 80%, at least about 85%, at least about 90%, at least about 95%, at least about 97%, at least about 98%, at least about 99% activity compared with its activity after being incubated in the absence of pepsin. Suitably, the alpha amylase may have about 80-120%, about 85-115%, about 90-110%, about 95-105%, about 96-104%, about 97-103%, about 98-102%, or about 99-101% compared with its activity in the absence of pepsin. The alpha amylase may have the same activity compared with its activity after being incubated in the absence of pepsin.

The pepsin resistant alpha amylase has an amino acid sequence:

i) as set forth in SEQ ID No. 1;
iii) having at least 85% (preferably, at least 90%, 95%, 97%, 98% or 99%) identity to SEQ ID No. 1; or
iv) which is produced by expression of a nucleotide sequence comprising the sequence of SEQ ID No. 2;
v) which is produced by expression of a nucleotide sequence which differs from SEQ ID No. 2 due to the degeneracy of the genetic code; or
vii) which is produced by expression of a nucleotide sequence which has at least 70% (preferably, at least 75%, 80%, 85%, 90%, 95%, 97%, 98% or 99%) identity to SEQ ID No. 2, or

the pepsin resistant alpha amylases on gap alignment with SEQ ID No. 1 comprises any one or more of the following amino acids selected from the group consisting of: K88; I103; H133; Y175; Y290; F292; R442 and H450, wherein the amino acid numbering relates to SEQ ID No. 1 and may comprise one or more of the following amino acid sequences:

i) SAIKSL (SEQ ID NO:7);
ii) DWINH (SEQ ID NO:8);
iii) SGEHLI (SEQ ID NO:9);
iv) NRIYKF (SEQ ID NO:10);
v) PLHYQFHA (SEQ ID NO:11);
vi) YVGRQN (SEQ ID NO:12); and

vii) ETWHDI (SEQ ID NO:13);

or may comprise an amino acid sequence having at least 80%, 85%, or 90% identity to any of i) to vii).

Furthermore, the pepsin resistant alpha amylase may comprise one or more of the following amino acid sequences:

i) SAIKSL (SEQ ID NO:7); Position 85-90
ii) DWINH (SEQ ID NO:8); Position 100-105
iii) SGEHLI (SEQ ID NO:9); Position 130-135
iv) NRIYKF (SEQ ID NO:10); Position 172-177
v) PLHYQFHA (SEQ ID NO:11); Position 287-295
vi) YVGRQN (SEQ ID NO:12); Position 439-444
vii) ETWHDI (SEQ ID NO:13) Position 447-452

or may comprise an amino acid sequence having at least 80% or 85% or 90% identity to any of i) to vii), wherein the amino acid numbering relates to SEQ ID NO:1.

It will be understood that any combinations of i) to vii) and/or any combination of the specifically recited amino acids are encompassed herein.

In some embodiments said feed supplement/pepsin resistant alpha amylase improves monogastric animal performance and/or increases energy absorption/feed efficacy and/or weight gain of the monogastric animal and/or. Any feed materials may be used. Suitably, any one or more of the following feed materials may be used: a) cereals, such as small grains (e.g., wheat, barley, rye, oats and combinations thereof) and/or large grains such as maize or sorghum; b) by-products from cereals, such as corn gluten meal, Distillers Dried Grain Solubles (DDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone metal, potato protein, whey, copra, sesame; d) oils and fats obtained from vegetable and animal sources; e) minerals and vitamins f) premixes of any one or more of a) to d).

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

The feedstuff comprises less than 3000, less than 2000, less than 1900, less than 1800, less than 1700, less than 1600, less than 1500, iess than 1400, less than 1300, less than 1200, less than 1100, iess than 1000, iess than 900, less than 800, less than 700, less than 600, less than 500, or less than about 400 units of alpha amylase per kilogram feed.

The present invention further provides a feedstuff for monogastric animals prepared by a method comprising mixing a feed supplement of the present invention with one or more feed materials.

Suitably, the alpha amylase may retain at least about 80%, at least about 85%, at least about 90%, at least about 95%, at least about 97%, at least about 98%, at least about 99% activity compared with its activity after being incubated in the absence of pepsin. Suitably, the alpha amylase may have about 80-120%, about 85-115%, about 90-110%, about 95-105%, about 96-104%, about 97-103%, about 98-102%, or about 99-101% compared with its activity in the absence of pepsin. The alpha amylase may have the same activity compared with its activity after being incubated in the absence of pepsin.

The pepsin resistant alpha amylase has an amino acid sequence:

i) as set forth in SEQ ID No. 1;
iii) having at least 85% (preferably, at least 90%, 95%, 97%, 98% or 99%) identity to SEQ ID No. 1; or
iv) which is produced by expression of a nucleotide sequence comprising the sequence of SEQ ID No. 2;
v) which is produced by expression of a nucleotide sequence which differs from SEQ ID No. 2 due to the degeneracy of the genetic code;
vii) which is produced by expression of a nucleotide sequence which has at least 70% (preferably, at least 75%, 80%, 85%, 90%, 95%, 97%, 98% or 99%) identity to SEQ ID No. 2, or

the pepsin resistant alpha amylase on gap alignment with SEQ ID No. 1 comprises any one or more of the following amino acids selected from the group consisting of: K88; I103; H133; Y175; Y290; F292; R442 and H450, wherein the amino acid numbering relates to SEQ ID No. 1 and/or may comprise one or more of the following amino acid sequences:

i) SAIKSL (SEQ ID NO:7);

ii) D\/VINH (SEQ ID NO:8);
iii) SGEHLI (SEQ ID NO:9);
iv) NRIYKF (SEQ ID NO:10);
v) PLHYQFHA (SEQ ID NO:11);
vi) YVGRQN (SEQ ID NO:12); and
vii) ETWHDI (SEQ ID NO:13)

or may comprise an amino acid sequence having at least 80%, 85%, or 90% identity to any of i) to vii).

Furthermore, the pepsin resistant alpha amylase may comprise one or more of the following amino acid sequences:

i) SAIKSL (SEQ ID NO:7); Position 85-90
ii) DWINH (SEQ ID NO:8); Position 100-105
iii) SGEHLI (SEQ ID NO:9); Position 130-135
iv) NRIYKF (SEQ ID NO:10); Position 172-177
v) PLHYQFHA (SEQ ID NO:11); Position 287-295
vi) YVGRQN (SEQ ID NO:12); Position 439-444
vii) ETWHDI (SEQ ID NO:13) Position 447-452

or may comprise an amino acid sequence having at least 80% or 85% or 90% identity to any of i) to vii), wherein the amino acid numbering relates to SEQ ID NO:1.

It will be understood that any combinations of i) to vii) and/or any combination of the specifically recited amino acids are encompassed herein.

The feedstuff comprises less than 3000, less than 2000, less than 1900, less than 1800, less than 1700, less than 1600, less than 1500, less than 1400, less than 1300, less than 1200, less than 1100, less than 1000, less than 900, less than 800, less than 700, less than 600, less than 500, or less than about 400 units of alpha amylase per kilogram feed.

In some embodiments said feed supplement improves monogastric animal performance and/or increases energy absorption/feed efficacy and/or weight gain of the monogastric animal.

The present invention also provides a poultry feed supplement and/ or feedstuff comprising a pepsin resistant alpha amylase wherein the pepsin resistant $\alpha$ amylase is as defined in claim 1 or claim 4. The pepsin resistant alpha amylase has an amino acid sequence:

i) as set forth in SEQ ID No. 1;
ii) having at least 85% (preferably, at least 90%, 95%, 97%, 98% or 99%) identity to SEQ ID No. 1; or
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code; or
v) which is produced by expression of a nucleotide sequence which has at least 70% (preferably, at least 75%, 80%, 85%, 90%, 95%, 97%, 98% or 99%) identity to SEQ ID No. 2; or

the pepsin resistant alpha amylase on gap alignment with SEQ ID No. 1 comprises any one or more of the following amino acids selected from the group consisting of: K88; I103; H133; Y175; Y290; F292; R442 and H450, wherein the amino acid numbering relates to SEQ ID No. 1 and/or may comprise one or more of the following amino acid sequences:

i) SAIKSL (SEQ ID NO:7);
ii) DWINH (SEQ ID NO:8);
iii) SGEHLI (SEQ ID NO:9);
iv) NRIYKF (SEQ ID NO:10)
v) PLHYQFHA (SEQ ID NO:11);
vi) YVGRQN (SEQ ID NO:12); and
vii) ETWHDI (SEQ ID NO:13)

or may comprise an amino acid sequence having at least 80%, 85%, or 90% identity to any of i) to vii).

Furthermore, the pepsin resistant alpha amylase may comprise one or more of the following amino acid sequences:

i) SAIKSL (SEQ ID NO:7); Position 85-90

ii) DWINH (SEQ ID NO:8); Position 100-105
iii) SGEHLI (SEQ ID NO:9); Position 130-135
iv) NRIYKF (SEQ ID NO:10); Position 172-177
v) PLHYQFHA (SEQ ID NO:11); Position 287-295
vi) YVGRQN (SEQ ID NO:12); Position 439-444
vii) ETWHDI (SEQ ID NO:13) Position 447-452

or may comprise an amino acid sequence having at least 80% or 85% or 90% identity to any of i) to vii), wherein the amino acid numbering relates to SEQ ID NO:1.

It will be understood that any combinations of i) to vii) and/or any combination of the specifically recited amino acids are encompassed herein.

Without wishing to be bound by theory, one or more of the recited amino acids and sequences above may play a role in conferring pepsin resistance to the alpha amylase enzyme.

The feedstuff comprises less than 3000, less than 2000, less than 1900, less than 1800, less than 1700, less than 1600, less than 1500, less than 1400, less than 1300, less than 1200, less than 1100, less than 1000, less than 900, less than 800, less than 700, less than 600, less than 500, or less than about 400 units of alpha amylase per kilogram feed.

Suitably, the alpha amylase may retain at least about 80%, at least about 85%, at least about 90%, at least about 95%, at least about 97%, at least about 98%, at least about 99% activity compared with its activity after being incubated in the absence of pepsin. Suitably, the alpha amylase may have about 80-120%, about 85-115%, about 90-110%, about 95-105%, about 96-104%, about 97-103%, about 98-102%, or about 99-101 % compared with its activity in the absence of pepsin. The alpha amylase may have the same activity compared with its activity after being incubated in the absence of pepsin.

In some embodiments said feed supplement improves monogastric animal performance and/or increases energy absorption/feed efficacy and/or weight gain of the monogastric animal.

The present invention further provides the use of a feed supplement according to the present invention for improving animal performance and/or increasing energy absorption and/or feed efficacy and/or for improving weight gain in a monogastric animal.

In another aspect of the present invention, there is provided a feedstuff for a monogastric animal comprising a pepsin resistant alpha amylase, wherein said feedstuff comprises less than 3000 units of alpha amylase per kilogram feed. Suitably, the feedstuff may comprise less than 2500, less than 2000, less than 1900, less than 1800, less than 1700, less than 1600, less than 1500, less than 1400, less than 1300, less than 1200, less than 1100, less than 1000, less than 900, less than 800, less than 700, less than 600, less than 500, or less than about 400 units alpha amylase per kilogram feed, wherein said pepsin resistant $\alpha$ amylase is as defined in claim 1 or claim 4.

In some embodiments feedstuff improves monogastric animal performance and/or increases energy absorption/feed efficacy.

The pepsin resistant alpha amylase has an amino acid sequence:

i) as set forth in SEQ ID No. 1;
ii) having at least 85% (preferably, at least 90%, 95%, 97%, 98% or 99%) identity to SEQ ID No. 1;
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code;
v) which is produced by expression of a nucleotide sequence which has at least 70% (preferably, at least 75%, 80%, 85%, 90%, 95%, 97%, 98% or 99%) identity to SEQ ID No. 2, or as defined in claim 4.

Suitably, the alpha amylase may retain at least about 80%, at least about 85%, at least about 90%, at least about 95%, at least about 97%, at least about 98%, at least about 99% activity compared with its activity after being incubated in the absence of pepsin. Suitably, the alpha amylase may have about 80-120%, about 85-115%, about 90-110%, about 95-105%, about 96-104%, about 97-103%, about 98-102%, or about 99-101% compared with its activity in the absence of pepsin. The alpha amylase may have the same activity compared with its activity after being incubated in the absence of pepsin.

Feedstuffs and/or feed supplements in accordance with the present invention may comprise at least one further feed enzyme. For example, the feedstuff and/or feed supplement may comprise enzymes involved in starch metabolism, fibre degradation, lipid metabolism, proteins or enzymes involved in glycogen metabolism, acetyl esterases, aminopeptidases, amylases, arabinases, arabinofuranosidases, carboxypeptidases, catalases, cellulases, chitinases, chymosin, cutinase, deoxyribonucleases, epimerases, esterases, $\alpha$-galactosidases, $\beta$-glucanases, glucan lysases, endo-glucanases, glucoamylases, glucose oxidases, $\beta$-glucosidases, including $\beta$-glucosidase, glucuronidases, hemicellulases, hex-

ose oxidases, hydrolases, invertases, isomerases, laccases, lyases, mannosidases, oxidases, oxidoreductases, pectate lyases, pectin acetyl esterases, pectin depolymerases, pectin methyl esterases, pectinolytic enzymes, peroxidases, phenoloxidases, polygalacturonases, proteases, rhamno-galacturonases, ribonucleases, thaumatin, transferases, transport proteins, transglutaminases, xylanases, including endo-1,4-β-xylanase (EC 3.2.1.8), hexose oxidase (D-hexose: $O_2$-oxidoreductase EC 1.1.3.5) β-glucanase, α-amylase, pectinase, cellobiohydrolase, acid phosphatases, phytases, including 3-phytase (EC 3.1.3.8) or 6-phytase (EC 3.1.3.26), lipolytic enzymes, mannanase or combinations thereof. These include enzymes that, for example, modulate the viscosity of the feed.

Suitably, a feedstuff and/or feed supplement of the present invention may comprise at least one xylanase and/or at least one phytase and/or at least one protease and/or at least one lipolytic enzyme. These may be from any source. Suitably, a xylanase may be from *Bacillus* or *Trichoderma;* a phytase may be from *E. coli* or *Buttiauxella;* a protease may be from *B. subtilis* and a lipolytic enzyme may be from *Aspergillus* sp.

The term 'lipolytic enzyme' refers to an enzyme capable of acting on a lipid substrate to liberate a free fatty acid molecule. Preferably, the lipolytic enzyme is an enzyme capable of hydrolysing an ester bond in a lipid substrate (particularly although not exclusively a triglyceride, a glycolipid and/or a phospholipid) to liberate a free fatty acid molecule. Suitably, the lipolytic enzyme for use in the present invention may have one or more of the following activities selected from the group consisting of: phospholipase activity (such as phospholipase A1 activity (E.C. 3.1.1.32) or phospholipase A2 activity (E.C. 3.1.1.4); glycolipase activity (E.C. 3.1.1.26), triacylglycerol hydrolysing activity (E.C. 3.1.1.3), lipid acyltransferase activity(generally classified as E.C. 2.3.1.x in accordance with the Enzyme Nomenclature Recommendations (1992) of the Nomenclature Committee of the International Union of Biochemistry and Molecular Biology), and any combination thereof.

Suitably, a feedstuff and/or feed supplement of the present invention may comprise a pepsin resistant alpha amylase from any source (such as *B. licheniformis* or *T. reesei*), a xylanase from *Bacillus* or *Trichoderma*; a phytase from *E. coli* or *Buttiauxella;* a protease from *B. subtilis* and a lipolytic enzyme from *Aspergillus* sp.

Any feedstuff of the present invention may comprise one or more feed materials selected from the group comprising a) cereals, such as small grains (e.g., wheat, barley, rye, oats and combinations thereof) and/or large grains such as maize or sorghum; b) by-products from cereals, such as corn gluten meal, Distillers Dried Grain Solubles (DDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canoia, fish meal, dried plasma protein, meat and bone meai, potato protein, whey, copra, sesame; d) oils and fats obtained from vegetable and animal sources; e) minerals and vitamins.

Suitably, any feedstuff of the present invention may comprise at least one low fibre feed material, selected from the group consisting of corn, wheat, an animal by-product meal or soybean and/or at least one by-product of the at least one low fibre feed material to provide a low fibre feedstuff.

Any feedstuff of the present invention may contain at least 30%, at least 40%, at least 50% or at least 60% by weight of corn and soybean meal or corn and full fat soy.

In addition or in the alternative, any feedstuff of the present invention may comprise at least one high fibre feed material and/or at least one by-product of the at least one high fibre feed material to provide a high fibre feedstuff. Examples of high fibre feed materials include: wheat, barley, rye, oats, by-products from cereals, such as corn gluten meal, Distillers Dried Grain Solubles (DDGS), wheat bran, wheat middlings, wheat shorts, rice bran, rice hulls, oat hulls, palm kernel, and citrus pulp. Some protein sources may also be regarded as high fibre: protein obtained from sources such as sunflower, lupin, fava beans and cotton.

As used herein, "animal performance" may be determined by the feed efficacy and/or growth rate of the animal and/or by the feed conversion ratio and/or weight gain of the animal and/or by the digestibility of a nutrient in a feed (e.g. starch digestibility) and/or digestible energy or metabolizable energy in a feed.

Preferably "animal performance" is determined by feed efficiency and/or weight gain of the animal and/or by the feed conversion ratio.

By "improved animal performance" it is meant that there is increased feed efficacy, and/or increased growth rate and/or reduced feed conversion ratio and/or weight gain of the animal and/or by the digestibility of a nutrient in a feed (e.g. starch digestibility) and/or digestible energy or metabolizable energy in a feed resulting from the use of a pepsin resistant alpha amylase in feed in comparison to feed which does not comprise a pepsin resistant alpha amylase. In preferred embodiments, at least one of feed efficacy, and/or growth rate and/or feed conversion ratio and/or weight gain of the animal and/or by the digestibility of a nutrient in a feed (e.g. starch digestibility) and/or digestible energy or metabolizable energy in a feed is improved by at least 1%, such as 1.5%, such as 2.0% such as 2.5%, such as 3.0%, such as 4%, such as 5% in an animal fed a feed comprising the pepsin resistant alpha amylase when compared to an animal fed a feed not comprising the pepsin resistant alpha amylase.

As used herein, the term "feed efficacy" refers to the amount of weight gain in an animal that occurs when the animal is fed a specified amount of food.

By "increased feed efficacy" it is meant that the use of a pepsin resistant alpha amylase in feed results in an increased weight gain on feeding an animal compared with an animal being fed the same amount of feed without a pepsin resistant alpha amylase being present.

As used herein, the term "feed conversion ratio" refers to the amount of feed fed to an animal to increase the weight of the animal by a specified amount.

By "lower feed conversion ratio" it is meant that the use of a pepsin resistant alpha amylase in feed results in a lower amount of feed being required to be fed to an animal to increase the weight of the animal by a specified amount compared to the amount of feed required to increase the weight of the animal by the same amount when the feed does not comprise a pepsin resistant alpha amylase.

Nutrient digestibility as used herein means the fraction of a nutrient that disappears from the gastro-intestinal tract or a specified segment of the gastro-intestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal trace, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastrointestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

The term survival as used herein means the number of subject remaining alive. The term "improved survival" may be another way of saying "reduced mortality".

The term carcass yield as used herein means the amount of carcass as a proportion of the live body weight, after a commercial or experimental process of slaughter. The term carcass means the body of an animal that has been slaughtered for food, with the head, entrails, part of the limbs, and feathers or skin removed. The term meat yield as used herein means the amount of edible meat as a proportion of the live body weight, or the amount of a specified meat cut as a proportion of the live body weight.

The present invention further provides a method of increasing weight gain in poultry or swine comprising feeding said poultry or swine a feedstuff comprising a pepsin resistant alpha amylase as defined in claim 1 or claim 4.

An "increased weight gain" refers to an animal having increased weight on being fed feed comprising a pepsin resistant alpha amylase compared with an animal being fed a feed without a pepsin resistant alpha amylase being present.

The pepsin resistant alpha amylase has an amino acid sequence:

i) as set forth in SEQ ID No. 1;
ii) having at least 85% (preferably, at least 90%, 95%, 97%, 98% or 99%) identity to SEQ ID No. 1; or
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code;
v) which is produced by expression of a nucleotide sequence which has at least 70% (preferably, at least 75%, 80%, 85%, 90%, 95%, 97%, 98% or 99%) identity to SEQ ID No. 2; or

the pepsin resistant alpha amylase on gap alignment with SEQ ID No. 1 comprises any one or more of the following amino acids selected from the group consisting of: K88; I103; H133; Y175; Y290; F292; R442 and H450, wherein the amino acid numbering relates to SEQ ID No. 1 and may comprise one or more of the following amino acid sequences:

i) SAIKSL (SEQ ID NO:7);
ii) DVVINH (SEQ ID NO:8);
iii) SGEHLI (SEQ ID NO:9);
iv) NRIYKF (SEQ ID NO:10);
v) PLHYQFHA (SEQ ID NO:11);

vi) YVGRQN (SEQ ID NO:12); and
vii) ETWHDI (SEQ ID NO:13)

or may comprise an amino acid sequence having at least 80%, 85%, or 90% identity to any of i) to vii).
Furthermore, the pepsin resistant alpha amylase may comprise one or more of the following amino acid sequences:

i) SAIKSL (SEQ ID NO:7); Position 85-90
ii) DVVINH (SEQ ID NO:8); Position 100-105
iii) SGEHLI (SEQ ID NO:9); Position 130-135
iv) NRIYKF (SEQ ID NO:10); Position 172-177
v) PLHYQFHA (SEQ ID NO:11); Position 287-295
vi) YVGRQN (SEQ ID NO:12); Position 439-444
vii) ETWHDI (SEQ ID NO:13) Position 447-452

or may comprise an amino acid sequence having at least 80% or 85% or 90% identity to any of i) to vii), wherein the amino acid numbering relates to SEQ ID NO:1.
It will be understood that any combinations of i) to vii) and/or any combination of the specifically recited amino acids are encompassed herein.
The feedstuff comprises less than 3000, less than 2000, less than 1900, less than 1800, less than 1700, less than 1600, less than 1500, less than 1400, less than 1300, less than 1200, less than 1100, less than 1000, less than 900, less than 800, less than 700, less than 600, less than 500, or less than about 400 units of alpha amylase per kilogram feed.
Suitably, the alpha amylase may retain at least about 80%, at least about 85%, at least about 90%, at least about 95%, at least about 97%, at least about 98%, at least about 99% activity compared with its activity after being incubated in the absence of pepsin. Suitably, the alpha amylase may have about 80-120%, about 85-115%, about 90-110%, about 95-105%, about 96-104%, about 97-103%, about 98-102%, or about 99-101 % compared with its activity in the absence of pepsin. The alpha amylase may have the same activity compared with its activity after being incubated in the absence of pepsin.
It will be understood that any of the preferred features disclosed herein are considered to be equally applicable to any of the aspects described above unless explicitly stated otherwise. Any preferred feature is also considered to be disclosed in combination with any other preferred feature disclosed herein.

**PEPSIN RESISTANCE ASSAY**

This assay comprises the following protocol: An alpha amylase (e.g. 100 μl enzyme solution) is admixed with corn based feed (e.g. 100 mg) and pepsin (e.g. at a concentration of at least 9000 U/ml which may be added in a volume of 900μl) and incubated at pH 3 (e.g. for at least about 120 mins). In addition, said alpha amylase is admixed with the same corn based feed and in the absence of pepsin under the same conditions as a control. Subsequently the alpha amylase activity for the sample and the control are determined.
Without wishing to be bound by theory, the inventor believes that the presence of the corn based feed may affect the pepsin resistance of an alpha amylase. The conditions used in the pepsin assay have been made to mimic the conditions used in the gastro intestinal tract of a monogastric animal.
A pepsin level of about 9000 U/ml may be used and the incubation time may be about 120 minutes. Amylases which substantially maintain aipna amylase activity unaer such conditions may be considered pepsin resistant alpha amylases . However, it will be understood that the assay is a balance of the incubation time and the pepsin level. Thus, by increasing the incubation time a lower pepsin level may be used to identify alpha amylases which are pepsin resistant. Likewise, by decreasing the incubation time, a higher pepsin level may be used to identify alpha amylases which are pepsin resistant.

*Pepsin level and incubation time:*

*Temperature:*

It will be understood that any temperature in which both the pepsin resistant alpha amylase and the pepsin are active may be used. For example, a temperature of 30-50°C may be used, preferably 40°C.

Buffer:

In the assay a buffer solution (e.g. 900 μl) may be used. For example, a buffer solution comprising pepsin may be used and a buffer solution which does not comprise pepsin may be used for the control.

It will be understood that any suitable buffer may be used which will provide the required pH. The composition of such buffers is common general knowledge to a person skilled in the art. An Example of a suitable pepsin incubation buffer is 0.1 M Glycine-HCl, pH 3.0, 3 mg/ml BSA, 2.9 mg Sodium chloride anhydrous/ml, 0.73 mg calcium chloride/ml. For solutions with pepsin, the incubation buffer is prepared to contain at least 9000 U/ml, preferably at least 10000 U/ml, preferably at least 10500 U/ml, preferably at least 11000 U/ml. Suitably, the incubation buffer may contain 9000 or 9250 U/ml.

By way of illustration, a buffer comprising 25 mg/ml (9250 U/ml) of pepsin (e.g. Sigma P-7000) could be made.

One pepsin unit is defined as the amount of enzyme that will produce a $\Delta OD_{280}$ of 0.001 per min at pH 2.0 at 37°C, measured as TCA-soluble products using haemoglobin as substrate (as described in e.g. Food Chemical Codex).

A positive control may also be prepared by admixing with the same corn based feed and a buffer solution of pH 5.6 wherein the buffer solution does not comprise pepsin and additionally comprises BSA.

For example, a suitable assay buffer for the positive control couid be Amylase assay buffer with BSA: Phosphate-citrate buffer 0,1 M, pH 5.6, 3mg/ml BSA.

In addition, further samples may be prepared without the alpha amylase being added to check the background absorbance from the chemicals used.

Suitably both samples and control samples may be prepared in duplicate.

Thus, the sample may be prepared and incubated as follows. In each 1,5 ml micro-centrifuge tube (Eppendorf), 100 mg of corn based feed is weighed out. A volume of 900 μl incubation buffer without or with the desired level of pepsin (i.e. at least 9000 U/ml) or 900 μl assay buffer (i.e. for the positive control) are added and pre-incubated in an Eppendorf Thermomixer 5436 at 500 rpm for 5 mins.

A volume of 100 μl enzyme solution (or 100 μl $H_20$) is added to each tube, the lids are closed, the tubes are incubated at 40°C in the Eppendorf Thermomixer 5436 at 500 rpm for the desired length of time (i.e. at least 120 minutes, e.g. 120 minutes exactly).

The samples are then analysed for alpha amylases activity.

An alpha amylase is considered to be "pepsin resistant" or to "substantially maintain alpha amylase activity" if the activity of the amylase in the sample comprising pepsin is in the range of at least about 75% (such as at least about 80%, 85%, 90%, 95%, 97%, 98% or 99%) to about 125% (or about 120%, 115%, 110%, 105%, 103%, 102% or 101%) when compared with the activity of the amylase in the control sample, i.e. without the presence of pepsin.

Accordingly, in useful embodiments, the activity of the amylase as set forth in SEQ ID NO:1 in the sample comprising pepsin is in the range of at least about 75% (such as at least about 80%, 85%, 90%, 95%, 97%, 98% or 99%) to about 125% (or about 120%, 115%, 110%, 105%, 103%, 102% or 101 %) when compared with the activity of said amylase in the control sample, i.e. without the presence of pepsin.

We describe herein the activity of the amylase as set forth in SEQ ID NO:3 in the sample comprising pepsin which may be in the range of at least about 75% (such as at least about 80%, 85%, 90%, 95%, 97%, 98% or 99%) to about 125% (or about 120%, 115%, 110%, 105%, 103%, 102% or 101%) when compared with the activity of said amylase in the control sample, i.e. without the presence of pepsin.

The present invention may encompass any combination of lower and upper limits between at least about 75% and about 125%. Thus, for example "pepsin resistant alpha amylase" or an alpha amylase considered to "substantially maintain" its activity may have at least about 80-120%, 85-115%, 90-110%, 95-105%, 96-104%, 97-103%, 98-102%, or 99-101% compared with its activity after incubation in the absence of pepsin. Accordingly, in useful embodiments, the activity of the amylase as set forth in SEQ ID NO:1 in the sample comprising pepsin is between 75% and about 125%, such as at least about 80-120%, 85-115%, 90-110%, 95-105%, 96-104%, 97-103%, 98-102%, or 99-101% compared with its activity after incubation in the absence of pepsin. We describe herein the activity of the amylase as set forth in SEQ ID NO:3 in the sample comprising pepsin is between 75% and about 125%, such as at least about 80-120%, 85-115%, 90-110%, 95-105%, 96-104%, 97-103%, 98-102%, or 99-101% compared with its activity after incubation in the absence of pepsin. The alpha amylase may have the same activity compared with its activity after incubation in the absence of pepsin.

The level of pepsin used in the assay is at least 9000 U/ml. Suitably, the level of pepsin used may be at least about 10000 U/ml, at least 10500 U/ml, at least 11000 U/ml, at least 12000 U/ml, at least 13000 U/ml, at least 14000 U/ml, at least 15000 U/ml, at least 16000 U/ml, at least 17000 U/ml, at least 18000 U/ml, at least 19000 U/ml, at least 20000 U/ml, at least 21000 U/ml, at least 22000 U/ml or at least 23000 U/ml pepsin.

The alpha amylases may be incubated with pepsin for at least about 2.5 hours, at least about 3 hours or at least about 3.5 hours.

Analysis of the activity of alpha amylase in the sample may be determined by any method known in the art.

The activity of alpha amylase could be measured using the KoneLab assay. The tubes are be spun down in a Eppendorf table centrifuge for 2 mins, 100 μl is withdrawn and mixed with 900 μl assay buffer (e.g. Amylase assay buffer: Phosphate-citrate buffer 0,1M, pH 5.6.). Samples are immediately analysed for activity in a KoneLab Arena 20XT (from Thermo Electron Corporation). Thus, pepsin resistance at a given pepsin concentration is defined as the activity of the amylase measured by the KoneLab assay after being incubated at the given pepsin concentration at pH 3 for at least two hours as described in the above protocol measured relative to the activity measured by the KoneLab assay after being incubated without pepsin at pH 3 for the same time as described in the above protocol.

Alternatively, the alpha amylase activity could be measured using the tablet assay described below.

## KONELAB ASSAY

The KoneLab assay is a colorimetric method for determination of bacterial alpha-amylase activity in liquid and solid products using a KoneLab-robot (KoneLab Arena 20 XT).

### Application and principles

An aliquot of product extract is incubated with Ceralpha-substrate mixture under defined conditions. The reaction is terminated by addition of a Tris-solution (Trizma base) and a yellow colour develops. The absorbance at 405 nm is measured and this relates directly to the level of alpha-amylase in the sample analysed. The enzyme activity of a sample is determined quantitatively by relating the absorbance measurement to that of a calibration enzyme with well-defined activity.

Bonds within the substrate can only be cleaved by alpha-amylase. The non-blocked reaction product is cleaved to glucose and free *p*-nitrophenyl by the excess quantities of glucoamylase and alpha-glucosidase which are part of the substrate mixture. The reaction releases free *p*-nitrophenyl and a yellow colour is developed on addition of Trizma-base.

The Ceralpha-substrate is a blocked *p*-nitrophenyl maltoheptaoside, BPNPG7, which do not distinguish between fungal and bacterial alpha-amylases.

Due to the pH and temperature sensitivities of the amyloglucosidase and alpha-glucosidase, the assay can be used only in the pH-range 5.0 to 6.0 and at 40°C or below.

### Apparatus

Glass filters, Advantec Toyo GA55, 110 mm
Magnetic stirrer
Various pipettes
Various test tubes
Various volumetric flasks
Test tube shaker
Konelab Arena 20 XT robot

### Chemicals

Citric acid monohydrate (Merck)
di-Sodium hydrogen phosphate (Merck)
Calcium chloride 2 aq (Merck)
Sodium chloride anhydrous (Merck)
Albumin from bovine serum (BSA, Sigma A 7906)
Cysteine (Merck 2838)
Tris(hydroxymethyl)aminomethane (Merck)

### Reagents

1. Stability reagent

Dissolve:

0.20 g Albumin from bovine serum (BSA),
0.05 g Cysteine,
2.0 g Sodium chloride anhydrous in 10 mL deionised water.

Keep refrigerated or frozen. Shelf life: refrigerated: 1 week at 5 +/- 3°C and frozen: 1 year at -18°C (can be re-refrigerated after thawing).

2. Assay buffer: Citric-phosphate buffer, 0.1 M, pH = 5.60

Dissolve:

4.41 g Citric acid monohydrate,
10.3 g di-Sodium hydrogen phosphate dihydrate,
2.90 g Sodium chloride anhydrous,
0.73 g Calcium chloride dihydrate in approx. 800 mL of deionised water.
1.00 mL stability-reagent (1) is added while stirring on magnetic stirrer.

When dissolved, pH is adjusted to 5.60 (HCl or NaOH) and the solution is transferred to a 1000 mL volumetric flask and adjust to 1000 mL with distilled water.

3. Stop solution : 1 % (w/v) TRIS (Trizma base)

Dissolve 10 g of TRIS ((hydroxymethyl)aminomethane) in deionised water in a volumetric flask and adjust to 1000 mL.

4. Substrate (Freeze-dried Ceralpha (BPNPG7) from Megazyme)

A brown bottle of cereal alpha-amylase assay reagent (BPNPG 7) is dissolved in 10.0 ml distilled water (2).

The solution is kept frozen at -18°C in brown bottle (10 ml batch)

Before use the solution is further diluted 1:1 also with distilled water.

1 bottle of Ceralpha contains 54.5 mg BPNPG7 and 125 U (pH = 6.0) alpha-glucosidase.

5. Control /Standard sample,

An amylase (LAT) standard: e.g. 485 TAU/g (Lot#102-05208-001)

Furthermore a LAT control sample e.g. (Lot#102-01128-lab) with a range of 7957-8162 TAU/g.

**Procedure**

**Preparation of Amylase standard curve**

An Amylase standard (5) is diluted to a concentration of approx. 1.9 U/g for LAT.
All dilutions are carried out in Assay buffer (2).
Further dilutions are programmed in Konelab:

| Standard | Dil. Ratio | Concentration, U/mL |
|---|---|---|
| 1 | 1+49.0 | 0.034 |
| 2 | 1+19.0 | 0.085 |
| 3 | 1+9.0 | 0.170 |
| 4 | 1+5.0 | 0.283 |
| 5 | 1+3.0 | 0.425 |

OD range should be between 0.2 and 1.5

**Sample preparation**

2 weighings are carried out for each sample.

*Liquid products:* 0.5 g of sample is weighed in a 50 ml volumetric flask. The Flask is filled with assay buffer (2) and mixed. Further dilutions are also carried out in Assay buffer (2). Final concentrations should be approx. 0.2 U/ml.

*Solid products:* 0.5 g of sample is weighed in a 50 ml volumetric flask and diluted in approx. 40 ml of Assay buffer (2). The solution is mixed on a magnetic stirrer for 10 minutes and filled up with buffer. The solution is filtered through a whatman glass filter and further dilutions are carried out in Assay buffer (2). Final concentrations should be approx. 0.2 U/ml.

*Binds:* 2 blind samples (Assay buffer (2)) are included in each run.

**Reaction conditions in the assay**

pH = 5.60
incubation temperature = 37°C +/- 0.1 °C
Wavelength = 405 nm

Substrate 50 μl
Pre-incubation 5 min
Sample 10 μl
incubation 15 min
Stop solution 100 μl

**Calculation of enzyme activity**

The activity of a sample is calculated according to the formula:

$$\text{Activity , U/g} = \frac{(OD_{sample} - \beta) * DF}{\alpha * W_{sample}}$$

OD sample = absorbance of the enzyme sample
DF = dilution factor for the sample
$W_{sample}$ = weight of sample in g
$\alpha$ = Slope of the standardcurve
$\beta$ = Intercept of standardcurve

**Quality Control (QC)**

The assay has to be redone if measured activity of the double determination has a CV% above 10%.

**TABLET ASSAY**

**Application and principles**

The enzyme activity is determined by measurement of the rate of enzymatic release of dyed oligomers from azurine-crosslinked starch. The enzyme activity of a sample is determined by relating the absorbance measurement to that of a calibration enzyme with well-defined activity.

**Apparatus**

Glass filter, Advantec Toyo GA55, 110 mm

Magnetic stirrer
Various pipettes
Test tubes
Water bath, 37°C
Stopwatch
Test tube shaker
Spectrophotometer, Shimadzu UV-160A, Shimadzu Europa GmbH

**Reagents**

1. Substrate

Phadebas Amylase test (Magle AB, Lund, Sweden). Each tablet contains 45 mg blue starch and buffer.

2. Assay buffer

Dilute 9.0 g of anhydrous sodium cloride (NaCl), 2.0 g of bovine serum albumin and 2.2 g of calcium chloride dihydrate ($CaCl_2$,$2H_2O$) in distilled water in a volumetric flask and fill to 1000 ml with distilled water.

3. Stop solution

0.5 M NaOH solution.
Dissolve 20.0 g of sodium hydroxide in distilled water in a volumetric flask and fill with distilled water to 1000 ml.

**Control sample**

Control samples with known activity and standard variation can be analysed in each run as QC check.

**Sample preparation**

For each sample, about 1-5 g is weighed precisely in duplicate. The samples are extracted in 100 ml assay buffer for 30 minutes with magnetic stirring.
The extract is filtered through a glass filter. Hereafter samples are diluted in assay buffer to an expected activity of about 0.3 U/ml (this dilution is to be added in the formula below).

**Assay procedure**

For each sample 200 μl extract and 4 ml assay buffer is pipetted into a test tube. All samples are analysed in duplicate. The solutions are equilibrated at 37°C for 5 minutes and at t = 0 minutes a Phadebas™ tablet [Magle Life Sciences, Lund, Sweden] is added and mixed well for 10 seconds. The samples are incubated at 37°C for 15 minutes and subsequently the reactions are stopped by addition of 1 ml stop solution. The solutions are mixed using a test tube shaker; the tubes rest for 5 mins (on the table) and are mixed again before centrifuged at 3500 rpm for 10 minutes. The absorbance at 620 nm is measured against a reagent blank (4.2 ml of assay buffer).
The absorbance of the enzyme samples should be within the range of 0.3 to 0.5.

**Calculation of enzyme activity**

A calibration curve follows with the Phadebas™ tablets. The activity corresponding to the OD620 is read from this calibration curve and the activity of the samples is then calculated according to the formula:

$$U/g = 1000 * \frac{100 * Dilution}{Weight} * A$$

where A is the activity found in the Phadebas™ calibration curve.

**Quality Control (QC)**

The assay has to be redone if measured activity of the double determination has a CV% above 10%.

Quantification limit: 100 U/kg

**IODINE REACTION ASSAY**

The Wohlgemuth Iodine reaction method (Sandstedt, R. M., Kneen, E., and Blish, M.J.: "A Standardized Wohlgemuth procedure for alpha-amylase activity", Cereal Chem. 16, 712-723 (1939)) may also be used in order to measure the alpha amylase activity. This method is based on the blue color that forms when long starch chains coil around iodine molecules. When alpha amylases convert starch to dextrins, the blue color diminishes in proportion to the activity. Iodine reaction methods use moderate reaction conditions and so work on both fungal and bacterial enzyme samples.

**ALPHA AMYLASE**

Alpha amylases (alpha-1,4-glucan-4-glucanohydrolases, EC 3.2.1.1) constitute a group of enzymes, which catalyze hydrolysis of starch and other linear and branched 1,4-glucosidic oligo- and polysaccharides.
A pepsin resistant alpha amylase having amylolytic activity, which can be measured with one of the above described assays may be isolated from any source. Suitably the alpha amylase may be isolated from bacteria (such as Bacillus, for example *B. licheniformis*) and/or from fungi (such as *Trichoderma,* for example *Trichoderma reesei*).
It will be understood by the skilled person that the pepsin resistant alpha amylase can be provided as either liquid or as solid/granulated compositions.
Preferably, when said enzyme is in liquid form, said enzyme is in the medium into which the enzyme has been secreted following culturing of a cell comprising said enzyme. Preferably said medium is cell-free (i.e. the cell(s) have been separated from the medium). Preferably said medium is concentrated. It will be understood that the medium can be granulated to provide a solid enzyme composition.
It will be further understood that the feed supplement may be provided in the form of a solution or as a solid - depending on the use and/or the mode of application and/or the mode of administration.
In one embodiment the feed supplement is in a liquid formulation suitable for consumption, preferably such liquid composition contains buffer, salts, sorbitol and/ or glycerol.
In an alternative embodiment, the feed supplement according to the present invention can be provided as one or more cells comprising a pepsin resistant alpha amylase as defined in claim 1 or claim 4.
In one embodiment the feed supplement is granulated or co-granulated with other enzymes.
Preferably, the feed supplement further comprises at least one physiologically acceptable carrier.
The at least one physiologically acceptable carrier is preferably selected from the group consisting of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.
In one embodiment the alpha amylase enzyme is dried on the physiologically acceptable carrier.
In preferred embodiments, the feed supplement or feedstuff may comprise at least one further enzyme. In preferred embodiments, at least one further feed enzyme is selected from the group consisting of those involved in starch metabolism, fibre degradation, lipid metabolism, proteins or enzymes involved in glycogen metabolism, acetyl esterases, aminopeptidases, amylases, arabinases, arabinofuranosidases, carboxypeptidases, catalases, cellulases, chitinases, chymosin, cutinase, deoxyribonucleases, epimerases, esterases, $\alpha$-galactosidases, $\beta$-glucanases, glucan lysases, endo-$\beta$glucanases, glucoamylases, glucose oxidases, $\beta$-glucosidases, including $\beta$-glucosidase, glucuronidases, hemicellulases, hexose oxidases, hydrolases, invertases, isomerases, laccases, lyases, lipolytic enzymes, mannosidases, oxidases, oxidoreductases, pectate lyases, pectin acetyl esterases, pectin depolymerases, pectin methyl esterases, pectinolytic enzymes, peroxidases, phenoloxidases, polygalacturonases, proteases, rhamno-galacturonases, ribonucleases, thaumatin, transferases, transport proteins, transglutaminases, xylanases, including endo-1,4-$\beta$-xylanase (EC 3.2.1.8), hexose oxidase (D-hexose: $O_2$-oxidoreductase, EC 1.1.3.5) $\beta$-glucanase, pectinase, cellobiohydrolase, acid phosphatases, phytases, including 3-phytase (EC 3.1.3.8) or 6-phytase (EC 3.1.3.26), mannanases and combinations thereof. These include enzymes that, for example, modulate the viscosity of the feed.
In a more preferred embodiment the feed supplement or feedstuff also comprises a phytase, a lipolytic enzyme, a xylanase and/or a protease. In a most preferred embodiment, the feed supplement or feedstuff further comprises a phytase, a lipolytic enzyme, a xylanase and a protease.
Preferably, the amylase is present in the range of about 10U/kg feed to about 10000U/kg feed, more preferably, about 50U/kg feed to about 7500U/kg feed, and even more preferably, about 100U/kg feed to about 5000U/kg feed. For some aspects, the pepsin resistant alpha amylase is present in an amount of less than about 4000, less than about 3000, less than about 2000, less than about 1900, less than about 1800, less than about 1700, less than about 1600, less than about 1500, less than about 1400, less than about 1300, less than about 1200, less than about 1100, less than about 1000, less than about 900, less than about 800, less than about 700, less than about 600, less than about 500, less than about 400, less than about 300, or less than about 200 units/kg of feed. It will be understood that one amylase U is the amount of enzyme that releases 1 mmol of glucosidic linkages from a water insoluble cross-linked starch polymer substrate per min at pH 6.5 and 37°C.

Preferably, the amylase is present at about 50 to 300, more preferably 100 to 200 units/kg of feed.

Preferably, when used, xylanase is present in the range of about 100U/kg to about 10000U/kg feed, more preferably, about 250U/kg feed to about 7500U/kg feed, and even more preferably, about 500U/kg feed to about 5000U/kg feed. It will be understood that one xylanse U is the amount of enzyme that releases 0.5 μmol of reducing sugar equivalents (as xylose by the DNS [4]) reducing sugar method) from a oat-spelt-xylan substrate per min at pH 5.3 and 50°C. (Bailey, M.J. Biely, P. and Poutanen, K., Journal of Biotechnology, Volume 23, (3), May 1992, 257-270).

Preferably, when used, phytase is present at a level of about 250 FTU/kg to about 15,000 FTU/kg feed (e.g. about 250 to about 10,000 FTU/kg feed, about 400 - about 7,500 FTU/kg feed or about 500 - about 5000 FTU/kg feed).

Preferably, when used, lipolytic enzyme is present at a level of about 125 LIPU/kg to about 45,000 LIPU/kg feedstuff (e.g. about 500 to about 30,000 LIPU/kg, about 1000 - about 20000 LIPU/kg and also about 3000 - about 10000 LIPU/kg).

Preferably, when used, protease is present at a level of about 250 U/kg feed to about 15,000 U/kg feed (e.g. about 500 to about 10,000 U/kg feed, about 1,000 - about 8,000 U/kg feed, about 2,000 to about 7,000 U/kg feed or about 3,000 - about 6,000 FTU/kg feed). It will be understood that one protease U is the amount of enzyme that liberates from the substrate (0.6% casein solution) one microgram of phenolic compound (expressed as tyrosine equivalents) in one minute at pH 7.5 (40mM $Na_2PO_4$ / lactic acid buffer) and 40°C.

It will be understood that the feed supplement and/or feedstuff may be for any suitable monogastric animal, for example poultry or swine.

It will be obvious to the skilled person that a feedstuff and/or feed supplement in accordance with the present invention may comprise other components such as stabilising agents and/or bulking agents and/or other enzymes.

Preferably, the feed supplement of the present invention will be thermally stable to heat treatment up to about 70 °C; up to about 85°C; or up to about 95°C. The heat treatment may be performed for up to about 1 minute; up to about 5 minutes; up to about 10 minutes; up to about 30 minutes; up to about 60 minutes. The term "thermally stable" means that at least about 75% of the enzyme components that were present/active in the additive before heating to the specified temperature are still present/active after it cools to room temperature. Preferably, at least about 80% of the enzyme components that were present and active in the additive before heating to the specified temperature are still present and active after it cools to room temperature.

In a particularly preferred embodiment the feed supplement is homogenized to produce a powder.

In an alternative preferred embodiment, the feed supplement is formulated to granules as described in WO2007/044968 (referred to as TPT granules).

in another preferred embodiment when the feed supplement is formulated into granules the granules comprises a hydrated barrier salt coated over the protein core. The advantage of such salt coating is improved thermo-tolerance, improved storage stability and protection against other feed additives otherwise having adverse effect on the enzyme.

Preferably, the salt used for the salt coating has a water activity greater than 0.25 or constant humidity greater than 60 % at 20 °C.

Preferably, the salt coating comprises a $Na_2SO_4$.

The method of preparing a feed supplement may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 85°C, 90°C or 95°C. The residence time can be variable from seconds to minutes and even hours. Such as 5 seconds, 10 seconds, 15 seconds, 30 seconds, 1 minutes 2 minutes, 5 minutes, 10 minutes, 15 minutes, 30 minutes and 1 hour.

It will be understood that the feed supplement of the present invention is suitable for addition to any appropriate feed material.

As used herein, the term "feed material" refers to the basic feed material to be consumed by an animal. It will be further understood that this may comprise, for example, at least one or more unprocessed grains, and/or processed plant and/or animal material such as soybean meal or bone meal.

In some embodiments, the feed material will comprise one or more of the following components: a) cereals, such as small grains (e.g., wheat, barley, rye, oats and combinations thereof) and/or large grains such as maize or sorghum; b) by-products from cereals, such as corn gluten meal, Distillers Dried Grain Solubles (DDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

As used herein, the term "feedstuff" refers to a feed material to which one or more feed supplements have been added.

It will be understood by the skilled person that different animals require different feedstuffs, and even the same animal may require different feedstuffs, depending upon the purpose for which the animal is reared. It will be further understood that depending on the starting feed material, the feedstuff may be a high fibre feedstuff or a low fibre feedstuff.

Preferably, the feedstuff may comprise feed materials comprising maize or corn, wheat, barley, triticale, rye, rice, tapioca, sorghum, and/ or any of the by-products, as well as protein rich components like soybean mean, rape seed meal, canola meal, cotton seed meal, sunflower seed mean, animal-by-product meals and mixtures thereof. More preferably, the feedstuff may comprise animal fats and / or vegetable oils.

Optionally, the feedstuff may also contain additional minerals such as, for example, calcium and/or additional vitamins.

As defined herein, a "low fibre feedstuff' is a feedstuff comprising one or more feed materials, which contains a maximum content of water insoluble cell walls of about 25%, and/or a maximum content of soluble non-starch polysaccharides of about 4%. More preferably, a maximum content of water insoluble cell walls of about 22.5%, about 20%, about 17.5%, about 15%, about 12.5%; and/or a maximum content of soluble non-starch polysaccharides of about 3%, about 2.5%, about 2%, about 1.75%, about 1.5%, about 1.25%.

In some embodiments, the feed supplement is mixed with at least one low fibre feed material, for example, corn, wheat, an animal-by product meal, or soybean and/or any of the by-products to provide a low fibre feedstuff.

Preferably, the feedstuff is a corn soybean meal mix.

In one embodiment, preferably the feed is not pet food.

As defined herein, a "high fibre feedstuff" is a feedstuff comprising one or more feed materials, which contains a minimum content of water insoluble cell walls of about 25%, and/or a minimum content of soluble non-starch polysaccharides of about 4%. More preferably, a minimum content of water insoluble cell walls of about 30%, about 35%, about 40%, about 45%, about 50%, about 60%, about 70%; and/or a minimum content of soluble non-starch polysaccharides of about 5%, about 10%, about 15%, about 20%, about 25%, about 30%.

In some embodiments, the feed supplement is mixed with at least one high fibre feed material (preferably selected from the group consisting of: wheat, barley, rye, oats, by-products from cereals, such as corn gluten meal, Distillers Dried Grain Solubles (DDGS), wheat bran, wheat middlings, wheat shorts, rice bran, rice hulls, oat hulls, palm kernel, and citrus pulp) and/or any of the by-products to provide a high fibre feedstuff.

In another aspect there is provided a method for producing a feedstuff. Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting - in particular by suitable techniques that may include at least the use of steam.

The feedstuff may be a feedstuff for a monogastric animal, such as poultry (for example, broiler, layer, broiler breeders, turkey, duck, geese, water fowl), swine (all age categories), a pet (for example dogs, cats) or fish, preferably the feedstuff is for poultry.

Optionally the feedstuff may comprise further additives. For example, caicium may be added to the feedstuff in any suitable amount to supplement the diet of the animal and/or as a bulking agent.

The feedstuff may comprise at least 0.0001% by weight of the feed supplement. Suitably, the feedstuff may comprise at least 0.0005%; at least 0.0010%; at least 0.0020%; at least 0.0025%; at least 0.0050%; at least 0.0100% by weight of the feed supplement. Suitably, the feedstuff may comprise about 0.0010% to about 0.0200%; preferably about 0.005% to about 0.0100% by weight of the feed supplement.

In a further aspect there is provided a pepsin resistant alpha amylase for use in feed for increasing the available metabolic energy from a feed material.

As used herein, the term "increasing the available metabolic energy" means an increase in the amount of energy available for use by the animal consuming a unit weight feed material compared to the availability of the nutrient or energy available from a unit weight of the feed material to which no amylase enzyme or feed supplement has been added.

The invention will now be described with reference to the following figures in which:

Figure 1 shows the amino acid sequence (SEQ ID No. 1) of a pepsin resistant alpha amylase from *Bacillus licheniformis.*

Figure 2 shows the nucleotide sequence (SEQ ID No. 2) of a pepsin resistant alpha amylase from *Bacillus licheniformis.*

Figure 3 shows the amino acid sequence (SEQ ID No. 3) of a pepsin resistant alpha amylase from *Trichoderma reesei.*

Figure 4 shows the nucleotide sequence (SEQ ID No. 4) of a pepsin resistant alpha amylase from *Trichoderma reesei.*

Figure 5 shows an alignment between a *Bacillus amyloliquefaciens* alpha amylase (designated LTAA) and a *Bacillus licheniformis* pepsin resistant alpha amylase (designated LAT). Amino acids of interest from within LAT are underlined.

Figure 6 shows the pepsin resistance of a *Bacillus licheniformis* alpha amylase (LAT), a *Bacillus amyloliquefaciens* α-amylase (LTAA), a *Trichoderma reesei* α-amylase (Tric. amyl. #266) and a commercially available α-amylase (BAN).

Figure 7 shows the pepsin resistance of a *Bacillus amyloliquefaciens* α-amylase (LTAA) and a *Bacillus licheniformis* variant alpha amylase (FRED).

Figure 8 shows the amino acid sequence for a *Bacillus licheniformis* variant alpha amylase (FRED).

Figure 9 shows body weight gain (meta analysis of 4 trials) from 0-42 days for broiler chicks treated with an alpha amylase from *Bacillus licheniformis* (LAT) and an alpha amylase from *Bacillus amyloliquefaciens* (LTAA). Treatment with LAT is statistically significant ($P<0.05$).

Figure 10 shows the feed conversion ratio (corrected for weight) from 0-42 days. This is a meta analysis of 4 trials. Treatment with LAT is statistically significant ($P<0.05$).

## DETAILED DISCLOSURE OF THE INVENTION

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

The terms "amino acid residue equivalent to", "amino acid corresponding to" and grammatical equivalents thereof are used herein to refer to an amino acid residue of a protein having the similar position and effect as that indicated in a particular amino acid sequence of a particular protein. The person of skill in the art will recognize the equivalence of specified residues in comparable proteins.

The term "property" or grammatical equivalents thereof in the context of a polypeptide, as used herein, refer to any characteristic or attribute of a polypeptide that can be selected or detected. These properties include, but are not limited to oxidative stability, substrate specificity, catalytic activity, thermal stability, temperature and/or pH activity profile, feed processing stability, and ability to be secreted.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The amino acid sequence may be prepared/isolated from a suitable source, or it may be made synthetically or it may be prepared by use of recombinant DNA techniques.

The terms "protein" and "polypeptide" are used interchangeably herein. In the present disclosure and claims, the conventional one-letter and three-letter codes for amino acid residues are used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

The term "signal sequence" or "signal peptide" refers to any sequence of nucleotides and/or amino acids which may participate in the secretion of the mature or precursor forms of the protein. This definition of signal sequence is a functional one, meant to include all those amino acid sequences encoded by the N-terminal portion of the protein gene, which participate in the effectuation of the secretion of protein. They are often, but not universally, bound to the N-terminal portion of a protein or to the N-terminal portion of a precursor protein.

By "functional fragment" is meant a fragment of the polypeptide that retains the characteristic properties of that polypeptide. In the context of the present invention, a functional fragment of a phytase or lipolytic enzyme is a fragment that retains the phytase or lipolytic enzyme cleavage capability of the whole protein.

The term "isolated", "recovered" or "purified" refers to a material that is removed from its original environment. The term "substantially purified" means that the material has been purified to at least a substantial degree.

In one aspect, preferably the nucleotide or amino acid sequence is in an isolated form. The term "isolated" means that the sequence is at least substantially free from at least one other component with which the sequence is naturally associated in nature and as found in nature.

Other definitions of terms may appear throughout the specification. Before the exemplary embodiments are described in more detail, it is to understand that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaiier ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "a gene" includes a plurality of such candidate agents and reference to "the cell" includes reference to one or more cells and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The enzymes for use in the present invention can be produced either by solid or submerged culture, including batch, fed-batch and continuous-flow processes. Culturing is accomplished in a growth medium comprising an aqueous mineral salts medium, organic growth factors, the carbon and energy source material, molecular oxygen, and, of course, a starting inoculum of one or more particular microorganism species to be employed.

## VARIANTS/DERIVATIVES

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

## OTHER COMPONENTS

The feed supplement of the present invention may be used in combination with other components or carriers.

Suitable carriers for feed enzymes include maltodextrin, limestone (calcium carbonate), cyclodextrin, wheat or a wheat component, sucrose, starch, anti-foam, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof. In addition there are a number of encapsulation techniques including those based on fat/wax coverage, adding plant gums etc.

Examples of other components include one or more of: thickeners, gelling agents, emulsifiers, binders, crystal modifiers, sweeteners (including artificial sweeteners), rheology modifiers, stabilisers, anti-oxidants, dyes, enzymes, carriers, vehicles, excipients, diluents, lubricating agents, flavouring agents, colouring matter, suspending agents, disintegrants, granulation binders etc. These other components may be natural. These other components may be prepared by use of chemical and/or enzymatic techniques.

As used herein the term "thickener or gelling agent" as used herein refers to a product that prevents separation by slowing or preventing the movement of particles, either droplets of immiscible liquids, air or insoluble solids.

The term "stabiliser" as used here is defined as an ingredient or combination of ingredients that keeps a product (e.g. a food product) from changing over time.

The term "emulsifier" as used herein refers to an ingredient (e.g. a food product ingredient) that prevents the separation of emulsions.

As used herein the term "binder" refers to an ingredient (e.g. a food ingredient) that binds the product together through a physical or chemical reaction.

The term "crystal modifier" as used herein refers to an ingredient (e.g. a food ingredient) that affects the crystallisation of either fat or water.

"Carriers" or "vehicles" mean materials suitable for compound administration and include any such material known in the art such as, for example, any liquid, gel, solvent, liquid diluent, solubiliser, or the like, which is non-toxic and which does not interact with any components of the composition in a deleterious manner.

Examples of nutritionally acceptable carriers include, for example, grain, water, salt solutions, alcohol, silicone, waxes, petroleum jelly, vegetable oils, and the like.

Examples of excipients include one or more of: microcrystalline cellulose and other celluloses, lactose, sodium citrate, calcium carbonate, dibasic calcium phosphate, glycine, starch, milk sugar and high molecular weight polyethylene glycols.

Examples of disintegrants include one or more of: starch (preferably corn, potato or tapioca starch), sodium starch glycollate, croscarmellose sodium and certain complex silicates. Examples of granulation binders include one or more of: polyvinylpyrrolidone, hydroxypropylmethylcellulose (HPMC), hydroxypropylcellulose (HPC), sucrose, maltose, gelatin and acacia.

Examples of lubricating agents include one or more of: magnesium stearate, stearic acid, glyceryl behenate and talc.

Examples of diluents include one or more of: water, ethanol, propylene glycol and glycerin, and combinations thereof.

The other components may be used simultaneously (e.g. when they are in admixture together or even when they are delivered by different routes) or sequentially (e.g. they may be delivered by different routes).

As used herein the term "component suitable for animal or human consumption" means a compound which is or can be added to the composition of the present invention as a supplement which may be of nutritional benefit, a fibre substitute or have a generally beneficial effect to the consumer.

By way of example, the components may be prebiotics such as alginate, xanthan, pectin, locust bean gum (LBG), inulin, guar gum, galacto-oligosaccharide (GOS), fructo-oligosaccharide (FOS), lactosucrose, soybean oligosaccharides, palatinose, isomalto-oligosaccharides, gluco-oligosaccharides and xylo-oligosaccharides.

**Lipase Units (LIPU)**

As used herein, 1 LIPU (lipase unit) is defined as the amount of enzyme which releases 1 μmol of $H^+$ per minute under the conditions described herein below.

5% (v/v) tributyrin substrate is prepared by Mixing 15.00 ml tributyrin, 50.00 ml emulsifying agent and 235 ml dist. water for 20 sec on a homogenizer. The pH of the substrate is adjusted to approx. 5.4 with 0.5 M NaOH.

Emulsifying agent is prepared by mixing 17.9 g NaCl, 0.41 g $KH_2PO_4$, 400 ml dist. water, and 450 ml glycerol in a 2000 ml beaker. Under vigorous stirring add 6.0 g Gum Arabic and continue stirring until gum Arabic is completely dissolved. Transfer the solution to a 1000 ml volumetric flask and fill to the mark with dist water.

For dry samples: in a volumetric flask dissolve an amount of enzyme calculated to give a final solution of approximately 3.5LIPU/ml in half of the final dilution and subject to magnetic stirring for 20 min.

After stirring, adjust to final dilution with dist. water. Any further dilution should be made with dist. water. **Samples in solution** are diluted directly in dist. water

25.00 mL of substrate is adjusted to 30.0°C.

Adjust substrate pH to 5.50 with NaOH/HCl

While stirring, add 2.00 mL sample, and initiate immediately pH-stat titrator.

Stop titration after 6 minutes.

Calculate slope of the titration curve. The slope of the titration curve is calculated from data between 3 and 6 min.

The slope must be in the interval 0.1 - 0.2 mL/min.

The activity (LIPU/g) of the enzyme is calculated using the following:

$$LIPU/g = \frac{ml/min. \times N \times 1000 \times F \times \text{factor for tributyrin}}{A \times 2}$$

ml/min.: Slope of titration curve
N : Normality of NaOH
F : Dilution of sample
A : Gram sample weighed
2 : ml sample

**ISOLATED**

In one aspect, preferably the pepsin resistant alpha amylase enzyme for use in the present invention is in an isolated form. The term "isolated" means that the pepsin resistant alpha amylase enzyme is at least substantially free from at least one other component with which the enzyme is naturally associated in nature and as found in nature. The term "isolated" may mean that the pepsin resistant alpha amylase enzyme is at least substantially free from at least one other component in the culture media in which it is produced. The pepsin resistant alpha amylase enzyme of the present invention may be provided in a form that is substantially free of one or more contaminants with which the substance might otherwise be associated or with which the enzyme may be produced.

Thus, for example it may be substantially free of the cell(s) or one or more potentially contaminating polypeptides and/or nucleic acid molecules. The alpha amylase may be isolated by separating the cell(s) from the broth during or after fermentation so that the lipolytic enzyme remains in the broth. The alpha amylase may be isolated by subjecting the fermentation broth to cell separation by vacuum filtration.

**PURIFIED**

In one aspect, preferably the pepsin resistant alpha amylase for use in the present invention is in a purified form. The term "purified" means that the given component is present at a high level. The component is desirably the predominant component present in a composition. Preferably, it is present at a level of at least about 60%, or at least about 65%, or at least about 70%, or at least about 75%, or at least about 80% said level being determined on a dry weight/dry weight basis with respect to the total composition under consideration. For some embodiments the amount is at least about 85% said level being determined on a dry weight/dry weight basis with respect to the total composition under consideration.

**CONCENTRATE**

In one aspect, preferably the pepsin resistant alpha amylase for use in the present invention is used as a concentrate. The concentrate may be a concentrated form of the medium into which the enzyme has been excreted. Preferably, the concentrate may be a concentrated form of the medium into which the enzyme has been secreted and wherein the cell(s) have been removed.

**NUCLEOTIDE SEQUENCE**

The scope of the present invention encompasses nucleotide sequences encoding proteins having the specific properties as defined herein.

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or anti-sense strand.

The term "nucleotide sequence" in relation to the present invention includes genomic DNA, cDNA, synthetic DNA, and RNA. Preferably it means DNA, more preferably cDNA sequence coding for the present invention.

In a preferred embodiment, the nucleotide sequence when relating to and when encompassed by the *per se* scope of the present invention does not include the native nucleotide sequence according to the present invention when in its natural environment and when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. However, the amino acid sequence encompassed by the scope of the present invention can be isolated and/or purified post expression of a nucleotide sequence in its native organism. Preferably, however, the amino acid sequence encompassed by scope of the present invention may be expressed by a nucleotide sequence in its native organism but wherein the nucleotide sequence is not under the control of the promoter with which it is naturally associated within that organism.

Typically, the nucleotide sequence encompassed by the scope of the present invention is prepared using recombinant DNA techniques (i.e. recombinant DNA). However, in an alternative embodiment of the invention, the nucleotide sequence could be synthesised, in whole or in part, using chemical methods well known in the art (see Caruthers MH et al., (1980) Nuc Acids Res Symp Ser 215-23 and Horn T et al., (1980) Nuc Acids Res Symp Ser225-232).

## PREPARATION OF THE NUCLEOTIDE SEQUENCE

A nucleotide sequence encoding either a protein which has the specific properties as defined herein or a protein which is suitable for modification may be identified and/or isolated and/or purified from any cell or organism producing said protein. Various methods are well known within the art for the identification and/or isolation and/or purification of nucleotide sequences. By way of example, PCR amplification techniques to prepare more of a sequence may be used once a suitable sequence has been identified and/or isolated and/or purified.

By way of further example, a genomic DNA and/or cDNA library may be constructed using chromosomal DNA or messenger RNA from the organism producing the enzyme. If the amino acid sequence of the enzyme is known, labelled oligonucleotide probes may be synthesised and used to identify enzyme-encoding clones from the genomic library prepared from the organism. Alternatively, a labelled oligonucleotide probe containing sequences homologous to another known enzyme gene could be used to identify enzyme-encoding clones. In the latter case, hybridisation and washing conditions of lower stringency are used.

Alternatively, enzyme-encoding clones could be identified by inserting fragments of genomic DNA into an expression vector, such as a plasmid, transforming enzyme-negative bacteria with the resulting genomic DNA library, and then plating the transformed bacteria onto agar plates containing a substrate for enzyme (i.e. maltose), thereby allowing clones expressing the enzyme to be identified.

In a yet further alternative, the nucleotide sequence encoding the enzyme may be prepared synthetically by established standard methods, e.g. the phosphoroamidite method described by Beucage S.L. et al., (1981) Tetrahedron Letters 22, p 1859-1869, or the method described by Matthes et al., (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al., (Science (1988) 239, pp 487-491).

## AMINO ACID SEQUENCES

The scope of the present invention also encompasses amino acid sequences of enzymes having the specific properties as defined herein.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The amino acid sequence may be prepared/isolated from a suitable source, or it may be made synthetically or it may be prepared by use of recombinant DNA techniques.

The protein encompassed in the present invention may be used in conjunction with other proteins, particularly enzymes. Thus the present invention also covers a combination of proteins wherein the combination comprises the protein/enzyme of the present invention and another protein/enzyme, which may be another protein/enzyme according to the present invention.

Preferably the amino acid sequence when relating to and when encompassed by the *per se* scope of the present invention is not a native enzyme. In this regard, the term "native enzyme" means an entire enzyme that is in its native environment and when it has been expressed by its native nucleotide sequence.

## SEQUENCE IDENTITY OR SEQUENCE HOMOLOGY

The present invention also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences. % homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons.

Calculation of maximum % homology therefore firstly requires the production of an optimal alignment, taking into consideration gap penalties. A suitable computer program for carrying out such an alignment is the Vector NTI (Invitrogen Corp.). Examples of software that can perform sequence comparisons include, but are not limited to, the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov), FASTA (Altschul et al 1990 J. Mol. Biol. 403-410) and AlignX for example. At least BLAST, BLAST 2 and FASTA are available for offline and online searching (see Ausubel et al 1999, pages 7-58 to 7-60).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

Should Gap Penalties be used when determining sequence identity, then preferably the following parameters are used for pairwise alignment:

| FOR BLAST | |
|---|---|
| GAP OPEN | 0 |
| GAP EXTENSION | 0 |

| FOR CLUSTAL | DNA | PROTEIN | |
|---|---|---|---|
| WORD SIZE | 2 | 1 | K triple |
| GAP PENALTY | 15 | 10 | |
| GAP EXTENSION | 6.66 | 0.1 | |

In one embodiment, CLUSTAL may be used with the gap penalty and gap extension set as defined above.

Suitably, the degree of identity with regard to a nucleotide sequence is determined over at least 20 contiguous nucleotides, preferably over at least 30 contiguous nucleotides, preferably over at least 40 contiguous nucleotides, preferably over at least 50 contiguous nucleotides, preferably over at least 60 contiguous nucleotides, preferably over at least 100 contiguous nucleotides.

Suitably, the degree of identity with regard to a nucleotide sequence may be determined over the whole sequence.

The present invention also encompasses the use of variants, homologues and derivatives of any amino acid sequence of a protein or of any nucleotide sequence encoding such a protein as defined in claim 1 or claim 4.

**VARIANTS/HOMOLOGUES/DERIVATIVES**

Here, the term "homologue" means an entity having a certain homology with the subject amino acid sequences and the subject nucleotide sequences. Here, the term "homology" can be equated with "identity".

In the present context, a homologous sequence is taken to include an amino acid sequence which may be at least 75, 80, 85 or 90% identical, preferably at least 95, 96, 97, 98 or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, an homologous sequence is taken to include a nucleotide sequence which may be at least 75, 80, 85 or 90% identical, preferably at least 95, 96, 97, 98 or 99% identical to a nucleotide sequence encoding an enzyme of the present invention (the subject sequence). In preferred embodiments, a nucleotide sequence useful in the present invention includes a nucleotide sequence which is 75, 85 or 90% identical, preferably at least 95 or 98% identical to the nucleotide sequence as set forth in SEQ ID NO:1. Furthermore, a nucleotide sequence useful in the present invention includes a nucleotide sequence which is 75, 85 or 90% identical, preferably at least 95 or 98% identical to the nucleotide sequence as set forth in SEQ ID NO:3. Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software than can perform sequence comparisons include, but are not limited to, the BLAST package (see Ausubel et al., 1999 Short Protocols in Molecular Biology, 4th Ed - Chapter 18), FASTA (Altschul et al., 1990 J. Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention as defined by the claims also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid#, 7-amino heptanoic acid*, L-methionine sulfone#*, L-norleucine*, L-norvaline*, p-nitro-L-phenylalanine*, L-hydroxyproline#, L-thioproline*, methyl derivatives of phenylalanine (Phe) such as 4-methyl-Phe*, pentamethyl-Phe*, L-Phe (4-amino)#, L-Tyr (methyl)*, L-Phe (4-isopropyl)*, L-Tic (1,2,3,4-tetrahydroisoquinoline-3-carboxyl acid)*, L-diaminopropionic acid # and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide seauences of the present invention

If a sequence is complementary to a fragment thereof then that sequence can be used as a probe to identify similar coding sequences in other organisms etc.

Polynucleotides which are not 100% homologous to the sequences of the present invention but fall within the scope of the invention can be obtained in a number of ways. Other variants of the sequences described herein may be obtained for example by probing DNA libraries made from a range of individuals, for example individuals from different populations. In addition, other homologues may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Polynucleotides (nucleotide sequences) of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein. Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. The primers may be designed to contain suitable restriction enzyme recognition sites so that the amplified DNA can be cloned into a suitable cloning vector.

We describe herein sequences that are complementary to the nucleic acid sequences of the present invention or sequences that are capable of hybridising either to the sequences of the present invention or to sequences that are complementary thereto.

**HYBRIDISATION**

The term "hybridisation" as used herein shall include "the process by which a strand of nucleic acid joins with a complementary strand through base pairing" as well as the process of amplification as carried out in polymerase chain reaction (PCR) technologies.

We describe herein the use of nucleotide sequences that are capable of hybridising to the sequences that are complementary to the sequences presented herein, or any derivative, fragment or derivative thereof.

The term "variant" also encompasses sequences that are complementary to sequences that are capable of hybridising to the nucleotide sequences presented herein.

Preferably, the term "variant" encompasses sequences that are complementary to sequences that are capable of hybridising under stringent conditions (e.g. 50°C and 0.2xSSC {1xSSC = 0.15 M NaCl, 0.015 M $Na_3$citrate pH 7.0}) to the nucleotide sequences presented herein.

More preferably, the term "variant" encompasses sequences that are complementary to sequences that are capable of hybridising under high stringent conditions (e.g. 65°C and 0.1xSSC {1xSSC =0.15 M NaCl, 0.015 M $Na_3$citrate pH 7.0}) to the nucleotide sequences presented herein.

We describe herein nucleotide sequences that can hybridise to the nucleotide sequences of the present invention (including complementary sequences of those presented herein).

We describe herein nucleotide sequences that are complementary to sequences that can hybridise to the nucleotide sequences of the present invention (including complementary sequences of those presented herein).

We describe herein polynucleotide sequences that are capable of hybridising to the nucleotide sequences presented herein under conditions of intermediate to maximal stringency.

We describe herein nucleotide sequences that can hybridise to the nucleotide sequence of the present invention, or the complement thereof, under stringent conditions (e.g. 50°C and 0.2xSSC).

We describe herein nucleotide sequences that can hybridise to the nucleotide sequence of the present invention, or the complement thereof, under high stringent conditions (e.g. 65°C and 0.1xSSC).

**MOLECULAR EVOLUTION**

As a non-limiting example, it is possible to produce numerous site directed or random mutations into a nucleotide sequence, either in vivo or in vitro, and to subsequently screen for improved functionality of the encoded polypeptide by various means.

In addition, mutations or natural variants of a polynucleotide sequence can be recombined with either the wildtype or other mutations or natural variants to produce new variants. Such new variants can also be screened for improved functionality of the encoded polypeptide. The production of new preferred variants can be achieved by various methods well established in the art, for example the Error Threshold Mutagenesis (WO 92/18645), oligonucleotide mediated random mutagenesis (US 5,723, 323), DNA shuffling (US 5,605,793), exo-mediated gene assembly WO00/58517. The application of these and similar random directed molecular evolution methods allows the identification and selection of variants of the enzymes of the present invention which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate.

## SITE-DIRECTED MUTAGENESIS

Once a protein-encoding nucleotide sequence has been isolated, or a putative protein-encoding nucleotide sequence has been identified, it may be desirable to mutate the sequence in order to prepare a protein of the present invention.

Mutations may be introduced using synthetic oligonucleotides. These oligonucleotides contain nucleotide sequences flanking the desired mutation sites.

A suitable method is disclosed in Morinaga et al., (Biotechnology (1984) 2, p646-649). Another method of introducing mutations into enzyme-encoding nucleotide sequences is described in Nelson and Long (Analytical Biochemistry (1989), 180, p 147-151).

## RECOMBINANT

in one aspect the sequence for use in the present invention is a recombinant sequence - i.e. a sequence that has been prepared using recombinant DNA techniques.

These recombinant DNA techniques are within the capabilities of a person of ordinary skill in the art. Such techniques are explained in the literature, for example, J. Sambrook, E. F. Fritsch, and T. Maniatis, 1989, Molecular Cloning: A Laboratory Manual, Second Edition, Books 1-3, Cold Spring Harbor Laboratory Press.

## SYNTHETIC

In one aspect the sequence for use in the present invention is a synthetic sequence - i.e. a sequence that has been prepared by *in vitro* chemical or enzymatic synthesis. It includes, but is not limited to, sequences made with optimal codon usage for host organisms - such as the methylotrophic yeasts *Pichia* and *Hansenula.*

## EXPRESSION OF ENZYMES

The nucleotide sequence for use in the present invention may be incorporated into a recombinant replicable vector. The vector may be used to replicate and express the nucleotide sequence, in protein/enzyme form, in and/or from a compatible host cell.

Expression may be controlled using control sequences e.g. regulatory sequences.

The protein produced by a host recombinant cell by expression of the nucleotide sequence may be secreted or may be contained intracellularly depending on the sequence and/or the vector used. The coding sequences may be designed with signal sequences which direct secretion of the substance coding sequences through a particular prokaryotic or eukaryotic cell membrane.

## EXPRESSION VECTOR

The term "expression vector" means a construct capable of *in vivo* or *in vitro* expression.

Preferably, the expression vector is incorporated into the genome of a suitable host organism. The term "incorporated" preferably covers stable incorporation into the genome.

The nucleotide sequence of the present invention may be present in a vector in which the nucleotide sequence is operably linked to regulatory sequences capable of providing for the expression of the nucleotide sequence by a suitable host organism.

The vectors for use in the present invention may be transformed into a suitable host cell as described below to provide for expression of a polypeptide of the present invention.

The choice of vector e.g. a plasmid, cosmid, or phage vector will often depend on the host cell into which it is to be introduced.

The vectors for use in the present invention may contain one or more selectable marker genes- such as a gene, which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect, transform, transduce or infect a host cell.

Thus, in a further embodiment, the invention provides a method of making nucleotide sequences of the present invention by introducing a nucleotide sequence of the present invention into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

## REGULATORY SEQUENCES

In some applications, the nucleotide sequence for use in the present invention is operably linked to a regulatory sequence which is capable of providing for the expression of the nucleotide sequence, such as by the chosen host cell. By way of example, the present invention covers a vector comprising the nucleotide sequence of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme of the present invention may also be achieved by the selection of heterologous regulatory regions, e.g. promoter, secretion leader and terminator regions.

Preferably, the nucleotide sequence according to the present invention is operably linked to at least a promoter.

Other promoters may even be used to direct expression of the polypeptide of the present invention.

Examples of suitable promoters for directing the transcription of the nucleotide sequence in a bacterial, fungal or yeast host are well known in the art.

The promoter can additionally include features to ensure or to increase expression in a suitable host. For example, the features can be conserved regions such as a Pribnow Box or a TATA box.

## CONSTRUCTS

The term "construct" - which is synonymous with terms such as "conjugate", "cassette" and "hybrid" - includes a nucleotide sequence for use according to the present invention directly or indirectly attached to a promoter.

An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker, which allows for the selection of the genetic construct.

For some applications, preferably the construct of the present invention comprises at least the nucleotide sequence of the present invention operably linked to a promoter.

## HOST CELLS

The term "host cell" - in relation to the present invention includes any cell that comprises either the nucleotide sequence or an expression vector as described above and which is used in the recombinant production of a protein having the specific properties as defined herein.

We describe herein host cells transformed or transfected with a nucleotide sequence that expresses the protein of the present invention. The cells will be chosen to be compatible with the said vector and may for example be prokaryotic (for example bacterial), fungal, yeast or plant cells.

Examples of suitable bacterial host organisms are gram positive or gram negative bacterial species.

Depending on the nature of the nucleotide sequence encoding the polypeptide of the present invention, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

The use of suitable host cells - such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

The host cell may be a protease deficient or protease minus strain. This may for example be the protease deficient strain *Aspergillus oryzae* JaL 125 having the alkaline protease gene named "alp" deleted. This strain is described in WO97/35956.

## ORGANISM

The term "organism" in relation to the present invention includes any organism that could comprise the nucleotide sequence coding for the polypeptide according to the present invention and/or products obtained therefrom, and/or wherein a promoter can allow expression of the nucleotide sequence according to the present invention when present in the organism.

Suitable organisms may include a prokaryote, fungus, yeast or a plant.

The term "transgenic organism" in relation to the present invention includes any organism that comprises the nucleotide sequence coding for the polypeptide according to the present invention and/or the products obtained therefrom, and/or wherein a promoter can allow expression of the nucleotide sequence according to the present invention within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism of the present invention includes an organism comprising any one of, or combinations of, the nucleotide sequence coding for the polypeptide according to the present invention, constructs according to the present invention, vectors according to the present invention, plasmids according to the present invention, cells according to the present invention, tissues according to the present invention, or the products thereof.

For example the transgenic organism may also comprise the nucleotide sequence coding for the polypeptide of the present invention under the control of a heterologous promoter.

## TRANSFORMATION OF HOST CELLS/ORGANISM

As indicated earlier, the host organism can be a prokaryotic or a eukaryotic organism. Examples of suitable prokaryotic hosts include *E. coli* and *Bacillus subtilis.*

Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

Filamentous fungi cells may be transformed using various methods known in the art - such as a process involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

## TRANSFORMED FUNGUS

A host organism may be a fungus - such as a mould. Examples of suitable such hosts include any member belonging to the genera Thermomyces, Acremonium, Aspergillus, Penicillium, Mucor, Neurospora, Trichoderma and the like.

A host organism may be a filamentous fungus.

Transforming filamentous fungi is discussed in US-A-5741665 which states that standard techniques for transformation of filamentous fungi and culturing the fungi are well known in the art. An extensive review of techniques as applied to *N. crassa* is found, for example in Davis and de Serres, Methods Enzymol (1971) 17A: 79-143.

Further teachings which may also be utilised in transforming filamentous fungi are reviewed in US-A-5674707.

In addition, gene expression in filamentous fungi is taught in in Punt et al. (2002) Trends Biotechnol 2002 May;20(5):200-6, Archer & Peberdy Crit Rev Biotechnol (1997) 17(4):273-306.

A host organism can be of the genus *Aspergillus,* such as *Aspergillus niger.*

A transgenic *Aspergillus* can also be prepared by following, for example, the teachings of Turner G. 1994 (Vectors for genetic manipulation. In: Martinelli S.D., Kinghorn J.R.(Editors) Aspergillus: 50 years on. Progress in industrial microbiology vol 29. Elsevier Amsterdam 1994. pp. 641-666).

A transgenic organism can be a yeast.

## TRANSFORMED YEAST

A review of the principles of heterologous gene expression in yeast are provided in, for example, Methods Mol Biol (1995), 49:341-54, and Curr Opin Biotechnol (1997) Oct;8(5):554-60

In this regard, yeast - such as the species *Saccharomyces cerevisi or Pichia pastoris* (*see* FEMS Microbiol Rev (2000 24(1):45-66), may be used as a vehicle for heterologous gene expression.

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic Saccharomyces can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells may be selected using various selective markers - such as auxotrophic markers dominant antibiotic resistance markers.

## TRANSFORMED PLANTS/PLANT CELLS

A host organism may be a plant. In this respect, the basic principle in the construction of genetically modified plants is to insert genetic information in the plant genome so as to obtain a stable maintenance of the inserted genetic material. A review of the general techniques may be found in articles by Potrykus (Annu Rev Plant Physiol Plant Mol Biol [1991] 42:205-225) and Christou (Agro-Food-Industry Hi-Tech March/April 1994 17-27).

Direct infection of plant tissues by *Agrobacterium* is a simple technique which has been widely employed and which is described in Butcher D.N. et al., (1980), Tissue Culture Methods for Plant Pathologists, eds.: D.S. Ingrams and J.P. Helgeson, 203-208.

Other techniques for transforming plants include ballistic transformation, the silicon whisker carbide technique (see Frame BR, Drayton PR, Bagnaall SV, Lewnau CJ, Bullock WP, Wilson HM, Dunwell JM, Thompson JA & Wang K (1994) Production of fertile transgenic maize plants by silicon carbide whisker-mediated transformation, The Plant Journal 6: 941-948) and viral transformation techniques (e.g. see Meyer P, Heidmann I & Niedenhof I (1992) The use of cassava mosaic virus as a vector system for plants, Gene 110: 213-217).

Further teachings on plant transformation may be found in EP-A-0449375.

Plant cells may be grown and maintained in accordance with well-known tissue culturing methods such as by culturing the cells in a suitable culture medium supplied with the necessary growth factors such as amino acids, plant hormones, vitamins, etc.

We describe herein a vector system which carries a nucleotide sequence or construct according to the present invention and which is capable of introducing the nucleotide sequence or construct into the genome of an organism, such as a plant. A vector system may comprise one vector, but it may comprise two vectors. In the case of two vectors, the vector system is normally referred to as a binary vector system. Binary vector systems are described in further detail in Gynheung An et al., (1980), Binary Vectors, Plant Molecular Biology Manual A3, 1-19.

One extensively employed system for transformation of plant cells uses the Ti plasmid from *Agrobacterium tumefaciens* or a Ri plasmid from *Agrobacterium rhizogenes* An et al., (1986), Plant Physiol. 81, 301-305 and Butcher D.N. et al., (1980), Tissue Culture Methods for Plant Pathologists, eds.: D.S. Ingrams and J.P. Helgeson, 203-208. After each introduction method of the desired promoter or construct or nucleotide sequence according to the present invention in the plants, the presence and/or insertion of further DNA sequences may be necessary. If, for example, for the transformation the Ti- or Ri-plasmid of the plant cells is used, at least the right boundary and often however the right and the left boundary of the Ti- and Ri-plasmid T-DNA, as flanking areas of the introduced genes, can be connected. The use of T-DNA for the transformation of plant cells has been intensively studied and is described in EP-A-120516; Hoekema, in: The Binary Plant Vector System Offset-drukkerij Kanters B.B., Alblasserdam, 1985, Chapter V; Fraley, et al., Crit. Rev. Plant Sci., 4:1-46; and An et al., EMBO J. (1985) 4:277-284.

Host cells transformed with the nucleotide sequence as defined in claim 1 or claim 4 may be cultured under conditions conducive to the production of the encoded polypeptide and which facilitate recovery of the polypeptide from the cells and/or culture medium.

## CULTURING AND PRODUCTION

The medium used to cultivate the cells may be any conventional medium suitable for growing the host cell in questions and obtaining expression of the polypeptide.

The protein produced by a recombinant cell may be displayed on the surface of the cell.

The protein may be secreted from the host cells and may conveniently be recovered from the culture medium using well-known procedures.

Often, it is desirable for the protein to be secreted from the expression host into the culture medium from where the protein may be more easily recovered. The secretion leader sequence may be selected on the basis of the desired expression host. Hybrid signal sequences may also be used with the context of the present invention.

## SECRETION

Typical examples of heterologous secretion leader sequences are those originating from the fungal amylocosidase (AG) gene (*gla*A - both 18 and 24 amino acid versions e.g. from *Aspergillus*), the a-factor gene (yeasts e.g. *Saccharomyces, Kluyveromyces* and *Hansenula*) or the α-amylase gene (*Bacillus*).

By way of example, the secretion of heterologous proteins in *E. coli* is reviewed in Methods Enzymol (1990) 182:132-43.

## DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies such as Pharmacia Biotech (Piscataway, NJ), Promega (Madison, WI), and US Biochemical Corp (Cleveland, OH) supply commercial kits and protocols for these procedures.

Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.

Also, recombinant immunoglobulins may be produced as shown in US-A-4,816,567.

## ADDITIONAL PROTEINS OF INTEREST (POIs)

The sequences for use according to the present invention may also be used in conjunction with one or more additional proteins of interest (POIs) or nucleotide sequences of interest (NOIs).

Non-limiting examples of POIs include: proteins or enzymes involved in starch metabolism, proteins or enzymes involved in glycogen metabolism, acetyl esterases, aminopeptidases, amylases, arabinases, arabinofuranosidases, carboxypeptidases, catalases, cellulases, chitinases, chymosin, cutinase, deoxyribonucleases, epimerases, esterases, α-galactosidases, β-galactosidases, α-glucanases, glucan lysases, endo-β-glucanases, glucoamylases, glucose oxidases, α-glucosidases, β-glucosidases, glucuronidases, hemicellulases, hexose oxidases, hydrolases, invertases, isomerases, laccases, lipolytic enzymes, lyases, mannosidases, oxidases, oxidoreductases, pectate lyases, pectin acetyl esterases, pectin depolymerases, pectin methyl esterases, pectinolytic enzymes, peroxidases, phenoloxidases, phytases including 3-phytase (EC 3.1.3.8) or 6-phytase (EC 3.1.3.26), polygalacturonases, proteases, rhamno-galacturonases, ribonucleases, thaumatin, transferases, transport proteins, transglutaminases, xylanases, including endo-1,4-β-xylanase (EC 3.2.1.8), hexose oxidase (D-hexose: $O_2$-oxidoreductase, EC 1.1.3.5) or combinations thereof. The NOI may even be an antisense sequence for any of those sequences.

The POI may even be a fusion protein, for example to aid in extraction and purification.

The POI may even be fused to a secretion sequence.

Other sequences can also facilitate secretion or increase the yield of secreted POI. Such sequences could code for chaperone proteins as for example the product of *Aspergillus niger cyp B* gene described in UK patent application 9821198.0.

The NOI may be engineered in order to alter their activity for a number of reasons, including but not limited to, alterations which modify the processing and/or expression of the expression product thereof. By way of further example, the NOI may also be modified to optimise expression in a particular host cell. Other sequence changes may be desired in order to introduce restriction enzyme recognition sites.

The NOI may include within it synthetic or modified nucleotides- such as methylphosphonate and phosphorothioate backbones.

The NOI may be modified to increase intracellular stability and half-life. Possible modifications include, but are not limited to, the addition of flanking sequences of the 5' and/or 3' ends of the molecule or the use of phosphorothioate or 2' O-methyl rather than phosphodiesterase linkages within the backbone of the molecule.

The invention will now be described, by way of example only, with reference to the following Examples.

## EXAMPLE 1

This example was designed to assess the survival of amylases in the digestive system of monogastric animals. *In vivo* animals excrete amylases which makes it difficult to examine survival of added enzymes. An *in vitro* test was therefore developed. The conditions in the experimental set-up were designed to mimic the conditions in the Gastro Intestinal Tract (GIT) of monogastric animals. The amylases were tested to see if they were active after being exposed to these conditions. The α-amylase enzyme LTAA was used as a reference, as this α-amylase was known to improve animal performance in animal trials.

### Material and Methods

Buffers:

Pepsin incubation buffer: 0.1 M Glycine-HCl, pH 3.0, 3 mg/ml BSA, 2.9 mg Sodium chloride anhydrous/mL, 0.73 mg calcium chloride/mL. For solutions with pepsin, the incubation buffer is prepared to contain 25 mg/ml (9250 U/ml) of pepsin (Sigma P-7000) followed by three continuous ten-times dilutions to end up with four solutions containing 25, 2.5, 0.25, and 0.025 mg/ml pepsin, respectively. One pepsin unit is defined as the amount of enzyme that will produce a $\Delta OD_{280}$ of 0.001 per min at pH 2.0 at 37°C, measured as TCA-soluble products using hemoglobin as substrate (described in Food Chemical Codex).

Amylase assay buffer: Phosphate-citrate buffer 0,1 M, pH 5.6.

Amylase assay buffer with BSA: Phosphate-citrate buffer 0,1M, pH 5.6, 3mg/ml BSA.

Enzymes:

- FRED (*Bacillus licheniformis* α-amylase, variant of LAT), available from Genencor and described in WO2009/149271.
- LAT (*Bacillus licheniformis* α-amylase), available from Genencor (Purastar ST15000L©) and described in US6211134.
- LTAA or EBA (*Bacillus amyloliquefaciens* α-amylase) was an enzyme standard used by feed enzyme service and is described in US5763385. Activity: 57492 FFI U/g.
- BAN (available from Novozymes).
- Tric. Amyl #26 (TrAA -*Tricoderma reesei* α-amylase), described in WO2008/112729.

Resistance against increasing pepsin concentration:

The set-ups for all enzymes were the same: Six samples with enzyme and feed were prepared (in duplicate): Four samples with increasing amount of pepsin in buffer (pH 3), one sample without pepsin but in incubation buffer (pH 3), and one positive control sample with enzyme in assay buffer with BSA. This was done in duplicate, i.e. for each enzyme to test for pepsin resistance, there were 12 eppendorf tupes prepared.

Beside the 2x6 tubes for each enzyme to be tested, 2x6 similar samples without enzyme added were prepared to check the background absorbance from the chemicals.

In each 1,5 ml micro-centrifuge tube from Eppendorf, 100 mg of corn based feed was weighed out. A volume of 900 μl incubation buffer without or with increasing amounts of pepsin or 900 μl assay buffer were added and pre-incubated in an Eppendorf Thermomixer 5436 at 500 rpm for 5 mins. A volume of 100 μl enzyme solution (or 100 μl $H_2O$) was added to each tube, the lids were closed, where after they were incubated at 40°C in the Eppendorf Thermomixer 5436 at 500 rpm. After exactly 120 min incubation, the tubes (6 at a time) were spun down in a Eppendorf table centrifuge for 2 mins, 100 μl was withdrawn and mixed with 900 μl assay buffer. Samples were immediately analysed for activity in a KoneLab Arena 20XT (from Thermo Electron Corporation).

Pepsin resistance at a given pepsin concentration is defined as the activity of the amylase measured by the KoneLab assay after being incubated at the given pepsin concentration at pH 3 for two hours as described in the above protocol measured relative to the activity measured by the KoneLab assay after being incubated without pepsin at pH 3 for two hours as described in the above protocol.

**KoneLab Assay**

Chemicals:

Citric acid monohydrate (Merck)
di-Sodium hydrogen phosphate (Merck)
Calcium chloride 2 aq (Merck)
Sodium chloride anhydrous (Merck)
Albumin from bovine serum (BSA, Sigma A 7906)
Cysteine (Merck 2838)
Tris(hydroxymethyl)aminomethane (Merck)

Reagents:

1. Stability reagent

Dissolved:

0.20 g Albumin from bovine serum (BSA),
0.05 g Cysteine,
2.0 g Sodium chloride anhydrous in 10 mL deionised water.

2. Assay buffer: Citric-phosphate buffer, 0.1 M, pH = 5.60

Dissolved:

4.41 g Citric acid monohydrate,
10.3 g di-Sodium hydogen phosphate dihydrate,
2.90 g Sodium chloride anhydrous,
0.73 g Calcium chloride dihydrate in approx. 800 mL of deionised water.
1.00 mL stability-reagent (1) is added while stirring on magnetic stirrer.

When dissolved, pH is adjusted to 5.60 (HCl or NaOH) and the solution is transferred to a 1000 mL volumetric flask and adjust to 1000 mL with distilled water.

3. Stop solution : 1% (w/v) TRIS (Trizma base)

Dissolved 10 g of TRIS ((hydroxymethyl)aminomethane) in deionised water in a volumetric flask and adjusted to 1000 mL.

4. Substrate (Freeze-dried Ceralpha (BPNPG7) from Megazyme)

A brown bottle of cereal alpha-amylase assay reagent (BPNPG 7) was dissolved in 10.0 ml distilled water (2).
Before use the solution was further diluted 1:1 also with distilled water.
1 bottle of Ceralpha contains 54.5 mg BPNPG7 and 125 U (pH = 6.0) alpha-glucosidase.

5. Control /Standard sample

Amylase (LAT) standard: 485 TAU/g (Lot#102-05208-001)
Furthermore a LAT control sample (Lot#102-01128-lab) with a range of 7957-8162 TAU/g.

**Procedure**

Preparation of Amylase standard curve:

An Amylase standard (5) is diluted to a concentration of approx. 1.9 U/g for LAT.

All dilutions are carried out in Assay buffer (2).

Further dilutions are programmed in Konelab:

| Standard | Dil. Ratio | Concentration, U/mL |
|---|---|---|
| 1 | 1+49.0 | 0.034 |
| 2 | 1+19.0 | 0.085 |
| 3 | 1+9.0 | 0.170 |
| 4 | 1+5.0 | 0.283 |
| 5 | 1+3.0 | 0.425 |

OD range should be between 0.2 and 1.5

Sample preparation:

2 weighings were carried out for each sample.

*Liquid products:* 0.5 g of sample is weighed in a 50 ml volumetric flask. The Flask is filled with assay buffer (2) and mixed. Further dilutions are also carried out in Assay buffer (2). Final concentrations should be approx. 0.2 U/ml.

*Solid products:* 0.5 g of sample is weighed in a 50 ml volumetric flask and diluted in approx. 40 ml of Assay buffer (2). The solution is mixed on a magnetic stirrer for 10 minutes and filled up with buffer. The solution is filtered through a whatman glass filter and further dilutions were carried out in Assay buffer (2). Final concentrations were approx. 0.2 U/ml.

The weight of the sample was written down with 4 decimals for later calculations. To make the procedure of dilating easier, all dilutions wire carried out using a Diluter (Hamilton).

*Blinds:* 2 blind samples (Assay buffer (2)) were included in each run.

Reaction conditions in the assay:

pH = 5.60
Incubation temperature = 37°C +/- 0.1 °C
Wavelength = 405 nm

| | |
|---|---|
| Substrate | 50 µl |
| Pre-incubation | 5 min |
| Sample | 10 µl |
| Incubation | 15 min |
| Stop solution | 100 µl |

Calculation of enzyme activity:

The activity of a sample wass calculated according to the formula:

$$\text{Activity, } U/g = \frac{(OD_{sample} - \beta) * DF}{\alpha * W_{sample}}$$

$OD_{sample}$ = absorbance of the enzyme sample
DF = dilution factor for the sample
$W_{sample}$ = weight of sample in g
$\alpha$ = Slope of the standardcurve
$\beta$ = Intercept of standardcurve

Results

The results can be seen in Figures 6 and 7. Interestingly, the existing α-amylases used in feed - LTAA (Genencor) and BAN (Novozymes) showed a reduction in activity at higher pepsin concentrations whereas FRED, LAT and TrAA, showed excellent stability towards low pH and pepsin in presence of 10% feed.

The conditions in the experimental set-up are designed to mimic the conditions in the Gastro Intestinal Tract (GIT) of monogastric animals. LTAA was tested as a reference, as this α-amylase is known to improve animal performance. FRED, LAT and TrAA show better stability in the assay, so they are therefore expected to show equal or better stability in the GIT.

**Example 2**

This example was designed to compare the performance of two amylases LTAA (a *B. amyloliquefaciens* alpha amylase presently used in feed) and LAT, the pepsin resistant alpha amylase according to the invention. The purpose was furthermore to test if the evaluation/comparison of the two amylases done *in vitro* could be validated in animal trials. The LTAA was dosed as recommended from the supplier (Danisco) 2000 U/kg of feed, whereas the LAT was dosed to be equivalent in performance based on laboratory trials, i.e. 100 U/kg feed.

**MATERIALS AND METHODS (trials 1, 2, 3 and 4)**

**Performance trials**

Male broiler (Ross 308) day-old chicks were obtained from a commercial hatchery, weighed and assigned on the basis of body weight to 48 floor pens: six treatments with eight replicate pens. Floor pens were located in environmentally controlled rooms. Each pen was identical in layout, with one bell drinker and one feed hopper per pen. Feed in mash form was provided *ad libitum* and water was freely available throughout the study. Ingredients and calculated diet composition are presented in Tables 1 and 2. Body weights and feed intake were recorded on days 1, 21 and 42. Mortality was recorded daily. Any bird that died was weighed and the weight was used to adjust feed conversion ratio. Feed conversion ratios were calculated by dividing total feed intake by weight gain of live plus dead birds.

**Statistical analysis**

Data were analyzed using a generalized linear model (Proc Mixed; SAS Institute, Cary, NC) that included the main effects of dietary enzyme, as well as the random effects of trial site and block x trial site. Least squares mean separation was done through multiple t-tests. Significance was assessed at P<0.05.

**Enzyme added**

Amylases; *Bacillus amyloliquefaciens* alpha amylase (LTAA) at 2000 U/kg of feed or *Bacillus licheniformis* alpha amylase (LAT) at 100 U/kg of feed. To both diets were also added 2000 U/kg of *Trichoderma* xylanase.

Table 1. Broiler trials. Experimental diets of trials 1, 2, 3, and 4. Starter (0 to 21 d).

| Ingredient | Trial 1 | Trial 2 | | Trial 3 | Trial 4 |
|---|---|---|---|---|---|
| Corn | 60.60 | 54.84 | (%) | 57.70 | 53.97 |
| Soybean meal 48% | 33.79 | 28.91 | | 37.21 | 33.90 |
| DDGS | 0.00 | 10.00 | | 0.00 | 7.00 |
| Soy oil | 1.50 | 2.21 | | 1.24 | 1.31 |
| Starch or enzyme | 0.05 | 0.05 | | 0.05 | 0.05 |
| Dicalcium phosphate | 1.38 | 1.21 | | 1.35 | 1.25 |
| Limestone | 1.17 | 1.29 | | 1.17 | 1.21 |
| Sodium bicarbonate | 0.27 | 0.16 | | 0.00 | 0.00 |
| Salt | 0.20 | 0.23 | | 0.35 | 0.35 |
| Lysine-HCl | 0.18 | 0.27 | (%) | 0.05 | 0.10 |
| DL-methionine | 0.26 | 0.22 | | 0.23 | 0.21 |
| L-threonine | 0.00 | 0.01 | | 0.00 | 0.00 |
| Broiler Premix[1] | 0.30 | 0.30 | | 0.35 | 0.35 |

(continued)

| Ingredient | Trial 1 | Trial 2 | Trial 3 | Trial 4 |
|---|---|---|---|---|
| Inert marker | 0.30 | 0.30 | 0.30 | 0.30 |
| Total Calculated nutrient composition | 100.00 | 100.00 | 100.00 | 100.00 |
| ME (kcal/kg) ME (kcal/kg) | 2,995.0 0 | 2,995.0 0 | 2,950.0 0 | 2,950.0 0 |
| CP (%) | 21.70 | 21.70 | 23.00 | 22.90 |
| Calcium (%) | 0.85 | 0.85 | 0.85 | 0.85 |
| Av. phosphorus (%) | 0.38 | 0.38 | 0.38 | 0.38 |
| Lysine (%) | 1.30 | 1.30 | 1.30 | 1.30 |
| Methionine (%) | 0.60 | 0.59 | 0.58 | 0.57 |
| TSAA (%) | 0.95 | 0.95 | 0.95 | 0.95 |

Table 2. Broiler trials. Experimental diets of trials 1, 2, 3, and 4. Finisher (21 to 42 d).

| Ingredient | Trial 1 | Trial 2 | (%) | Trial 3 | Trial 4 |
|---|---|---|---|---|---|
| Corn | 63.30 | 57.50 | | 64.64 | 61.08 |
| Soybean meal 48% | 31.69 | 26.78 | | 29.82 | 26.80 |
| DDGS | 0.00 | 10.00 | | 0.00 | 7.00 |
| Soy oil | 1.74 | 2.53 | | 2.35 | 2.28 |
| Starch or enzyme | 0.05 | 0.05 | | 0.05 | 0.05 |
| Dicalcium phosphate | 1.01 | 0.85 | | 0.75 | 0.70 |
| Limestone | 1.05 | 1.17 | | 1.32 | 1.33 |
| Sodium bicarbonate | 0.32 | 0.16 | | 0.00 | 0.00 |
| Salt | 0.16 | 0.23 | | 0.35 | 0.35 |
| Lysine-HCl | 0.00 | 0.09 | | 0.00 | 0.00 |
| DL-methionine | 0.18 | 0.13 | | 0.07 | 0.06 |
| L-threonine | 0.00 | 0.01 | | 0.00 | 0.00 |
| Broiler Premix[1] | 0.20 | 0.20 | | 0.35 | 0.35 |
| Inert marker | 0.30 | 0.30 | | 0.30 | 0.00 |
| *Total* | 100.00 | 100.00 | | 100.00 | 100.00 |
| Calculated nutrient composition | | | | | |
| ME (kcal/kg) | 3,050.0 0 | 3,050.0 0 | | 3,100.0 0 | 3,100.0 0 |
| CP (%) | 20.68 | 20.68 | | 20.00 | 20.00 |
| Calcium (%) | 0.72 | 0.72 | | 0.76 | 0.76 |
| Av. phosphorus (%) | 0.31 | 0.31 | | 0.26 | 0.26 |
| Lysine (%) | 1.10 | 1.10 | | 1.05 | 1.01 |
| Methionine (%) | 0.51 | 0.50 | | 0.39 | 0.39 |
| TSAA(%) | 0.85 | 0.85 | | 0.72 | 0.72 |

**Results**

The results are detailed in Figures 9 and 10. The use of a pepsin resistant alpha amylase (LAT) resulted in statistically significant weight gain compared with the control amylase LTAA (Figure 9). The use of a pepsin resistant alpha amylase (LAT) also resulted in an improved feed conversion (statistically significant) compared with the control (Figure 10), where feed conversion is the amount of feed needed to produce 1 kg of animal (the inverse of feed efficacy).

## Claims

**1.** A method for preparing a feed supplement for a monogastric animal comprising admixing a pepsin resistant alpha amylase with at least one physiologically acceptable carrier selected from the group consisting of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof; wherein the pepsin resistant amylase has an amino acid sequence:

i) as set forth in SEQ ID No. 1;
ii) having at least 85% identity to SEQ ID No. 1;
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code; or
v) which is produced by expression of a nucleotide sequence which has at least 70% identity to SEQ ID No. 2.

**2.** A feed supplement which is:

a) a poultry feed supplement comprising a pepsin resistant alpha amylase; or
b) a feed supplement for monogastric animals comprising a pepsin resistant alpha amylase and at least one physiologically acceptable carrier selected from the group consisting of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof; wherein the pepsin resistant amylase has an amino acid sequence:

i) as set forth in SEQ ID No. 1;
ii) having at least 85% identity to SEQ ID No. 1;
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code; or

v) which is produced by expression of a nucleotide sequence which has at least 70% identity to SEQ ID No. 2.

**3.** A feedstuff for a monogastric animal comprising a pepsin resistant alpha amylase, wherein said feedstuff comprises less than 3000 units of alpha amylase per kilogram feed; wherein the pepsin resistant amylase has an amino acid sequence:

i) as set forth in SEQ ID No. 1;
ii) having at least 85% identity to SEQ ID No. 1;
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code; or
v) which is produced by expression of a nucleotide sequence which has at least 70% identity to SEQ ID No. 2.

**4.** A method for preparing a feed supplement for a monogastric animal comprising admixing a pepsin resistant alpha amylase with at least one physiologically acceptable carrier selected from the group consisting of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof;
wherein the pepsin resistant alpha amylase on gap alignment with SEQ ID No. 1 comprises any one or more of the following amino acids selected from the group consisting of: K88; 1103; H133; Y175; Y290; F292; R442 and H450, wherein the amino acid numbering relates to SEQ ID No. 1.

**5.** A feed supplement which is:

a) a poultry feed supplement comprising a pepsin resistant alpha amylase; or
b) a feed supplement for monogastric animals comprising a pepsin resistant alpha amylase and at least one physiologically acceptable carrier selected from the group consisting of maltodextrin, limestone (calcium carbonate), cyclodextrin, wheat or a wheat component, sucrose, starch, $Na_2SO_4$, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof;

wherein the pepsin resistant alpha amylase on gap alignment with SEQ ID No. 1 comprises any one or more of the following amino acids selected from the group consisting of: K88; 1103; H133; Y175; Y290; F292; R442 and H450, wherein the amino acid numbering relates to SEQ ID No. 1.

**6.** A feedstuff for a monogastric animal comprising a pepsin resistant alpha amylase, wherein said feedstuff comprises less than 3000 units of alpha amylase per kilogram feed; wherein the pepsin resistant alpha amylase on gap alignment with SEQ ID No. 1 comprises any one or more of the following amino acids selected from the group consisting of: K88; 1103; H133; Y175; Y290; F292; R442 and H450, wherein the amino acid numbering relates to SEQ ID No. 1.

**7.** A method for preparing a feedstuff for a monogastric animal comprising mixing feed supplement according to any one of claims 2, or 5 with one or more feed materials.

**8.** The method according to claim 7, wherein the feedstuff is for poultry.

**9.** The method according to claim 7 or 8, wherein the one or more feed materials are selected from the group consisting of a) small grain or large grain cereals; b) by-products from cereals; c) proteins; d) oils and fats obtained from vegetable and animal sources; and e) minerals and vitamins.

**10.** The method according to claim 9, wherein the small grain cereals are selected from wheat, barley, rye, oats and combinations thereof; the large grain cereals are selected from maize and sorghum; the by-products from cereals are selected from corn gluten meal, Distillers Dried Grain Solubles (DDGS), wheat bran, wheat middlings, wheat shorts, rice bran, rice hulls, oat hulls, palm kernel, and citrus pulp; and/or the proteins are obtained from soya, sunflower, peanut, lupin, peas, fava beans, cotton, canola, fish meal, dried plasma protein, meat and bone meal, potato protein, whey, copra and/or sesame.

**11.** A feedstuff for a monogastric animal prepared by the method of any one of claims 7 to 10 or comprising the feed supplement of any one of claims 2, or 5.

**12.** The feedstuff according to claim 11 wherein the monogastric animals are poultry and/or swine.

**13.** A method of increasing weight gain in poultry or swine comprising feeding said poultry or swine a feedstuff comprising a pepsin resistant alpha amylase;
wherein the pepsin resistant amylase has an amino acid sequence:

i) as set forth in SEQ ID No. 1;
ii) having at least 85% identity to SEQ ID No. 1;
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code; or
v) which is produced by expression of a nucleotide sequence which has at least 70% identity to SEQ ID No. 2.

**14.** Use of a feed supplement comprising a pepsin resistant alpha amylase for improving monogastric animal performance and/or increasing energy absorption and/or feed efficacy and/or for improving weight gain in a monogastric animal;
wherein the pepsin resistant amylase has an amino acid sequence:

i) as set forth in SEQ ID No. 1;
ii) having at least 85% identity to SEQ ID No. 1;
iii) which is produced by expression of a nucleotide sequence comprising the sequence of SEQ ID No. 2;
iv) which is produced by expression of a nucleotide sequence which differs from SEQ ID No. 2 due to the degeneracy of the genetic code; or
v) which is produced by expression of a nucleotide sequence which has at least 70% identity to SEQ ID No. 2.

**15.** The use according to claim 14, wherein the pepsin resistant alpha amylase is used for improving starch digestibility.

**16.** A method of increasing weight gain in poultry or swine comprising feeding said poultry or swine a feedstuff comprising a pepsin resistant alpha amylase;
wherein the pepsin resistant alpha amylase on gap alignment with SEQ ID No. 1 comprises any one or more of the following amino acids selected from the group consisting of: K88; 1103; H133; Y175; Y290; F292; R442 and H450, wherein the amino acid numbering relates to SEQ ID No. 1.

**17.** Use of a feed supplement comprising a pepsin resistant alpha amylase for improving monogastric animal performance and/or increasing energy absorption and/or feed efficacy and/or for improving weight gain in a monogastric animal;
wherein the pepsin resistant alpha amylase on gap alignment with SEQ ID No. 1 comprises any one or more of the following amino acids selected from the group consisting of: K88; 1103; H133; Y175; Y290; F292; R442 and H450, wherein the amino acid numbering relates to SEQ ID No. 1.

**18.** The use according to claim 17, wherein the pepsin resistant alpha amylase is used for improving starch digestibility.

## Patentansprüche

**1.** Verfahren zum Herstellen eines Futterzusatzes für ein monogastrisches Tier, umfassend Mischen einer pepsinresistenten alpha-Amylase mit mindestens einem physiologisch verträglichen Träger, ausgewählt aus der Gruppe, bestehend aus Maltodextrin, Kalkstein (Calciumcarbonat), Cyclodextrin, Weizen oder einer Weizenkomponente, Saccharose, Stärke, $Na_2SO_4$, Talk, PVA, Sorbitol, Benzoat, Sorbat, Glycerol, Saccharose, Propylenglycol, 1,3-Propandiol, Glucose, Parabenen, Natriumchlorid, Citrat, Acetat, Phosphat, Calcium, Metabisulfit, Formiat und Gemischen davon; wobei die pepsinresistente Amylase eine Aminosäuresequenz aufweist:

i) wie angegeben in SEQ ID No. 1;
ii) aufweisend mindestens 85 % Identität mit SEQ ID No. 1;
iii) welche durch Expression einer Nucleotidsequenz, umfassend die Sequenz von SEQ ID No. 2, erzeugt wird;
iv) welche durch Expression einer Nukleotidsequenz erzeugt wird, welche sich aufgrund der Degeneriertheit des genetischen Codes von SEQ ID Nr. 2 unterscheidet; oder
v) welche durch Expression einer Nucleotidsequenz erzeugt wird, welche mindestens 70 % Identität mit SEQ ID No. 2 aufweist.

2. Futterzusatz, welcher eines von Folgendem ist:

a) ein Geflügelfutterzusatz, umfassend eine pepsinresistente alpha-Amylase; oder
b) ein Futterzusatz für monogastrische Tiere, umfassend eine pepsinresistente alpha-Amylase und mindestens einen physiologisch verträglichen Träger, ausgewählt aus der Gruppe, bestehend aus Maltodextrin, Kalkstein (Calciumcarbonat), Cyclodextrin, Weizen oder einer Weizenkomponente, Saccharose, Stärke, $Na_2SO_4$, Talk, PVA, Sorbitol, Benzoat, Sorbat, Glycerol, Saccharose, Propylenglycol, 1,3-Propandiol, Glucose, Parabenen, Natriumchlorid, Citrat, Acetat, Phosphat, Calcium, Metabisulfit, Formiat und Gemischen davon; wobei die pepsinresistente Amylase eine Aminosäuresequenz aufweist:

i) wie angegeben in SEQ ID No. 1;
ii) aufweisend mindestens 85 % Identität mit SEQ ID No. 1;
iii) welche durch Expression einer Nucleotidsequenz, umfassend die Sequenz von SEQ ID No. 2, erzeugt wird;
iv) welche durch Expression einer Nukleotidsequenz erzeugt wird, welche sich aufgrund der Degeneriertheit des genetischen Codes von SEQ ID Nr. 2 unterscheidet; oder
v) welche durch Expression einer Nucleotidsequenz erzeugt wird, welche mindestens 70 % Identität mit SEQ ID No. 2 aufweist.

3. Futtermittel für ein monogastrisches Tier, umfassend eine pepsinresistente alpha-Amylase, wobei das Futtermittel weniger als 3 000 Einheiten von alpha-Amylase pro Kilogramm Futter umfasst; wobei die pepsinresistente Amylase eine Aminosäuresequenz aufweist:

i) wie angegeben in SEQ ID No. 1;
ii) aufweisend mindestens 85 % Identität mit SEQ ID No. 1;
iii) welche durch Expression einer Nucleotidsequenz, umfassend die Sequenz von SEQ ID No. 2, erzeugt wird;
iv) welche durch Expression einer Nukleotidsequenz erzeugt wird, welche sich aufgrund der Degeneriertheit des genetischen Codes von SEQ ID Nr. 2 unterscheidet; oder
v) welche durch Expression einer Nucleotidsequenz erzeugt wird, welche mindestens 70 % Identität mit SEQ ID No. 2 aufweist.

4. Verfahren zum Herstellen eines Futterzusatzes für ein monogastrisches Tier, umfassend Mischen einer pepsinresistenten alpha-Amylase mit mindestens einem physiologisch verträglichen Träger, ausgewählt aus der Gruppe, bestehend aus Maltodextrin, Kalkstein (Calciumcarbonat), Cyclodextrin, Weizen oder einer Weizenkomponente, Saccharose, Stärke, $Na_2SO_4$, Talk, PVA, Sorbitol, Benzoat, Sorbat, Glycerol, Saccharose, Propylenglycol, 1,3-Propandiol, Glucose, Parabenen, Natriumchlorid, Citrat, Acetat, Phosphat, Calcium, Metabisulfit, Formiat und Gemischen davon;
wobei die pepsinresistente alpha-Amylase bei Lückenausrichtung mit SEQ ID No. 1 eine oder mehrere von den folgenden Aminosäuren umfasst, ausgewählt aus der Gruppe, bestehend aus: K88; 1103; H133; Y175; Y290; F292; R442 und H450, umfasst, wobei sich die Aminosäurenummerierung auf SEQ ID No. 1 bezieht.

5. Futterzusatz, welcher Folgendes ist:

a) ein Geflügelfutterzusatz, umfassend eine pepsinresistente alpha-Amylase; oder
b) ein Futterzusatz für monogastrische Tiere, umfassend eine pepsinresistente alpha-Amylase und mindestens einen physiologisch verträglichen Träger, ausgewählt aus der Gruppe, bestehend aus Maltodextrin, Kalkstein (Calciumcarbonat), Cyclodextrin, Weizen oder einer Weizenkomponente, Saccharose, Stärke, $Na_2SO_4$, Talk, PVA, Sorbitol, Benzoat, Sorbat, Glycerol, Saccharose, Propylenglycol, 1,3-Propandiol, Glucose, Parabenen, Natriumchlorid, Citrat, Acetat, Phosphat, Calcium, Metabisulfit, Formiat und Gemischen davon ist;

wobei die pepsinresistente alpha-Amylase bei Lückenausrichtung mit SEQ ID No. 1 eine oder mehrere von den folgenden Aminosäuren umfasst, ausgewählt aus der Gruppe, bestehend aus: K88; 1103; H133; Y175; Y290; F292; R442 und H450, umfasst, wobei sich die Aminosäurenummerierung auf SEQ ID No. 1 bezieht.

6. Futtermittel für ein monogastrisches Tier, umfassend eine pepsinresistente alpha-Amylase, wobei das Futtermittel weniger als 3 000 Einheiten von alpha-Amylase pro Kilogramm Futter umfasst; wobei die pepsinresistente alpha-Amylase bei Lückenausrichtung mit SEQ ID No. 1 eine oder mehrere von den folgenden Aminosäuren umfasst, ausgewählt aus der Gruppe, bestehend aus: K88; 1103; H133; Y175; Y290; F292; R442 und H450, umfasst, wobei sich die Aminosäurenummerierung auf SEQ ID No. 1 bezieht.

**7.** Verfahren zum Herstellen eines Futtermittels für ein monogastrisches Tier, umfassend Mischen von Futterzusatz gemäß einem der Ansprüche 2 oder 5 mit einem oder mehreren Futtermaterialien.

**8.** Verfahren gemäß Anspruch 7, wobei das Futtermittel für Geflügel bestimmt ist.

**9.** Verfahren gemäß Anspruch 7 oder 8, wobei das eine oder die mehreren Futtermaterialien aus der Gruppe, bestehend aus a) Getreide mit kleinen Körnern oder mit großen Körnern; b) Nebenprodukten von Getreide; c) Proteinen; d) Ölen und Fetten, erhalten aus pflanzlichen und tierischen Ausgangsstoffen; und e) Mineralstoffen und Vitaminen, ausgewählt sind.

**10.** Verfahren gemäß Anspruch 9, wobei das Getreide mit kleinen Körnern aus Weizen, Gerste, Roggen, Hafer und Kombinationen davon ausgewählt ist; das Getreide mit großen Körnern aus Mais und Sorghum ausgewählt ist; die Nebenprodukte von Getreide aus Maisglutenmehl, Trockenschlempe (DDGS), Weizenkleie, Weizenmittelmehl, feiner Weizenkleie, Reiskleie, Reishülsen, Haferspelzen, Palmkern und Citruspulpe ausgewählt sind; und/oder die Proteine aus Soja, Sonnenblume, Erdnuss, Lupine, Erbsen, Fava-Bohnen, Baumwolle, Canola, Fischmehl, getrocknetem Plasmaprotein, Fleisch- und Knochenmehl, Kartoffelprotein, Molke, Kopra und/oder Sesam erhalten werden.

**11.** Futtermittel für ein monogastrisches Tier, hergestellt durch das Verfahren nach einem der Ansprüche 7 bis 10 oder umfassend den Futterzusatz nach einem der Ansprüche 2 oder 5.

**12.** Futtermittel gemäß Anspruch 11, wobei die monogastrischen Tiere Geflügel und/oder Schwein sind.

**13.** Verfahren zum Erhöhen der Gewichtszunahme bei Geflügel oder Schwein, umfassend Füttern des Geflügels oder Schweines mit einem Futtermittel, umfassend eine pepsinresistente alpha-Amylase;
wobei die pepsinresistente Amylase eine Aminosäuresequenz aufweist:

i) wie angegeben in SEQ ID No. 1;
ii) aufweisend mindestens 85 % Identität mit SEQ ID No. 1;
iii) welche durch Expression einer Nucleotidsequenz, umfassend die Sequenz von SEQ ID No. 2, erzeugt wird;
iv) welche durch Expression einer Nukleotidsequenz erzeugt wird, welche sich aufgrund der Degeneriertheit des genetischen Codes von SEQ ID Nr. 2 unterscheidet; oder
v) welche durch Expression einer Nucleotidsequenz erzeugt wird, welche mindestens 70 % Identität mit SEQ ID No. 2 aufweist.

**14.** Verwendung eines Futterzusatzes, umfassend eine pepsinresistente alpha-Amylase zum Verbessern der Leistung monogastrischer Tiere und/oder Erhöhen der Energieaufnahme und/oder Futterwirksamkeit und/oder zum Verbessern der Gewichtszunahme bei einem monogastrischen Tier;
wobei die pepsinresistente Amylase eine Aminosäuresequenz aufweist:

i) wie angegeben in SEQ ID No. 1;
ii) aufweisend mindestens 85 % Identität mit SEQ ID No. 1;
iii) welche durch Expression einer Nucleotidsequenz, umfassend die Sequenz von SEQ ID No. 2, erzeugt wird;
iv) welche durch Expression einer Nukleotidsequenz erzeugt wird, welche sich aufgrund der Degeneriertheit des genetischen Codes von SEQ ID Nr. 2 unterscheidet; oder
v) welche durch Expression einer Nucleotidsequenz erzeugt wird, welche mindestens 70 % Identität mit SEQ ID No. 2 aufweist.

**15.** Verwendung gemäß Anspruch 14, wobei die pepsinresistente alpha-Amylase zum Verbessern der Stärkeverdaulichkeit verwendet wird.

**16.** Verfahren zum Erhöhen der Gewichtszunahme bei Geflügel oder Schwein, umfassend Füttern des Geflügels oder Schweines mit einem Futtermittel, umfassend eine pepsinresistente alpha-Amylase;
wobei die pepsinresistente alpha-Amylase bei Lückenausrichtung mit SEQ ID No. 1 eine oder mehrere von den folgenden Aminosäuren umfasst, ausgewählt aus der Gruppe, bestehend aus: K88; 1103; H133; Y175; Y290; F292;

R442 und H450, umfasst, wobei sich die Aminosäurenummerierung auf SEQ ID No. 1 bezieht.

**17.** Verwendung eines Futterzusatzes, umfassend eine pepsinresistente alpha-Amylase zum Verbessern der Leistung monogastrischer Tiere und/oder Erhöhen der Energieaufnahme und/oder Futterwirksamkeit und/oder zum Verbessern der Gewichtszunahme bei einem monogastrischen Tier;
wobei die pepsinresistente alpha-Amylase bei Lückenausrichtung mit SEQ ID No. 1 eine oder mehrere von den folgenden Aminosäuren umfasst, ausgewählt aus der Gruppe, bestehend aus: K88; 1103; H133; Y175; Y290; F292; R442 und H450, umfasst, wobei sich die Aminosäurenummerierung auf SEQ ID No. 1 bezieht.

**18.** Verwendung gemäß Anspruch 17, wobei die pepsinresistente alpha-Amylase zum Verbessern der Stärkeverdaulichkeit verwendet wird.

**Revendications**

**1.** Procédé de préparation d'un supplément alimentaire pour un animal monogastrique, comprenant mélanger par admixtion une alpha amylase résistante à la pepsine avec au moins un véhicule physiologiquement acceptable sélectionné parmi le groupe consistant en maltodextrine, calcaire (carbonate de calcium), cyclodextrine, blé ou un composant du blé, saccharose, amidon, $Na_2SO_4$, talc, PVA, sorbitol, benzoate, sorbiate, glycérol, saccharose, propylène glycol, 1,3-propane diol, glucose, parabènes, chlorure de sodium, citrate, acétate, phosphate, calcium, métabisulfite, formiate et des mélanges de ceux-ci, dans lequel l'amylase résistante à la pepsine a une séquence d'acides aminés :

i) telle que présentée dans la SEQ ID NO: 1 ;
ii) ayant au moins 85 % d'identité à la SEQ ID NO: 1 ;
iii) qui est produite par l'expression d'une séquence de nucléotides comprenant la séquence de la SEQ ID NO: 2 ;
iv) qui est produite par l'expression d'une séquence de nucléotides qui diffère de la SEQ ID NO: 2 du fait de la dégénérescence du code génétique ; ou
v) qui est produite par l'expression d'une séquence de nucléotides qui a au moins 70 % d'identité à la SEQ ID NO: 2.

**2.** Supplément alimentaire pour animaux qui est :

a) un supplément alimentaire pour volaille comprenant une alpha amylase résistante à la pepsine ; ou
b) un supplément alimentaire pour animaux monogastriques comprenant une alpha amylase résistante à la pepsine et au moins un véhicule physiologiquement acceptable sélectionné parmi le groupe consistant en maltodextrine, calcaire (carbonate de calcium), cyclodextrine, blé ou un composant du blé, saccharose, amidon, $Na_2SO_4$, talc, PVA, sorbitol, benzoate, sorbiate, glycérol, saccharose, propylène glycol, 1,3-propane diol, glucose, parabènes, chlorure de sodium, citrate, acétate, phosphate, calcium, métabisulfite, formiate et des mélanges de ceux-ci; dans lequel l'amylase résistante à la pepsine a une séquence d'acides aminés :

i) telle que présentée dans la SEQ ID NO: 1 ;
ii) ayant au moins 85 % d'identité à la SEQ ID NO: 1 ;
iii) qui est produite par l'expression d'une séquence de nucléotides comprenant la séquence de la SEQ ID NO: 2 ;
iv) qui est produite par l'expression d'une séquence de nucléotides qui diffère de la SEQ ID NO: 2 du fait de la dégénérescence du code génétique ; ou
v) qui est produite par l'expression d'une séquence de nucléotides qui a au moins 70 % d'identité à la SEQ ID NO: 2.

**3.** Aliment pour animaux pour un animal monogastrique comprenant une alpha amylase résistante à la pepsine, dans lequel ledit aliment pour animaux comprend moins de 3 000 unités d'alpha amylase par kilogramme d'aliment pour animaux ; dans lequel l'amylase résistante à la pepsine a une séquence d'acides aminés :

i) telle que présentée dans la SEQ ID NO: 1 ;
ii) ayant au moins 85 % d'identité à la SEQ ID NO: 1 ;
iii) qui est produite par l'expression d'une séquence de nucléotides comprenant la séquence de la SEQ ID NO: 2 ;
iv) qui est produite par l'expression d'une séquence de nucléotides qui diffère de la SEQ ID NO: 2 du fait de la dégénérescence du code génétique ; ou
v) qui est produite par l'expression d'une séquence de nucléotides qui a au moins 70 % d'identité à la SEQ ID NO: 2.

**4.** Procédé de préparation d'un supplément alimentaire pour un animal monogastrique, comprenant mélanger par admixtion une alpha amylase résistante à la pepsine avec au moins un véhicule physiologiquement acceptable sélectionné parmi le groupe consistant en maltodextrine, calcaire (carbonate de calcium), cyclodextrine, blé ou un composant du blé, saccharose, amidon, $Na_2SO_4$, talc, PVA, sorbitol, benzoate, sorbiate, glycérol, saccharose, propylène glycol, 1,3-propane diol, glucose, parabènes, chlorure de sodium, citrate, acétate, phosphate, calcium, métabisulfite, formiate et des mélanges de ceux-ci ;
dans lequel l'alpha amylase résistante à la pepsine sur l'alignement de gap avec la SEQ ID NO: 1, comprend l'un quelconque ou plusieurs des acides aminés suivants sélectionnés parmi le groupe consistant en : K88 ; 1103 ; H133 ; Y175 ; Y290 ; F292 ; R442 et H450, où la numérotation des acides aminés se rapporte à la SEQ ID NO: 1.

**5.** Supplément alimentaire pour animaux qui est :

a) un supplément alimentaire pour volaille comprenant une alpha amylase résistante à la pepsine ; ou
b) un supplément alimentaire pour animaux monogastriques comprenant une alpha amylase résistante à la pepsine et au moins un véhicule physiologiquement acceptable sélectionné parmi le groupe consistant en maltodextrine, calcaire (carbonate de calcium), cyclodextrine, blé ou un composant du blé, saccharose, amidon, $Na_2SO_4$, talc, PVA, sorbitol, benzoate, sorbiate, glycérol, saccharose, propylène glycol, 1,3-propane diol, glucose, parabènes, chlorure de sodium, citrate, acétate, phosphate, calcium, métabisulfite, formiate et des mélanges de ceux-ci ;

dans lequel l'alpha amylase résistante à la pepsine sur l'alignement de gap avec la SEQ ID NO: 1, comprend l'un quelconque ou plusieurs des acides aminés suivants sélectionnés parmi le groupe consistant en : K88 ; 1103 ; H133 ; Y175 ; Y290 ; F292 ; R442 et H450, où la numérotation des acides aminés se rapporte à la SEQ ID NO: 1.

**6.** Aliment pour animaux pour un animal monogastrique comprenant une alpha amylase résistante à la pepsine, dans lequel ledit aliment pour animaux comprend moins de 3 000 unités d'alpha amylase par kilogramme d'aliment pour animaux ; dans lequel l'alpha amylase résistante à la pepsine sur l'alignement de gap avec la SEQ ID NO: 1 comprend l'un quelconque ou plusieurs des acides aminés suivants sélectionnés parmi le groupe consistant en : K88 ; 1103 ; H133 ; Y175 ; Y290 ; F292 ; R442 et H450, où la numérotation des acides aminés se rapporte à la SEQ ID NO: 1.

**7.** Procédé de préparation d'un aliment pour animaux destiné à un animal monogastrique comprenant mélanger un supplément alimentaire pour animaux selon l'une quelconque des revendications 2 ou 5, avec une ou plusieurs substances alimentaires pour animaux.

**8.** Procédé selon la revendication 7, dans lequel l'aliment pour animaux est destiné à la volaille.

**9.** Procédé selon la revendication 7 ou 8, dans lequel l'une ou les plusieurs substances alimentaires pour animaux sont sélectionnées parmi le groupe consistant en : a) céréales à petits grains ou à gros grains ; b) sous-produits céréaliers ; c) protéines ; d) huiles et graisses obtenues de sources végétales et animales ; et e) minéraux et vitamines.

**10.** Procédé selon la revendication 9, dans lequel les céréales à petits grains sont sélectionnées parmi : blé, orge, seigle, avoine et des combinaisons de ceux-ci ; les céréales à gros grains sont sélectionnées parmi : maïs et sorgho ; les sous-produits céréaliers sont sélectionnés parmi : farine de gluten de maïs, drêche de distillerie sèche avec solubles (DDGS), son de blé, issues de blé, farine basse de blé, son de riz, cosses de riz, cosses d'avoine, coeur de palmier et pulpe d'agrumes ; et/ou les protéines sont obtenues à partir de soja, tournesol, arachide, lupin, pois, haricots de fava, coton, colza, farine de poisson, protéine de plasma sec, farine de viande et d'os, protéine de pomme de terre, lactosérum, copra et/ou sésame.

**11.** Aliment pour animaux pour un animal monogastrique préparé par le procédé selon l'une quelconque des revendications 7 à 10 ou comprenant le supplément alimentaire pour animaux selon l'une quelconque des revendications 2 ou 5.

**12.** Aliment pour animaux selon la revendication 11, où les animaux monogastriques sont la volaille et/ou le porc.

**13.** Procédé destiné à augmenter le gain de poids chez la volaille ou le porc, comprenant administrer à ladite volaille ou audit porc un aliment pour animaux comprenant une alpha amylase résistante à la pepsine ;
dans lequel l'amylase résistante à la pepsine a une séquence d'acides aminés :

i) telle que présentée dans la SEQ ID NO: 1 ;
ii) ayant au moins 85 % d'identité à la SEQ ID NO: 1 ;
iii) qui est produite par l'expression d'une séquence de nucléotides comprenant la séquence de la SEQ ID NO: 2 ;
iv) qui est produite par l'expression d'une séquence de nucléotides qui diffère de la SEQ ID NO: 2 du fait de la dégénérescence du code génétique ; ou
v) qui est produite par l'expression d'une séquence de nucléotides qui a au moins 70 % d'identité à la SEQ ID NO: 2.

**14.** Utilisation d'un supplément alimentaire pour animaux comprenant une alpha amylase résistante à la pepsine pour améliorer la performance de l'animal monogastrique et/ou augmenter l'absorption d'énergie et/ou l'efficacité alimentaire et/ou pour améliorer le gain de poids chez un animal monogastrique :

dans lequel l'amylase résistante à la pepsine a une séquence d'acides aminés :

i) telle que présentée dans la SEQ ID NO: 1 ;
ii) ayant au moins 85 % d'identité à la SEQ ID NO: 1 ;
iii) qui est produite par l'expression d'une séquence de nucléotides comprenant la séquence de la SEQ ID NO: 2 ;
iv) qui est produite par l'expression d'une séquence de nucléotides qui diffère de la SEQ ID NO: 2 du fait de la dégénérescence du code génétique ; ou
v) qui est produite par l'expression d'une séquence de nucléotides qui a au moins 70 % d'identité à la SEQ ID NO: 2.

**15.** Utilisation selon la revendication 14, où l'alpha amylase résistante à la pepsine est utilisée pour améliorer la digestibilité de l'amidon.

**16.** Procédé destiné à augmenter le gain de poids chez la volaille ou le porc, comprenant administrer à ladite volaille ou audit porc un aliment pour animaux comprenant une alpha amylase résistante à la pepsine ;
dans lequel l'alpha amylase résistante à la pepsine sur l'alignement de gap avec la SEQ ID NO: 1, comprend l'un quelconque ou plusieurs des acides aminés suivants sélectionnés parmi le groupe consistant en : K88 ; 1103 ; H133 ; Y175 ; Y290 ; F292 ; R442 et H450, où la numérotation des acides aminés se rapporte à la SEQ ID NO: 1.

**17.** Utilisation d'un supplément alimentaire pour animaux comprenant une alpha amylase résistante à la pepsine pour améliorer la performance de l'animal monogastrique et/ou augmenter l'absorption d'énergie et/ou l'efficacité alimentaire et/ou pour améliorer le gain de poids chez un animal monogastrique ;
dans lequel l'alpha amylase résistante à la pepsine sur l'alignement de gap avec la SEQ ID NO: 1, comprend l'un quelconque ou plusieurs des acides aminés suivants sélectionnés parmi le groupe consistant en : K88 ; 1103 ; H133 ; Y175 ; Y290 ; F292 ; R442 et H450, où la numérotation des acides aminés se rapporte à la SEQ ID NO: 1.

**18.** Utilisation selon la revendication 17, où l'alpha amylase résistante à la pepsine est utilisée pour améliorer la digestibilité de l'amidon.

## FIGURE 1

1 anlngtlmqy fewympndgq hwkrlqndsa ylaehgitav wippaykgts qadvgygayd 60
61 lydlgefhqk gtvrtkygtk gelqsaiksl hsrdinvygd vvinhkggad atedvtavev 120
121 dpadrnrvis gehlikawth fhfpgrgsty sdfkwhwyhf dgtdwdesrk lnriykfqgk 180
181 awdwevsnen gnydylmyad idydhpdvaa eikrwgtwya nelqldgfrl davkhikfsf 240
241 lrdwvnhvre ktgkemftva eywqndlgal enylnktnfn hsvfdvplhy qfhaastqgg 300
301 gydmrkllng tvvskhplks vtfvdnhdtq pgqslestvq twfkplayaf iltresgypq 360
361 vfygdmygtk gdsqreipal khkiepilka rkqyaygaqh dyfdhhdivg wtregdssva 420
421 nsglaalitd gpggakrmyv grqnagetwh ditgnrsepv vinsegwgef hvnggsvsiy 480
481 vqr

## FIGURE 2

gcaaatcttaatgggacgctgatgcagtattttgaatggtacatgcccaatgacggccaacattggaagcgtttg
caaaacgactcggcatatttggctgaacacggtattactgccgtctggattcccccggcatataagggaacgagc
caagcggatgtgggctacggtgcttacgacctttatgatttaggggagtttcatcaaaaagggacggttcggaca
aagtacggcacaaaaggagagctgcaatctgcgatcaaaagtcttcattcccgcgacattaacgtttacggggat
gtggtcatcaaccacaaaggcggcgctgatgcgaccgaagatgtaaccgcggttgaagtcgatcccgctgaccgc
aaccgcgtaatttcaggagaacacctaattaaagcctggacacattttcattttccggggcgcggcagcacatac
agcgattttaaatggcattggtaccattttgacggaaccgattgggacgagtcccgaaagctgaaccgcatctat
aagtttcaaggaaaggcttgggattgggaagtttccaatgaaaacggcaactatgattatttgatgtatgccgac
atcgattatgaccatcctgatgtcgcagcagaaattaagagatggggcacttggtatgccaatgaactgcaattg
gacggtttccgtcttgatgctgtcaaacacattaaatttctttttgcgggattgggttaatcatgtcagggaa
aaaacggggaaggaaatgtttacggtagctgaatattggcagaatgacttgggcgcgctggaaaactatttgaac
aaaacaaattttaatcattcagtgtttgacgtgccgcttcattatcagttccatgctgcatcgacacagggaggc
ggctatgatatgaggaaattgctgaacggtacggtcgtttccaagcatccgttgaaatcggttacatttgtcgat
aaccatgatacacagccggggcaatcgcttgagtcgactgtccaaacatggtttaagccgcttgcttacgctttt
attctcacaagggaatctggataccctcaggttttctacggggatatgtacgggacgaaaggagactcccagcgc
gaaattcctgccttgaaacacaaaattgaaccgatcttaaaagcgagaaaacagtatgcgtacggagcacagcat
gattatttcgaccaccatgacattgtcggctggacaagggaaggcgacagctcggttgcaaattcaggtttggcg
gcattaataacagacggacccggtggggcaaagcgaatgtatgtcggccggcaaaacgccggtgagacatggcat
gacattaccggaaaccgttcggagccggttgtcatcaattcggaaggctggggagagtttcacgtaaacggcggg
tcggtttcaatttatgttcaaagatga

FIGURE 3

1 MKLRYALPLL LQLSLPVLSA DTAAWRSRTI YFALTDRIAR GSGDTGGSAC GNLGDYCGGT 60
61 FQGLESKLDY IKGMGFDAIW ITPVVTSDDG GYHGYWAEDI DSINSHYGSA DDLKSLVNAA 120
121 HSKGFYMMVD VVANHMGYAN ISDDSPSPLN QASSYHPECD IDYNNQTSVE NCWISGLPDL 180
181 NTQSSTIRSL YQDWVSNLVS TYGFDGVRID TVKHVEQDYW PGFVNATGVY CIGEVFDGDP 240
241 NYLLPYASLM PGLLNYAIYY PMTRFFLQQG SSQDMVNMHD QIGSMFPDPT ALGTFVDNHD 300
301 NPRFLSIKND TALLKNALTY TILSRGIPIV YYGTEQAFSG GNDPANREDL WRSGFNAQSD 360
361 MYDAISKLTY AKHAVGGLAD NDHKHLYVAD TAYAFSRAGG NMVALTTNSG SGSSAQHCFG 420
421 TQVPNGRWQN VFDEGNGPTY SADGNGQLCL NVSNGQPIVL LSS

FIGURE 4

atgaagctccggtacgctctcccgctgctcttgcagctctctttgccggtcctctccgcagacaccgccgcctggaggtcccgcacc
atctactttgccctgacagaccgcatcgctcgtggaagcggtgacacgggggcagtgcgtgtgggaacctgggggactactgc
ggtggcacgttccagggcttggagagcaagttggactacatcaagggcatgggattcgatgccatctggatcacacctgttgtgac
gagtgatgatgggggctaccatggctattgggcggaggacatcgactccatcaactctcattatggctctgcggacgatctcaaga
gtctcgtcaacgccgcgcatagcaagggcttctatatgatggtggacgtcgtggccaaccacatgggctacgccaatatctctgac
gatagtccctctccactgaaccaggcctcgtcgtatcaccccgagtgtgatatcgactacaacaaccaaaccagcgtcgagaact
gctggatcagcggcctcccggatctcaacacgcagagctcaaccatccgcagcctctaccaggactgggtctccaacctcgtgtc
cacgtacggcttcgacggcgtccgcatcgacaccgtcaagcacgtcgagcaagactactggcccggcttcgtcaacgccaccg
gcgtctactgcatcggcgaggtctttgacggagacccaaactacctgctgccctacgccagcctcatgccgggcctgctcaacta
cgccatctactaccccatgacgcgcttcttcctccagcagggctcctcgcaggacatggtcaacatgcacgaccagatcggcagc
atgttccccgacccgaccgcgctcggcacctttgtcgacaaccacgacaacccgcgcttcctgagcatcaagaacgacacggc
cctgctcaagaacgcgctgacgtacaccatcctctcgcgcggcatccccatcgtctactacggcaccgagcaggccttctcgggc
ggcaacgacccggccaacagggaggacctctggcgcagcggcttcaacgcccagtccgacatgtacgacgccatctccaag
ctcacctacgccaagcacgccgtcggcggcctcgccgacaacgaccacaagcacctgtacgtcgccgacacggcctacgcct
tcagccgcgccggcggcaacatggtggccctgaccaccaacagcggcagcgggagctcggcccagcactgcttcggcacgc
aggtgcccaacggccgctggcagaatgtctttgacgagggcaatggccgacgtattccgccgacggcaacggccagctttgct
tgaatgtgtccaacggtcagcccattgtcttgctgtcttcgtga

**FIGURE 5**

```
                1                                                 50
 LAT      (1)  ANLNGTLMQYFEWYMPNDGQHWKRLQNDSAYLAEHGITAVWIPPAYKGTS
LTAA      (1)  --VNGTLMQYFEWYTPNDGQHWKRLQNDAEHLSDIGITAVWIPPAYKGLS
               51                                                100
 LAT     (51)  QADVGYGAYDLYDLGEFHQKGTVRTKYGTKGELQSAIKSLHSRDINVYGD
LTAA     (49)  QSDNGYGPYDLYDLGEFQQKGTVRTKYGTKSELQDAIGSLHSRNVQVYGD
               101                                               150
 LAT    (101)  VVINHKGGADATEDVTAVEVDPADRNRVISGEHLIKAWTHFHFPGRGSTY
LTAA     (99)  VVLNHKAGADATEDVTAVEVNPANRNQETSEEYQIKAWTDFRFPGRGNTY
               151                                               200
 LAT    (151)  SDFKWHWYHFDGTDWDESRKLNRIYKFQG--KAWDWEVSNENGNYDYLMY
LTAA    (149)  SDFKWHWYHFDGADWDESRKISRIFKFRGEGKAWDWEVSSENGNYDYLMY
               201                                               250
 LAT    (199)  ADIDYDHPDVAAEIKRWGTWYANELQLDGFRLDAVKHIKFSFLRDWVNHV
LTAA    (199)  ADVDYDHPDVVAETKKWGIWYANELSLDGFRIDAAKHIKFSFLRDWVQAV
               251                                               300
 LAT    (249)  REKTGKEMFTVAEYWQNDLGALENYLNKTNFNHSVFDVPLHYQFHAASTQ
LTAA    (249)  RQATGKEMFTVAEYWQNNAGKLENYLNKTSFNQSVFDVPLHFNLQAASSQ
               301                                               350
 LAT    (299)  GGGYDMRKLLNGTVVSKHPLKSVTFVDNHDTQPGQSLESTVQTWFKPLAY
LTAA    (299)  GGGYDMRRLLDGTVVSRHPEKAVTFVENHDTQPGQSLESTVQTWFKPLAY
               351                                               400
 LAT    (349)  AFILTRESGYPQVFYGDMYGTKGDSQREIPALKHKIEPILKARKQYAYGA
LTAA    (349)  AFILTRESGYPQVFYGDMYGTKGTSPKEIPSLKDNIEPILKARKEYAYGP
               401                                               450
 LAT    (399)  QHDYFDHHDIVGWTREGDSSVANSGLAALITDGPGGAKRMYVGRQNAGET
LTAA    (399)  QHDYIDHPDVIGWTREGDSSAAKSGLAALITDGPGGSKRMYAGLKNAGET
               451                               485
 LAT    (449)  WHDITGNRSEPVVINSEGWGEFHVNGGSVSIYVQR
LTAA    (449)  WYDITGNRSDTVKIGSDGWGEFHVNDGSVSIYVQK
```

FIGURE 6

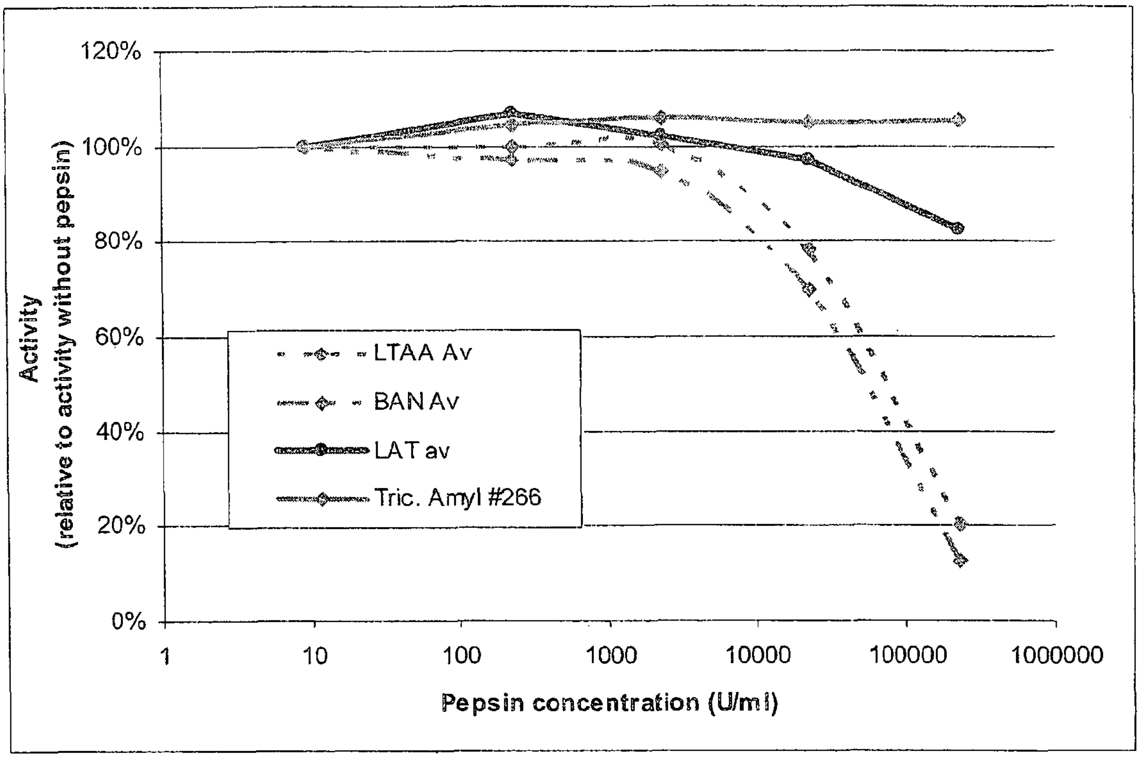
120%
100%
80%
60%
40%
20%
0%
Activity
(relative to activity without pepsin)
1
10
100
1000
10000
100000
1000000
Pepsin concentration (U/ml)
LTAA Av
BAN Av
LAT av
Tric. Amyl #266

FIGURE 7

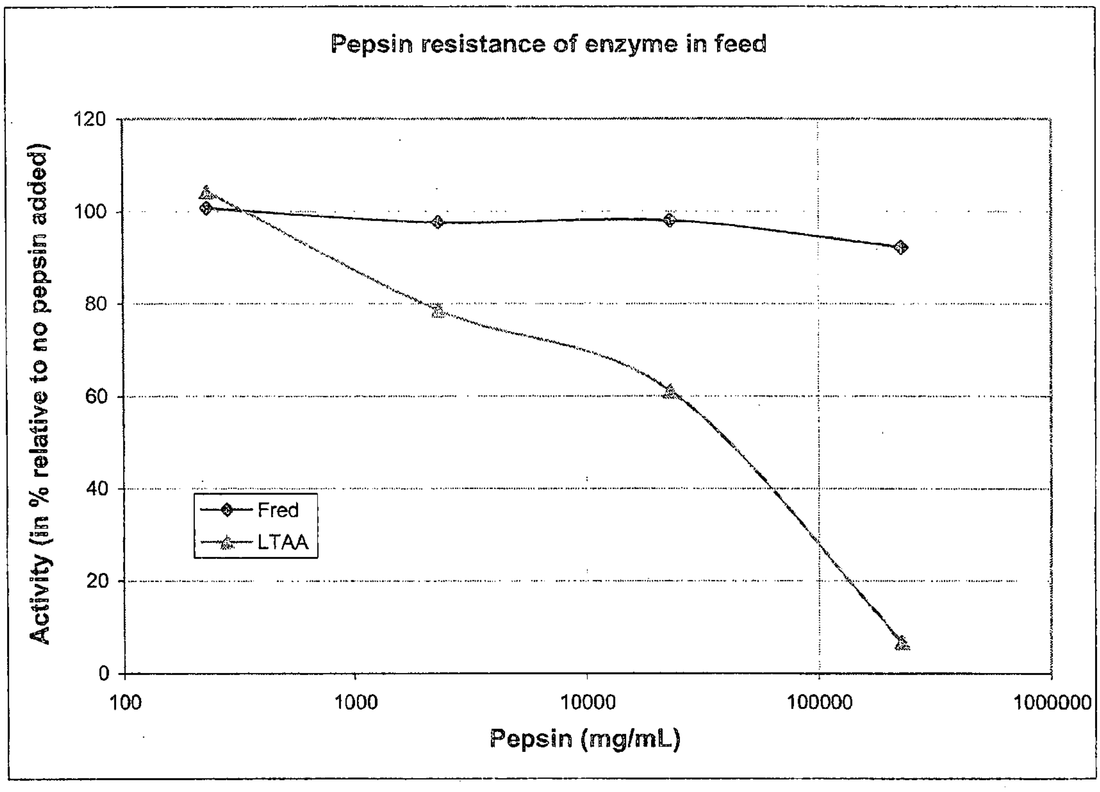
Pepsin resistance of enzyme in feed
Activity (in % relative to no pepsin added)
120
100
80
60
40
20
0
100
1000
10000
100000
1000000
Pepsin (mg/mL)
Fred
LTAA

FIGURE 8

ANLNGTLMQYFEWYTPNDGQHWKRLQNDSAYLAEHGITAVWIPPAYKGTSQADVGYGAYDLYDLGE
FHQKGTVRTKYGTKGELQSAIKSLHSRDINVYGDVVINHKGGADATEDVTAVEVDPADRNRVISGEYLI
KAWTHFHFPGRGSTYSDFKWHWYHFDGTDWDESRKLNRIYKFQGKAWDWEVSSENGNYDYLMYA
DIDYDHPDVVAEIKRWGTWYANELQLDGFRLDAVKHIKFSFLRDWVNHVREKTGKEMFTVAEYWQN
DLGALENYLNKTNFNHSVFDVPLHYQFHAASTQGGGYDMRKLLNGTVVSKHPLKSVTFVDNHDTQP
GQSLESTVQTWFKPLAYAFILTRESGYPQVFYGDMYGTKGDSQREIPALKHKIEPILKARKQYAYGAQ
HDYFDHHDIVGWTREGDSSVANSGLAALITDGPGGAKRMYVGRQNAGETWHDITGNRSEPVVINSE
GWGEFHVNGGSVSIYVQR

FIGURE 9

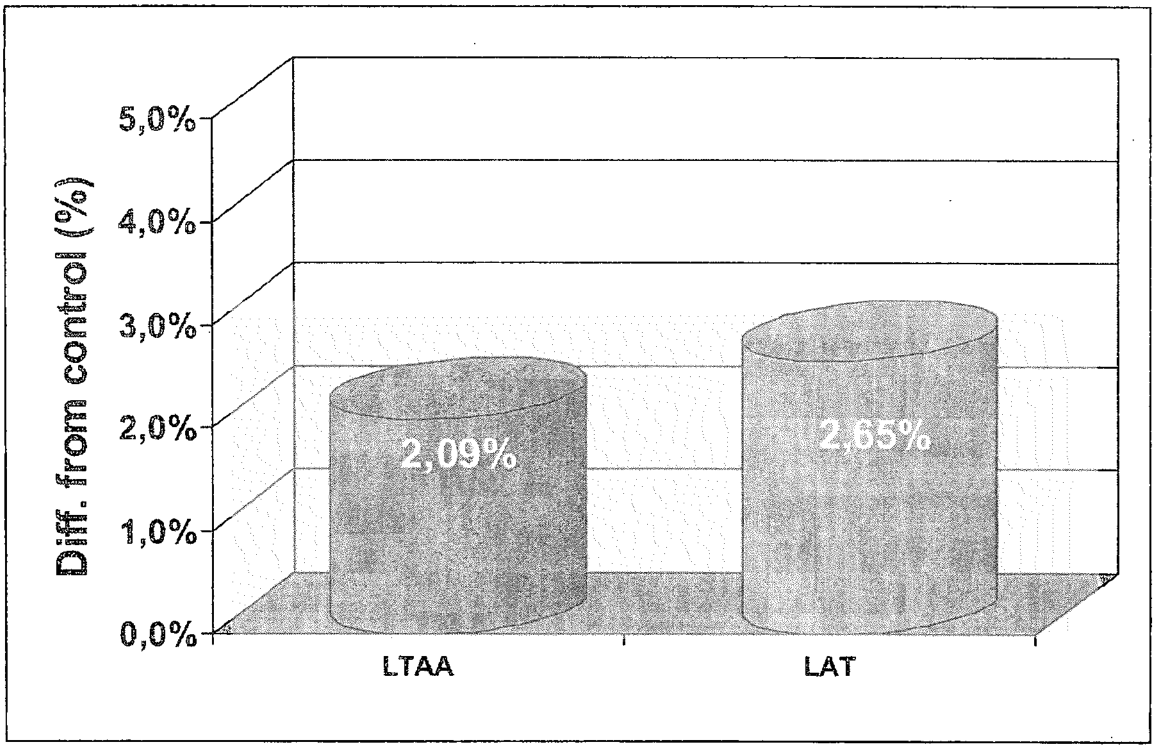

FIGURE 10

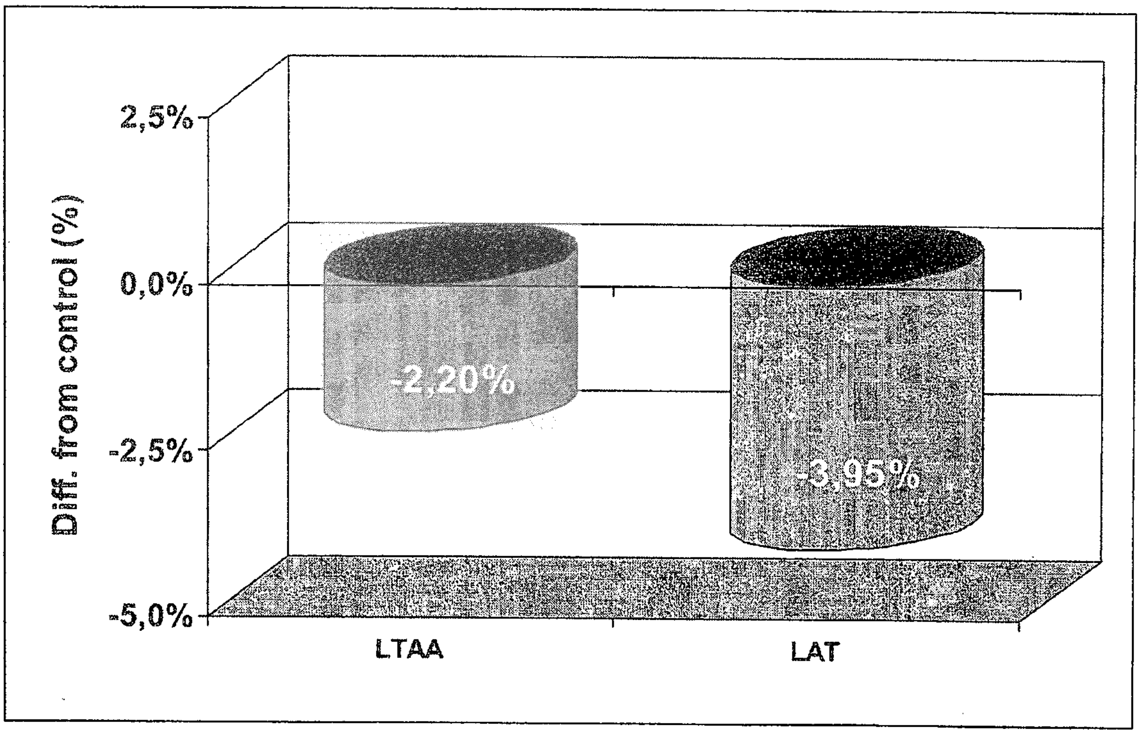

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008006881 A
- US 20080206401 A
- US 5372811 A
- WO 2008080093 A
- WO 2007044968 A
- US 4683202 A
- WO 9218645 A
- US 5723323 A
- US 5605793 A
- WO 0058517 A
- WO 9117243 A
- WO 9735956 A
- EP 0238023 A
- EP 0449375 A
- US 5741665 A
- US 5674707 A
- EP 120516 A
- US 3817837 A
- US 3850752 A
- US 3939350 A
- US 3996345 A
- US 4277437 A
- US 4275149 A
- US 4366241 A
- US 4816567 A
- GB 9821198 A
- WO 2009149271 A
- US 6211134 B
- US 5763385 A
- WO 2008112729 A
- GB 1011513 A
- US 61363865 B

### Non-patent literature cited in the description

- **SANDSTEDT, R. M. ; KNEEN, E. ; BLISH, M.J.** A Standardized Wohlgemuth procedure for alpha-amylase activity. *Cereal Chem.,* 1939, vol. 16, 712-723
- **BAILEY, M.J. ; BIELY, P. ; POUTANEN, K.** *Journal of Biotechnology,* May 1992, vol. 23 (3), 257-270
- **SINGLETON et al.** DICTIONARY OF MICROBIOLOGY AND MOLECULAR BIOLOGY. John Wiley and Sons, 1994
- **HALE ; MARHAM.** THE HARPER COLLINS DICTIONARY OF BIOLOGY. Harper Perennial, 1991
- **SIMON RJ et al.** *PNAS,* 1992, vol. 89 (20), 9367-9371
- **HORWELL DC.** *Trends Biotechnol.,* 1995, vol. 13 (4), 132-134
- **CARUTHERS MH et al.** *Nuc Acids Res Symp Ser,* 1980, 215-23
- **HORN T et al.** *Nuc Acids Res Symp Ser,* 1980, 225-232
- **BEUCAGE S.L. et al.** *Tetrahedron Letters,* 1981, vol. 22, 1859-1869
- **MATTHES et al.** *EMBO J.,* 1984, vol. 3, 801-805
- **SAIKI R K et al.** *Science,* 1988, vol. 239, 487-491
- **AUSUBEL et al.** Short Protocols in Molecular Biology. 1999
- *FEMS Microbiol Lett,* 1999, vol. 174 (2), 247-50
- *FEMS Microbiol Lett,* 1999, vol. 177 (1), 187-8
- **ALTSCHUL et al.** *J. Mol. Biol.,* 1990, 403-410
- **HIGGINS DG ; SHARP PM.** *Gene,* 1988, vol. 73 (1), 237-244
- **DEVEREUX et al.** *Nuc. Acids Research,* 1984, vol. 12, 387
- **AUSUBEL et al.** *Short Protocols in Molecular Biology,* 1999, 7-58, 7-60
- **MORINAGA et al.** *Biotechnology,* 1984, vol. 2, 646-649
- **NELSON ; LONG.** *Analytical Biochemistry,* 1989, vol. 180, 147-151
- **J. SAMBROOK ; E. F. FRITSCH ; T. MANIATIS.** Molecular Cloning: A Laboratory Manual. Cold Spring Harbor Laboratory Press, 1989, vol. 1-3
- **SAMBROOK et al.** Molecular Cloning: A Laboratory Manual. Cold Spring Harbor Laboratory Press, 1989
- **POTRYKUS.** *Annu Rev Plant Physiol Plant Mol Biol,* 1991, vol. 42, 205-225
- **CHRISTOU.** *Agro-Food-Industry Hi-Tech,* March 1994, 17-27
- **DAVIS ; DE SERRES.** *Methods Enzymol,* 1971, vol. 17A, 79-143

- **PUNT et al.** *Trends Biotechnol,* May 2002, vol. 20 (5), 200-6
- **ARCHER ; PEBERDY.** *Crit Rev Biotechnol,* 1997, vol. 17 (4), 273-306
- Vectors for genetic manipulation. **TURNER G.** Aspergillus: 50 years on. Progress in industrial microbiology. Elsevier, 1994, vol. 29, 641-666
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression of heterologous genes. **E HINCHCLIFFE ; E KENNY.** Yeasts. Academic Press Ltd, 1993, vol. 5
- **HINNEN et al.** *Proceedings of the National Academy of Sciences of the USA,* 1978, vol. 75, 1929
- **BEGGS, J D.** *Nature, London,* 1978, vol. 275, 104
- **ITO, H et al.** *J Bacteriology,* 1983, vol. 153, 163-168
- **BUTCHER D.N. et al.** Tissue Culture Methods for Plant Pathologists. 1980, 203-208
- **FRAME BR ; DRAYTON PR ; BAGNAALL SV ; LEWNAU CJ ; BULLOCK WP ; WILSON HM ; DUNWELL JM ; THOMPSON JA ; WANG K.** Production of fertile transgenic maize plants by silicon carbide whisker-mediated transformation. *The Plant Journal,* 1994, vol. 6, 941-948
- **MEYER P ; HEIDMANN I ; NIEDENHOF I.** The use of cassava mosaic virus as a vector system for plants. *Gene,* 1992, vol. 110, 213-217
- Binary Vectors. **GYNHEUNG AN et al.** Plant Molecular Biology Manual. 1980, vol. A3, 1-19
- **AN et al.** *Plant Physiol.,* 1986, vol. 81, 301-305
- **HOEKEMA.** The Binary Plant Vector System Offset-drukkerij. 1985
- **FRALEY et al.** *Crit. Rev. Plant Sci.,* vol. 4, 1-46
- **AN et al.** *EMBO J.,* 1985, vol. 4, 277-284
- *Methods Enzymol,* 1990, vol. 182, 132-43

SEQUENCE LISTING

<110> Danisco A/S

<120> METHOD

<130> P040743WO

<150> GB 1011513.7
<151> 2010-07-08

<150> US 61/363,865
<151> 2010-07-13

<160> 13

<170> PatentIn version 3.5

<210> 1
<211> 483
<212> PRT
<213> Bacillus licheniformis

<400> 1

```
Ala Asn Leu Asn Gly Thr Leu Met Gln Tyr Phe Glu Trp Tyr Met Pro
1               5                   10                  15

Asn Asp Gly Gln His Trp Lys Arg Leu Gln Asn Asp Ser Ala Tyr Leu
            20                  25                  30

Ala Glu His Gly Ile Thr Ala Val Trp Ile Pro Pro Ala Tyr Lys Gly
        35                  40                  45

Thr Ser Gln Ala Asp Val Gly Tyr Gly Ala Tyr Asp Leu Tyr Asp Leu
    50                  55                  60

Gly Glu Phe His Gln Lys Gly Thr Val Arg Thr Lys Tyr Gly Thr Lys
65                  70                  75                  80

Gly Glu Leu Gln Ser Ala Ile Lys Ser Leu His Ser Arg Asp Ile Asn
                85                  90                  95

Val Tyr Gly Asp Val Val Ile Asn His Lys Gly Gly Ala Asp Ala Thr
            100                 105                 110

Glu Asp Val Thr Ala Val Glu Val Asp Pro Ala Asp Arg Asn Arg Val
        115                 120                 125

Ile Ser Gly Glu His Leu Ile Lys Ala Trp Thr His Phe His Phe Pro
    130                 135                 140

Gly Arg Gly Ser Thr Tyr Ser Asp Phe Lys Trp His Trp Tyr His Phe
145                 150                 155                 160
```

```
Asp Gly Thr Asp Trp Asp Glu Ser Arg Lys Leu Asn Arg Ile Tyr Lys
                165                 170                 175

Phe Gln Gly Lys Ala Trp Asp Trp Glu Val Ser Asn Glu Asn Gly Asn
            180                 185                 190

Tyr Asp Tyr Leu Met Tyr Ala Asp Ile Asp Tyr Asp His Pro Asp Val
        195                 200                 205

Ala Ala Glu Ile Lys Arg Trp Gly Thr Trp Tyr Ala Asn Glu Leu Gln
    210                 215                 220

Leu Asp Gly Phe Arg Leu Asp Ala Val Lys His Ile Lys Phe Ser Phe
225                 230                 235                 240

Leu Arg Asp Trp Val Asn His Val Arg Glu Lys Thr Gly Lys Glu Met
                245                 250                 255

Phe Thr Val Ala Glu Tyr Trp Gln Asn Asp Leu Gly Ala Leu Glu Asn
            260                 265                 270

Tyr Leu Asn Lys Thr Asn Phe Asn His Ser Val Phe Asp Val Pro Leu
        275                 280                 285

His Tyr Gln Phe His Ala Ala Ser Thr Gln Gly Gly Gly Tyr Asp Met
    290                 295                 300

Arg Lys Leu Leu Asn Gly Thr Val Val Ser Lys His Pro Leu Lys Ser
305                 310                 315                 320

Val Thr Phe Val Asp Asn His Asp Thr Gln Pro Gly Gln Ser Leu Glu
                325                 330                 335

Ser Thr Val Gln Thr Trp Phe Lys Pro Leu Ala Tyr Ala Phe Ile Leu
            340                 345                 350

Thr Arg Glu Ser Gly Tyr Pro Gln Val Phe Tyr Gly Asp Met Tyr Gly
        355                 360                 365

Thr Lys Gly Asp Ser Gln Arg Glu Ile Pro Ala Leu Lys His Lys Ile
    370                 375                 380

Glu Pro Ile Leu Lys Ala Arg Lys Gln Tyr Ala Tyr Gly Ala Gln His
385                 390                 395                 400

Asp Tyr Phe Asp His His Asp Ile Val Gly Trp Thr Arg Glu Gly Asp
                405                 410                 415

Ser Ser Val Ala Asn Ser Gly Leu Ala Ala Leu Ile Thr Asp Gly Pro
```

420 425 430

Gly Gly Ala Lys Arg Met Tyr Val Gly Arg Gln Asn Ala Gly Glu Thr
435 440 445

Trp His Asp Ile Thr Gly Asn Arg Ser Glu Pro Val Val Ile Asn Ser
450 455 460

Glu Gly Trp Gly Glu Phe His Val Asn Gly Gly Ser Val Ser Ile Tyr
465 470 475 480

Val Gln Arg

<210> 2
<211> 1452
<212> DNA
<213> Bacillus licheniformis

<400> 2

```
gcaaatctta atgggacgct gatgcagtat tttgaatggt acatgcccaa tgacggccaa      60
cattggaagc gtttgcaaaa cgactcggca tatttggctg aacacggtat tactgccgtc     120
tggattcccc cggcatataa gggaacgagc caagcggatg tgggctacgg tgcttacgac     180
ctttatgatt taggggagtt tcatcaaaaa gggacggttc ggacaaagta cggcacaaaa     240
ggagagctgc aatctgcgat caaaagtctt cattcccgcg acattaacgt ttacggggat     300
gtggtcatca accacaaagg cggcgctgat gcgaccgaag atgtaaccgc ggttgaagtc     360
gatcccgctg accgcaaccg cgtaatttca ggagaacacc taattaaagc ctggacacat     420
tttcattttc cggggcgcgg cagcacatac agcgatttta aatggcattg gtaccatttt     480
gacggaaccg attgggacga gtcccgaaag ctgaaccgca tctataagtt tcaaggaaag     540
gcttgggatt gggaagtttc caatgaaaac ggcaactatg attatttgat gtatgccgac     600
atcgattatg accatcctga tgtcgcagca gaaattaaga gatggggcac ttggtatgcc     660
aatgaactgc aattggacgg tttccgtctt gatgctgtca aacacattaa attttctttt     720
ttgcgggatt gggttaatca tgtcagggaa aaaacgggga aggaaatgtt tacggtagct     780
gaatattggc agaatgactt gggcgcgctg gaaaactatt tgaacaaaac aaattttaat     840
cattcagtgt ttgacgtgcc gcttcattat cagttccatg ctgcatcgac acagggaggc     900
ggctatgata tgaggaaatt gctgaacggt acggtcgttt ccaagcatcc gttgaaatcg     960
gttacatttg tcgataacca tgatacacag ccggggcaat cgcttgagtc gactgtccaa    1020
acatggttta agccgcttgc ttacgctttt attctcacaa gggaatctgg ataccctcag    1080
gttttctacg gggatatgta cgggacgaaa ggagactccc agcgcgaaat tcctgccttg    1140
aaacacaaaa ttgaaccgat cttaaaagcg agaaaacagt atgcgtacgg agcacagcat    1200

gattatttcg accaccatga cattgtcggc tggacaaggg aaggcgacag ctcggttgca    1260
aattcaggtt tggcggcatt aataacagac ggacccggtg gggcaaagcg aatgtatgtc    1320
ggccggcaaa acgccggtga gacatggcat gacattaccg gaaaccgttc ggagccggtt    1380
gtcatcaatt cggaaggctg gggagagttt cacgtaaacg gcgggtcggt ttcaatttat    1440
gttcaaagat ga                                                        1452
```

<210> 3
<211> 463
<212> PRT
<213> Trichoderma reesei

<400> 3

Met Lys Leu Arg Tyr Ala Leu Pro Leu Leu Leu Gln Leu Ser Leu Pro
1               5                   10                  15

Val Leu Ser Ala Asp Thr Ala Ala Trp Arg Ser Arg Thr Ile Tyr Phe
            20                  25                  30

Ala Leu Thr Asp Arg Ile Ala Arg Gly Ser Gly Asp Thr Gly Gly Ser
        35                  40                  45

Ala Cys Gly Asn Leu Gly Asp Tyr Cys Gly Gly Thr Phe Gln Gly Leu
    50                  55                  60

Glu Ser Lys Leu Asp Tyr Ile Lys Gly Met Gly Phe Asp Ala Ile Trp
65                  70                  75                  80

Ile Thr Pro Val Val Thr Ser Asp Asp Gly Gly Tyr His Gly Tyr Trp
                85                  90                  95

Ala Glu Asp Ile Asp Ser Ile Asn Ser His Tyr Gly Ser Ala Asp Asp
            100                 105                 110

Leu Lys Ser Leu Val Asn Ala Ala His Ser Lys Gly Phe Tyr Met Met
        115                 120                 125

Val Asp Val Val Ala Asn His Met Gly Tyr Ala Asn Ile Ser Asp Asp
    130                 135                 140

Ser Pro Ser Pro Leu Asn Gln Ala Ser Ser Tyr His Pro Glu Cys Asp
145                 150                 155                 160

Ile Asp Tyr Asn Asn Gln Thr Ser Val Glu Asn Cys Trp Ile Ser Gly
                165                 170                 175

Leu Pro Asp Leu Asn Thr Gln Ser Ser Thr Ile Arg Ser Leu Tyr Gln
            180                 185                 190

Asp Trp Val Ser Asn Leu Val Ser Thr Tyr Gly Phe Asp Gly Val Arg
195 200 205

Ile Asp Thr Val Lys His Val Glu Gln Asp Tyr Trp Pro Gly Phe Val
210 215 220

Asn Ala Thr Gly Val Tyr Cys Ile Gly Glu Val Phe Asp Gly Asp Pro
225 230 235 240

Asn Tyr Leu Leu Pro Tyr Ala Ser Leu Met Pro Gly Leu Leu Asn Tyr
245 250 255

Ala Ile Tyr Tyr Pro Met Thr Arg Phe Phe Leu Gln Gln Gly Ser Ser
260 265 270

Gln Asp Met Val Asn Met His Asp Gln Ile Gly Ser Met Phe Pro Asp
275 280 285

Pro Thr Ala Leu Gly Thr Phe Val Asp Asn His Asp Asn Pro Arg Phe
290 295 300

Leu Ser Ile Lys Asn Asp Thr Ala Leu Leu Lys Asn Ala Leu Thr Tyr
305 310 315 320

Thr Ile Leu Ser Arg Gly Ile Pro Ile Val Tyr Tyr Gly Thr Glu Gln
325 330 335

Ala Phe Ser Gly Gly Asn Asp Pro Ala Asn Arg Glu Asp Leu Trp Arg
340 345 350

Ser Gly Phe Asn Ala Gln Ser Asp Met Tyr Asp Ala Ile Ser Lys Leu
355 360 365

Thr Tyr Ala Lys His Ala Val Gly Gly Leu Ala Asp Asn Asp His Lys
370 375 380

His Leu Tyr Val Ala Asp Thr Ala Tyr Ala Phe Ser Arg Ala Gly Gly
385 390 395 400

Asn Met Val Ala Leu Thr Thr Asn Ser Gly Ser Gly Ser Ser Ala Gln
405 410 415

His Cys Phe Gly Thr Gln Val Pro Asn Gly Arg Trp Gln Asn Val Phe
420 425 430

Asp Glu Gly Asn Gly Pro Thr Tyr Ser Ala Asp Gly Asn Gly Gln Leu
435 440 445

Cys Leu Asn Val Ser Asn Gly Gln Pro Ile Val Leu Leu Ser Ser
450 455 460

<210> 4
<211> 1392
<212> DNA
<213> Trichoderma reesei

<400> 4

```
atgaagctcc ggtacgctct cccgctgctc ttgcagctct ctttgccggt cctctccgca     60
gacaccgccg cctggaggtc ccgcaccatc tactttgccc tgacagaccg catcgctcgt    120
ggaagcggtg acacgggggg cagtgcgtgt gggaacctgg gggactactg cggtggcacg    180
ttccagggct tggagagcaa gttggactac atcaagggca tgggattcga tgccatctgg    240
atcacacctg ttgtgacgag tgatgatggg ggctaccatg gctattgggc ggaggacatc    300
gactccatca actctcatta tggctctgcg gacgatctca agagtctcgt caacgccgcg    360
catagcaagg gcttctatat gatggtggac gtcgtggcca accacatggg ctacgccaat    420
atctctgacg atagtccctc tccactgaac caggcctcgt cgtatcaccc cgagtgtgat    480
atcgactaca acaaccaaac cagcgtcgag aactgctgga tcagcggcct cccggatctc    540
aacacgcaga gctcaaccat ccgcagcctc taccaggact gggtctccaa cctcgtgtcc    600
acgtacggct tcgacggcgt ccgcatcgac accgtcaagc acgtcgagca agactactgg    660
cccggcttcg tcaacgccac cggcgtctac tgcatcggcg aggtctttga cggagaccca    720
aactacctgc tgccctacgc cagcctcatg ccgggcctgc tcaactacgc catctactac    780
cccatgacgc gcttcttcct ccagcagggc tcctcgcagg acatggtcaa catgcacgac    840
cagatcggca gcatgttccc cgacccgacc gcgctcggca cctttgtcga caaccacgac    900
aacccgcgct tcctgagcat caagaacgac acggccctgc tcaagaacgc gctgacgtac    960
accatcctct cgcgcggcat ccccatcgtc tactacggca ccgagcaggc cttctcgggc   1020
ggcaacgacc cggccaacag ggaggacctc tggcgcagcg gcttcaacgc ccagtccgac   1080
atgtacgacg ccatctccaa gctcacctac gccaagcacg ccgtcggcgg cctcgccgac   1140
aacgaccaca agcacctgta cgtcgccgac acggcctacg ccttcagccg cgccggcggc   1200
aacatggtgg ccctgaccac caacagcggc agcgggagct cggcccagca ctgcttcggc   1260
acgcaggtgc ccaacggccg ctggcagaat gtctttgacg agggcaatgg gccgacgtat   1320
tccgccgacg gcaacggcca gctttgcttg aatgtgtcca acggtcagcc cattgtcttg   1380
ctgtcttcgt ga                                                       1392
```

<210> 5
<211> 483
<212> PRT
<213> Bacillus licheniformis

<400> 5

```
Ala Asn Leu Asn Gly Thr Leu Met Gln Tyr Phe Glu Trp Tyr Thr Pro
1               5                   10                  15

Asn Asp Gly Gln His Trp Lys Arg Leu Gln Asn Asp Ser Ala Tyr Leu
            20                  25                  30

Ala Glu His Gly Ile Thr Ala Val Trp Ile Pro Pro Ala Tyr Lys Gly
        35                  40                  45

Thr Ser Gln Ala Asp Val Gly Tyr Gly Ala Tyr Asp Leu Tyr Asp Leu
    50                  55                  60

Gly Glu Phe His Gln Lys Gly Thr Val Arg Thr Lys Tyr Gly Thr Lys
65                  70                  75                  80

Gly Glu Leu Gln Ser Ala Ile Lys Ser Leu His Ser Arg Asp Ile Asn
                85                  90                  95

Val Tyr Gly Asp Val Val Ile Asn His Lys Gly Gly Ala Asp Ala Thr
            100                 105                 110

Glu Asp Val Thr Ala Val Glu Val Asp Pro Ala Asp Arg Asn Arg Val
        115                 120                 125

Ile Ser Gly Glu Tyr Leu Ile Lys Ala Trp Thr His Phe His Phe Pro
    130                 135                 140

Gly Arg Gly Ser Thr Tyr Ser Asp Phe Lys Trp His Trp Tyr His Phe
145                 150                 155                 160

Asp Gly Thr Asp Trp Asp Glu Ser Arg Lys Leu Asn Arg Ile Tyr Lys
                165                 170                 175

Phe Gln Gly Lys Ala Trp Asp Trp Glu Val Ser Ser Glu Asn Gly Asn
            180                 185                 190

Tyr Asp Tyr Leu Met Tyr Ala Asp Ile Asp Tyr Asp His Pro Asp Val
        195                 200                 205

Val Ala Glu Ile Lys Arg Trp Gly Thr Trp Tyr Ala Asn Glu Leu Gln
    210                 215                 220

Leu Asp Gly Phe Arg Leu Asp Ala Val Lys His Ile Lys Phe Ser Phe
225                 230                 235                 240

Leu Arg Asp Trp Val Asn His Val Arg Glu Lys Thr Gly Lys Glu Met
                245                 250                 255
```

```
Phe Thr Val Ala Glu Tyr Trp Gln Asn Asp Leu Gly Ala Leu Glu Asn
            260                 265                 270

Tyr Leu Asn Lys Thr Asn Phe Asn His Ser Val Phe Asp Val Pro Leu
        275                 280                 285

His Tyr Gln Phe His Ala Ala Ser Thr Gln Gly Gly Gly Tyr Asp Met
    290                 295                 300

Arg Lys Leu Leu Asn Gly Thr Val Val Ser Lys His Pro Leu Lys Ser
305                 310                 315                 320

Val Thr Phe Val Asp Asn His Asp Thr Gln Pro Gly Gln Ser Leu Glu
                325                 330                 335

Ser Thr Val Gln Thr Trp Phe Lys Pro Leu Ala Tyr Ala Phe Ile Leu
            340                 345                 350

Thr Arg Glu Ser Gly Tyr Pro Gln Val Phe Tyr Gly Asp Met Tyr Gly
        355                 360                 365

Thr Lys Gly Asp Ser Gln Arg Glu Ile Pro Ala Leu Lys His Lys Ile
    370                 375                 380

Glu Pro Ile Leu Lys Ala Arg Lys Gln Tyr Ala Tyr Gly Ala Gln His
385                 390                 395                 400

Asp Tyr Phe Asp His His Asp Ile Val Gly Trp Thr Arg Glu Gly Asp
                405                 410                 415

Ser Ser Val Ala Asn Ser Gly Leu Ala Ala Leu Ile Thr Asp Gly Pro
            420                 425                 430

Gly Gly Ala Lys Arg Met Tyr Val Gly Arg Gln Asn Ala Gly Glu Thr
        435                 440                 445

Trp His Asp Ile Thr Gly Asn Arg Ser Glu Pro Val Val Ile Asn Ser
    450                 455                 460

Glu Gly Trp Gly Glu Phe His Val Asn Gly Gly Ser Val Ser Ile Tyr
465                 470                 475                 480
```

Val Gln Arg

<210> 6
<211> 483
<212> PRT
<213> Bacillus amyloliquefaciens

<400> 6

```
Val Asn Gly Thr Leu Met Gln Tyr Phe Glu Trp Tyr Thr Pro Asn Asp
1               5                   10                  15

Gly Gln His Trp Lys Arg Leu Gln Asn Asp Ala Glu His Leu Ser Asp
            20                  25                  30

Ile Gly Ile Thr Ala Val Trp Ile Pro Pro Ala Tyr Lys Gly Leu Ser
        35                  40                  45

Gln Ser Asp Asn Gly Tyr Gly Pro Tyr Asp Leu Tyr Asp Leu Gly Glu
    50                  55                  60

Phe Gln Gln Lys Gly Thr Val Arg Thr Lys Tyr Gly Thr Lys Ser Glu
65                  70                  75                  80

Leu Gln Asp Ala Ile Gly Ser Leu His Ser Arg Asn Val Gln Val Tyr
                85                  90                  95

Gly Asp Val Val Leu Asn His Lys Ala Gly Ala Asp Ala Thr Glu Asp
            100                 105                 110

Val Thr Ala Val Glu Val Asn Pro Ala Asn Arg Asn Gln Glu Thr Ser
        115                 120                 125

Glu Glu Tyr Gln Ile Lys Ala Trp Thr Asp Phe Arg Phe Pro Gly Arg
    130                 135                 140

Gly Asn Thr Tyr Ser Asp Phe Lys Trp His Trp Tyr His Phe Asp Gly
145                 150                 155                 160

Ala Asp Trp Asp Glu Ser Arg Lys Ile Ser Arg Ile Phe Lys Phe Arg
                165                 170                 175

Gly Glu Gly Lys Ala Trp Asp Trp Glu Val Ser Ser Glu Asn Gly Asn
            180                 185                 190

Tyr Asp Tyr Leu Met Tyr Ala Asp Val Asp Tyr Asp His Pro Asp Val
        195                 200                 205

Val Ala Glu Thr Lys Lys Trp Gly Ile Trp Tyr Ala Asn Glu Leu Ser
    210                 215                 220

Leu Asp Gly Phe Arg Ile Asp Ala Ala Lys His Ile Lys Phe Ser Phe
225                 230                 235                 240

Leu Arg Asp Trp Val Gln Ala Val Arg Gln Ala Thr Gly Lys Glu Met
                245                 250                 255
```

Phe Thr Val Ala Glu Tyr Trp Gln Asn Asn Ala Gly Lys Leu Glu Asn
260 265 270

Tyr Leu Asn Lys Thr Ser Phe Asn Gln Ser Val Phe Asp Val Pro Leu
275 280 285

His Phe Asn Leu Gln Ala Ala Ser Ser Gln Gly Gly Gly Tyr Asp Met
290 295 300

Arg Arg Leu Leu Asp Gly Thr Val Val Ser Arg His Pro Glu Lys Ala
305 310 315 320

Val Thr Phe Val Glu Asn His Asp Thr Gln Pro Gly Gln Ser Leu Glu
325 330 335

Ser Thr Val Gln Thr Trp Phe Lys Pro Leu Ala Tyr Ala Phe Ile Leu
340 345 350

Thr Arg Glu Ser Gly Tyr Pro Gln Val Phe Tyr Gly Asp Met Tyr Gly
355 360 365

Thr Lys Gly Thr Ser Pro Lys Glu Ile Pro Ser Leu Lys Asp Asn Ile
370 375 380

Glu Pro Ile Leu Lys Ala Arg Lys Glu Tyr Ala Tyr Gly Pro Gln His
385 390 395 400

Asp Tyr Ile Asp His Pro Asp Val Ile Gly Trp Thr Arg Glu Gly Asp
405 410 415

Ser Ser Ala Ala Lys Ser Gly Leu Ala Ala Leu Ile Thr Asp Gly Pro
420 425 430

Gly Gly Ser Lys Arg Met Tyr Ala Gly Leu Lys Asn Ala Gly Glu Thr
435 440 445

Trp Tyr Asp Ile Thr Gly Asn Arg Ser Asp Thr Val Lys Ile Gly Ser
450 455 460

Asp Gly Trp Gly Glu Phe His Val Asn Asp Gly Ser Val Ser Ile Tyr
465 470 475 480

Val Gln Lys

<210> 7
<211> 6
<212> PRT
<213> Artificial Sequence

<220>

<223> Amino acid sequence motif

<400> 7

Ser Ala Ile Lys Ser Leu
1 5

<210> 8
<211> 6
<212> PRT
<213> Artificial Sequence

<220>
<223> Amino acid sequence motif

<400> 8

Asp Val Val Ile Asn His
1 5

<210> 9
<211> 6
<212> PRT
<213> Artificial Sequence

<220>
<223> Amino acid sequence motif

<400> 9

Ser Gly Glu His Leu Ile
1 5

<210> 10
<211> 6
<212> PRT
<213> Artificial Sequence

<220>
<223> Amino acid sequence motif

<400> 10

Asn Arg Ile Tyr Lys Phe
1 5

<210> 11
<211> 8
<212> PRT
<213> Artificial Sequence

<220>
<223> Amino acid sequence motif

<400> 11

Pro Leu His Tyr Gln Phe His Ala
1 5

<210> 12
<211> 6
<212> PRT
<213> Artificial Sequence

<220>
<223> Amino acid sequence motif

<400> 12

```
Tyr Val Gly Arg Gln Asn
1               5
```

<210> 13
<211> 6
<212> PRT
<213> Artificial Sequence

<220>
<223> Amino acid sequence motif

<400> 13

```
Glu Thr Trp His Asp Ile
1               5
```